(19) Europäisches Patentamt / European Patent Office / Office européen des brevets

(11) **EP 3 533 883 B1**

(12) **EUROPEAN PATENT SPECIFICATION**

(45) Date of publication and mention of the grant of the patent:
**10.12.2025 Bulletin 2025/50**

(51) International Patent Classification (IPC):
**G16H 50/20** (2018.01)    **G16B 25/10** (2019.01)
**G01N 33/574** (2006.01)    **C12Q 1/6886** (2018.01)
**G16B 20/00** (2019.01)    **G16B 40/00** (2019.01)
**G16H 30/40** (2018.01)    **G16H 40/63** (2018.01)
**G16H 50/30** (2018.01)

(21) Application number: **19156736.1**

(22) Date of filing: **12.02.2019**

(52) Cooperative Patent Classification (CPC):
**G16H 50/30; C12Q 1/6886; G01N 33/57434;
G16B 20/00; G16B 25/10; G16B 40/00;
G16H 30/40; G16H 40/63; G16H 50/20;
G01N 2800/54**

(54) **PREDICTING CANCER RECURRENCE USING A PROGNOSTIC MODEL THAT COMBINES IMMUNOHISTOCHEMICAL STAINING AND GENE EXPRESSION PROFILING**

VORHERSAGE DES TUMORREZIDIVS UNTER VERWENDUNG EINES PROGNOSTISCHEN MODELLS, DAS IMMUNHISTOCHEMISCHE FÄRBUNG UND GENEXPRESSIONSPROFILIERUNG KOMBINIERT

PRÉDICTION DE LA RÉCURRENCE DU CANCER À L'AIDE D'UN MODÈLE DE PRONOSTIC COMBINANT LA COLORATION IMMUNOHISTOCHIMIQUE ET LE PROFILAGE DE L'EXPRESSION GÉNIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.02.2018 US 201862629591 P**

(43) Date of publication of application:
**04.09.2019 Bulletin 2019/36**

(73) Proprietor: **AstraZeneca Computational Pathology GmbH**
**80636 München (DE)**

(72) Inventor: **SCHMIDT, Günter**
**80999 München (DE)**

(74) Representative: **AstraZeneca Intellectual Property**
**Eastbrook House**
**Shaftesbury Road**
**Cambridge CB2 8BF (GB)**

(56) References cited:
- **NATHALIE HARDER ET AL: "Combining immunophenomics with a gene expression panel for improved prostate cancer recurrence prediction", JOURNAL FOR IMMUNOTHERAPY OF CANCER, vol. 5, no. Suppl 2, 7 November 2017 (2017-11-07), pages 2 - 2, XP055607391**
- **JIA-MEI CHEN ET AL: "Computer-aided prognosis on breast cancer with hematoxylin and eosin histopathology images: A review", TUMOR BIOLOGY, vol. March 2017, 1 March 2017 (2017-03-01), pages 1 - 12, XP055607422**
- **PRANTIK CHATTERJEE ET AL: "Discovery of synergistic genetic network: A minimum spanning tree-based approach", JOURNAL OF BIOINFORMATICS AND COMPUTATIONAL BIOLOGY, vol. 14, no. 01, 24 February 2016 (2016-02-24), GB, pages 1650003, XP055607117, ISSN: 0219-7200, DOI: 10.1142/ S0219720016500037**

**Description**

**Field of the invention**

The invention relates to a method for determining a risk of a cancer patient of a cancer recurrence. The disclosure further relates to a method of generating a network of prognostic features for cancer recurrence of a cancer patient. More particularly, the invention relates to the prediction of prostate cancer recurrence using a prognostic model that combines immunohistochemical staining and gene expression profiling.

**Background of the invention**

A cancer patient can be treated such that the cancer goes into remission. Knowing whether and when the cancer might later come out of remission and recur would, for many reasons, be beneficial. Having such information may facilitate making better clinical and treatment decisions. Having such information may also allow the patient to improve the patient's quality of life, and to make better life decisions.

Harder et al., (Journal for Immunotherapy of Cancer, vol. 5, no. Suppl 2, 7 November 2017, page 2, abstract O3) describes combining immunophenomics with a gene expression panel for prostate cancer recurrence prediction.

Jia-Mei Chen et al., (Tumor Biology, March 2017, pages 1-12) summarizes recent works in image analysis of hematoxylin and eosin histopathology images for breast cancer prognosis. Prantik Chatterjee et al., (Journal of Bioinformatics and Computational Biology, vol. 14, no. 1, 24 February 2016, page 1650003) describes an approach for identifying synergistic gene interactions directly from the continuous expression levels, using a minimum spanning tree (MST)-based algorithm.

The identification of those cancer patients who will exhibit a cancer recurrence is an unsolved problem for many types of cancer. For example, the accurate identification of those prostate cancer patients who will exhibit a prostate specific antigen (PSA) recurrence after radical prostatectomy is an unsolved problem.

Therefore, an improved method for determining a risk of a cancer patient of a cancer recurrence is needed.

**Summary of the invention**

The invention relates to a method for determining a risk of a cancer patient of a cancer recurrence, the method comprising the steps of:

(a) receiving raw structural feature measurement data of cancer tissue samples of a plurality of patients having the cancer onto a system;

(b) defining a structural feature based at least in part on the raw structural feature measurement data received in (a), wherein the structural feature includes a list of rank values;

(c) receiving raw gene expression feature measurement data of the cancer tissue samples of the plurality of patients onto the system;

(d) defining a gene expression feature based at least in part on the raw gene expression feature measurement data received in (c), wherein the gene expression feature includes a list of rank values;

(e) defining a bivariate prognostic feature by combining rank values of the list of rank values of the structural feature with rank values of the list of rank values of the gene expression feature thereby generating a list of rank values for the bivariate prognostic feature, wherein the defining of the bivariate prognostic feature in (e) further involves determining and storing a cut-point value for the bivariate prognostic feature;

(f1) measuring *in vitro* a structural feature measurement data value of a structural feature of a cancer tissue sample of the patient;

(f2) receiving the structural feature measurement data value onto the system, wherein the structural feature measurement data value is data obtained by analyzing a digital image of a first portion of tissue of the tissue sample;

(g1) measuring *in vitro* a gene expression feature measurement data value of a gene expression feature of the tissue sample, wherein the gene expression feature is a gene expression level of a gene;

wherein a combination of the structural feature and the gene expression feature is the bivariate prognostic feature for the recurrence of the cancer in the plurality of patients having the cancer defined in (e), wherein the structural feature and the gene expression feature are elements of a minimal spanning tree that comprises nodes and edges, wherein each edge of the minimal spanning tree connects two nodes, wherein the minimal spanning tree is constructed from structural features, gene expression features and bivariate prognostic features for the recurrence of the cancer in the plurality of patients,

wherein each node of the minimal spanning tree represents a structural feature or a gene expression feature,

wherein each edge of the minimal spanning tree represents a bivariate prognostic feature, wherein the bivariate prognostic feature is a combination of the two features that are represented by the two nodes connected by the edge, wherein at least one of the two features is a univariate prognostic feature for the recurrence of the cancer in the plurality of patients, and

wherein the width of each edge indicates a bivariate prognostic value of the bivariate prognostic feature that is represented by the edge;

(g2) receiving the gene expression feature measurement data value onto the system, wherein the gene expression feature measurement data value is data obtained by analyzing a second portion of the tissue of the tissue sample;

(h) calculating a score value for the bivariate prognostic feature based at least in part on the structural feature measurement data value received in (f2), the gene expression feature measurement data value received in (g2), and the cut-point value of (e), wherein the receiving of (f2) and the receiving of (g2) and the calculating in (h) are all per- formed after the defining of the bivariate prognostic feature in (e);

(i) determining a score by evaluating a function, wherein the function is a function of the score value calculated in (h), wherein (a) through (i) are performed by the system, and wherein the score determined in (i) is indicative of whether the cancer patient will have a recurrence of the cancer; and

(j) displaying a graphical indication of the combination of the structural feature measurement data value and the gene expression featumeasurement data value on a graphical user interface to illustrate the risk of the patient of the recurrence of the cancer,

wherein the structural feature is an average distance between first objects and second objects of the tissue sample,

wherein the first objects and the second objects correspond to two different cell types and the average distance between the first objects and the second objects is determined by analyzing one or more digital images of one or more tissue slices of the tissue sample that have been stained by immuno- histochemistry (IHC) using protein-specific IHC biomarkers that allow identifying the two different cell types,

wherein the gene expression level of the gene is a measurement of expression of mRNA transcribed from the gene in the tissue sample by using an mRNA-specific probe biomarker,

wherein all steps of the method are performed by the system.

**Brief description of the drawings**

FIG. 1A shows the distribution of age and Gleason score of the patients. The columns marked with (+) represent patients with PSA recurrence. The columns marked with (-) represent patients without PSA recurrence.

FIG. 1B shows the patients' disease-free survival rate over time depending on their Gleason score.

FIG. 1C shows an overview of the immuno-phenomics workflow, i.e. the workflow of the IHC-based analysis.

FIG. 1D shows IHC images obtained from tissue sections stained with CK18/p63, CD68/CD163, CD3/CD8 and CD34, respectively (left column), and their segmentation for structure detection (right column). Fig. 1D further shows a hyperspectral heatmap obtained by aggregation of all structures and a table with data on the 462 features that were computed.

FIG. 1E shows an overview of the roles of the proteins that are encoded by the genes covered by the gene expression analysis.

FIG. 1F shows an overview of the workflow of the gene expression analysis using the NanoString nCounter PanCancer Immune Profiling Panel.

FIG. 1G shows the normalization of the C4B gene expression measurement.

FIG. 1H shows tests for the combination of C4B with CD47 and for the combination of CTSH with IL17B for bivariate feature selection. Lighter bubbles ("0" in the figure legend) correspond to patients without PSA recurrence. Darker bubbles correspond to patients with PSA recurrence ("1" in the figure legend). The bubble size indicates the score.

FIG. 2A shows an overview of the most prognostic univariate features including the log-p-value of the feature value cut point indicated with the bubble size. Darker bubbles correspond to non-significant log-p-values. Lighter bubbles correspond to significant log-p-values. Features having a significant ($p<0.05$) mean Kaplan Meier log-rank test p-value within a range of 5 cut points were selected (box in the center of the figure).

FIG. 2B shows an overview of the Kaplan-Meier curves of all 32 discovered prognostic univariate features.

FIG. 2C shows a matrix of the 9746 significant bivariate p-values obtained by the bivariate feature selection.

FIG. 2D shows a network including the prognostic bivariate features. The network is an extended Minimal Spanning Tree (MST) of bivariate models (bivariate features) predicting PSA recurrence, wherein the MST was extended by the most significant (75% quantile) bivariate models.

FIG. 2E shows an overview of the Kaplan-Meier curves of all 22 discovered prognostic bivariate features.

FIG. 2F shows an example stratification using the score (bivariate feature) "not C4B and CD47". A higher score indicates a higher risk of PSA recurrence as shown in the right panel (yes=1, no=0).

FIG. 2G shows an example of a user interface element that visualizes the PSA recurrence risk of a given patient (horizontal arrow) in the context of the results of this study using the bivariate feature "not C4B and CD47". The left box plot shows the feature values of study patients who did not show PSA recurrence. The right box shows the feature values of study patients who exhibited PSA recurrence.

FIG. 3A is a diagram of a system for predicting the recurrence of cancer.

FIG. 3B is a diagram that illustrates how, in a "diagnostic phase" operation of the system of FIG. 3A, a tissue sample from a patient is used to generate both raw phenomic feature measurement data as well as raw genomic feature measurement data.

FIG. 4A is a diagram of a Minimal Spanning Tree (MST) that includes univariate phenomic features, univariate genomic features, and bivariate features. One of the bivariate features represents a relationship between a univariate phenomic feature and a univariate genomic feature.

FIG. 4B is a two-dimensional matrix of the prognostic values (-log(log-rank p-values)) of bivariate relationships between various pairs of univariate features.

FIG. 5 is a table that sets forth thirty-two univariate features that are determined to have significant prognostic value in the prediction of the recurrence of cancer (specifically prostate cancer).

FIG. 6 is a grayscale version of a high-resolution digital image of a first slice of tissue that was duplex stained in an IHC-based image analysis process.

FIG. 7 is a grayscale version of a high-resolution digital image of a second slice of tissue that was duplex stained in the IHC-based image analysis process.

FIG. 8 is an expanded view of a portion of the first digital image of FIG. 6.

FIG. 9 is an expanded view of a portion of the second digital image of FIG. 7.

FIG. 10 is an illustrative diagram that shows how the average distance from a CD163(+) object to its nearest four CD3(+)CD8(-) objects is determined.

FIG. 11 is a diagram that sets forth LGALS3 raw measurements used in the learning phase of the system.

FIG. 12 shows how the information in the rows of FIG. 11 is reordered (i.e., "ranked") so that the top row is for the patient having the smallest raw measurement count, and so that the bottom row is for the patient having the largest raw measurement count.

FIG. 13 shows how the raw measurement count values of FIG. 12 are normalized by rank percentage.

FIG. 14 is a Kaplan-Meier plot for the data of FIG. 13.

FIG. 15 is a diagram that sets forth MAGEC2 raw measurements used in the learning phase of the system.

FIG. 16 shows how the information in the rows of FIG. 15 is reordered (i.e., "ranked") so that the top row is for the patient having the smallest raw measurement count, and so that the bottom row is for the patient having the largest raw measurement count.

FIG. 17 shows how the raw measurement count values of FIG. 16 are normalized by rank percentage.

FIG. 18 is a Kaplan-Meier plot for the data of FIG. 17.

FIG. 19 is a diagram that sets forth IHC_DIST_CD163(+)_CD3(+)CD8(-) raw measurements used in the learning phase of the system.

FIG. 20 shows how the information in the rows of FIG. 15 is reordered (i.e., "ranked") so that the top row is for the patient having the smallest raw measurement count, and so that the bottom row is for the patient having the largest raw measurement count.

FIG. 21 shows how the raw measurement count values of FIG. 20 are normalized by rank percentage.

FIG. 22 is a Kaplan-Meier plot for the data of FIG. 21.

FIG. 23 is a table that shows the four "fuzzy logic" bivariate scoring methods.

FIG. 24 is a diagram that shows how LGALS3-to-MAGEC2 values for scoring method SM1 are calculated.

FIG. 25 is a diagram that shows how LGALS3-to-MAGEC2 values for scoring method SM2 are calculated.

FIG. 26 is a diagram that shows how LGALS3-to-MAGEC2 values for scoring method SM3 are calculated.

FIG. 27 is a diagram that shows how LGALS3-to-MAGEC2 values for scoring method SM4 are calculated.

FIG. 28 is a table showing the p-values determined for the LGALS3-to-MAGEC2 bivariate relationship for each of the four scoring methods SM1, SM2, SM3 and SM4.

FIG. 29 is a Kaplan-Meier plot for the LGALS3-to-MAGEC2 bivariate relationship when the SM1 bivariate scoring method is used.

FIG. 30 is a diagram that shows how IHC_DIST_CD163(+)_CD3(+)CD8(-)-to-MAGEC2 values for scoring method SM1 are calculated.

FIG. 31 is a diagram that shows how IHC_DIST_CD163(+)_CD3(+)CD8(-)-to-MAGEC2 values for scoring method SM2 are calculated.

FIG. 32 is a diagram that shows how IHC_DIST_CD163(+)_CD3(+)CD8(-)-to-MAGEC2 values for scoring method SM3 are calculated.

FIG. 33 is a diagram that shows how IHC_DIST_CD163(+)_CD3(+)CD8(-)-to-MAGEC2 values for scoring method SM4 are calculated.

FIG. 34 is a table showing the p-values determined for IHC_DIST_CD163(+)_CD3(+)CD8(-)-to-MAGEC2 bivariate relationship for each of the four scoring methods SM1, SM2, SM3 and SM4.

FIG. 35 is a Kaplan-Meier plot for the IHC_DIST_CD163(+)_CD3(+)CD8(-)-to-MAGEC2 bivariate relationship when the SM3 scoring method is used.

FIG. 36 is a table showing three raw measurement values for the new patient (to be used in the diagnostic phase to determine a score for the new patient).

FIG. 37 shows how a score value is determined in the diagnostic phase for the LGALS3 univariate feature.

FIG. 38 shows how a score value is determined in the diagnostic phase for the MAGEC2 univariate feature.

FIG. 39 shows how a score value is determined in the diagnostic phase for the IHC_DIST_CD163(+)_CD3(+)CD8(-) univariate feature.

FIG. 40 shows how a score value is determined in the diagnostic phase for the LGALS3-to-MAGEC2 bivariate feature.

FIG. 41 shows how a score value is determined in the diagnostic phase for the IHC_DIST_CD163(+)_CD3(+) CD8(-)-to-MAGEC2 bivariate feature.

FIG. 42 sets forth the function that is used to determine the overall score from the underlying five feature score values.

FIG. 43 shows how the function of FIG. 42 is applied in the case of the new patient whose overall score is being determined in the diagnostic phase.

FIG. 44 is a diagram that shows the graphical user interface visualization element that is displayed in the window on the display of the system of FIG. 3A.

**Detailed description of the invention**

The inventor developed a novel method to generate a network of bivariate prognostic models (bivariate prognostic features) that surpasses the predictive power of its individual components. The network integrates measurements performed on cancer tissue samples extracted from resected cancer tissue such as a resected prostate from a prostate cancer patient. The measurements include information derived from immunohistochemically (IHC) stained tissue sections that has a high spatial resolution, but a low multiplexed resolution. The measurements also include information derived from mRNA-based gene expression profiling, which has a low spatial resolution, but a highly multiplexed resolution. In this way, data from multiple sources (multi-omics) is combined in order to facilitate an improved prediction of cancer recurrence. The network is a minimal spanning tree. The inventor also developed a prognostic test that utilizes the network of bivariate prognostic features and applied the test to predict (biochemical) prostate cancer recurrence. The test can be applied to automatically predict (biochemical) prostate cancer recurrence. Based on the prediction, an accurate stratification of cancer patients into treatment groups is possible.

The disclosure relates to an *in vitro* method for determining a risk of a cancer patient of a cancer recurrence, the method comprising the steps of:

(a) measuring *in vitro* a structural feature value of a structural feature of a cancer tissue sample of the patient;
(b) measuring *in vitro* a gene expression feature value of a gene expression feature of the tissue sample, wherein the gene expression feature is a gene expression level of a gene;

wherein a combination of the structural feature and the gene expression feature is a bivariate prognostic feature for the recurrence of the cancer in a plurality of patients having the cancer,

wherein the structural feature and the gene expression feature are elements of a minimal spanning tree that comprises nodes and edges, wherein each edge of the minimal spanning tree connects two nodes, wherein the minimal spanning tree is constructed from structural features, gene expression features and bivariate prognostic features for the recurrence of the cancer in the plurality of patients,

wherein each node of the minimal spanning tree represents a structural feature or a gene expression feature, wherein each edge of the minimal spanning tree represents a bivariate prognostic feature, wherein the bivariate prognostic feature is a combination of the two features that are represented by the two nodes connected by the edge, wherein at least one of the two features is a univariate prognostic feature for the recurrence of the cancer in the plurality of patients, and

wherein the width of each edge indicates a bivariate prognostic value of the bivariate prognostic feature that is represented by the edge; and

(c) displaying a graphical indication of the combination of the structural feature value and the gene expression feature value on a graphical user interface to illustrate the risk of the patient of the recurrence of the cancer.

The method allows predicting cancer recurrence in a cancer patient. It thus allows predicting the risk of cancer progression. The method is particularly suitable to predict PSA recurrence in a prostate cancer patient.

The method is suitable for all types of solid cancers. The method requires a cancer tissue sample that was obtained from the cancer patient. In the case of prostate cancer, tissue from radical prostatectomy can be used to provide a prostate tissue sample of the cancer patient.

The term "structural feature" as used herein refers to a structural characteristic of tissue that can be obtained by analyzing one or more digital images of the tissue. The terms "structural feature", "IHC-based feature", "phenomic feature" or "first feature" are used interchangeably herein.

To obtain a structural feature of a tissue, one or more slices of the tissue are stained by immunohistochemistry (IHC). In IHC, a slice of tissue is stained with at least one antibody that specifically binds to a protein of interest. The terms "protein-specific immunohistochemical (IHC) biomarker" or "immunohistochemical biomarker" as used herein refer to such an antibody used in IHC. Binding of the antibody to the tissue is usually detected by incubation of the tissue with a secondary antibody that carries a label. The label can be a fluorescent tag. One or more digital images are taken of the stained tissue slices. The tissue slices can be double-stained, i.e. stained with two different antibodies to mark two different proteins of interest on the same tissue slice. The selective staining of different proteins of interest allows identifying certain cell types and/or physical structures within the tissue so that one or more structural features of the tissue can be obtained. The structural feature may be, for example, a count of a certain cell type within the tissue, or a size of a certain structure within the tissue, or a density of certain cells within the tissue. One example of a structural feature is the number of M1 macrophages in parts of tissue referred to as "influence zones". Another example of a structural feature is the number of M1 macrophages in other parts of tissue referred to as "stroma regions". Another example of a structural feature is the density of M2 macrophages in other regions of the tissue. Another example of a structural feature is a number that in turn is a function of multiple other such structural feature numbers. Another example of a structural feature is a ratio of the number of one type of cells to another type of cells. US 2017/0270420 A1 describes how tissue sample slices may be prepared, and stained by IHC, and imaged, and analyzed using image analysis in order to identify and to measure and to quantify structural features present in tissue of a cancer patient.

In a preferred embodiment, the structural feature is an average distance between two different types of cells detected within the tissue.

In a further preferred embodiment, the structural feature is an average distance of CD163 positive (CD163(+)) cells to CD3 positive CD8 negative (CD3(+)CD8(-)) cells in a tissue sample. This structural feature is referred to as "IHC_Dist_CD163(+)_CD3(+)CD8(-)". To obtain this feature, IHC and image analysis are used to identify CD163(+) stained objects, which correspond to CD163 positive cells, in the tissue sample. These objects correspond to M2-type macrophages. IHC and image analysis is also used to identify CD3(+)CD8(-) objects, which correspond to CD3 positive CD8 negative cells, in the same tissue sample.

These objects correspond to non-cytotoxic T-cells in the tissue sample. Each CD163(+) object is then considered, and for that CD163(+) object the average distance (in micrometers) between it and the four nearest identified CD3(+)CD8(-) objects is determined. The average of all these averages for all the CD163(+) objects in the tissue sample is then determined, and this overall average is the raw measurement value for the IHC_Dist_CD163(+)_CD3(+)CD8(-) feature. Additional detail on how this raw measurement value is determined is set forth below.

The term "gene expression feature" as used herein refers to a gene expression level of a gene that is expressed in the tissue sample. The terms "gene expression feature", "genomic feature" or "second feature" are used interchangeably herein. The gene expression level may be given as a count, where the count is indicative of the degree of expression of the gene.

To obtain a gene expression feature of a tissue sample, a probe of labeled RNA that specifically hybridizes with (i.e. attaches to) a complementary sequence of an mRNA transcribed from the gene of interest is used. The terms "mRNA-specific probe biomarker", "gene-specific probe biomarker", "gene-specific probe" or "reporter probe" as used herein refer to such a probe of labeled RNA. In one example, a lysing buffer is used to lyse the tissue and to put mRNA molecules present in the tissue into solution. The probe of labeled RNA is then mixed in. This probe may have a color-coded barcode that can be illuminated and optically examined to identify it. In one example, a device called nCounter is used. The nCounter device is commercially available from NanoString Technologies, Inc., of Seattle, Washington. The nCounter device has a high-resolution CCD camera. The nCounter device is usable to illuminate the barcode on each reporter probe, and thereby to determine the barcode of the reporter probe and to count the number of times that a reporter probe with that same particular barcode was detected in a tissue sample. These absolute mRNA counts are then normalized with respect to multiple house-keeping genes.

The reporter probe can be used in combination with a capture probe that also specifically hybridizes with a complementary sequence of an mRNA transcribed from the gene of interest. Gene-specific probe pairs of reporter probes and capture probes are commercially available from multiple sources, including from NanoString Technologies, Inc. After the pair of probes have hybridized to their target mRNA molecules, excess probes (unattached probes) in the solution are removed. The remaining probe/mRNA complexes are then aligned and immobilized via the capture probes. The nCoutner device illuminates the probe/mRNA complexes and uses its high-resolution CCD camera to perform optical examination of the probes. In this way, the reporter probe on each individual target mRNA molecule is identified by its barcode to be a reporter probe of a particular type, and the count of this particular reporter probe type is incremented. After all the reporter probes in the sample have been detected and counted in this way, the nCounter device outputs a digital file. This digital file includes a count value. The count value indicates the number of times that a reporter probe of a particular type (bearing a particular color-coded barcode) was detected in the sample (absolute mRNA count). The count value is normalized with respect to multiple house-keeping genes. The expression level of a gene of interest is a measurement of how large the normalized count value is for the barcode of the reporter probe that is specific to the mRNA transcribed from the gene of interest.

Accordingly, in a preferred embodiment, the measuring of (b) involves a counter device that outputs a count value, wherein the count value is indicative of a number of gene-specific probes counted.

Although the nCounter device may involve a CCD camera and may perform optical inspections in order to identify probes, the nCounter is not doing wide-field phenomic image analysis in that it is not performing any analysis to identify cells, or particular types of cells, or groups of cells, or structural aspects of non-lysed tissue. The nCounter device is not measuring or outputting raw phenomic feature data. The term "phenomic" as used herein is intended to exclude the data that results from the optical identification of gene-specific probes.

The width of an edge of the minimal spanning tree corresponds to the weight of the edge. The terms "width" or "weight" are used interchangeably herein with respect to the edges of the minimal spanning tree.

The width of each edge of the minimal spanning tree indicates a bivariate prognostic value of the bivariate prognostic feature that is represented by the edge. This means that some edges of the minimal spanning tree as displayed on a graphical user interface are wider than other edges of the minimal spanning tree as displayed on the graphical user interface. Edges in the network that have more prognostic importance are rendered as thicker lines, whereas edges in the network that have less prognostic importance are rendered as thinner lines.

The type of bivariate relationship (one of four "fuzzy logic" combinations) that was determined to have the most prognostic importance can be indicated in the minimal spanning tree by the type of arrow or line that is representing the edge.

In a preferred embodiment, the bivariate prognostic value is a Kaplan Meier log rank test p-value or a hazard ratio, preferably a Kaplan Meier log rank test p-value.

In a preferred embodiment, the structural feature is the univariate prognostic feature.

In a preferred embodiment, the structural feature and the gene expression feature are each univariate prognostic features.

In a preferred embodiment, the minimal spanning tree consists of nodes and edges.

In a preferred embodiment, the minimal spanning tree is generated by Prim's algorithm, and the edge weights indicate a Kaplan Meier log rank test p-value of the bivariate prognostic features in the plurality of patients.

In a preferred embodiment, the bivariate prognostic feature is generated by a combination of two features f1 and f2 using a formula taken from the group consisting of: $f1 \times f2$ and $(1-f1) \times f2$ and $f1 \times (1-f2)$ and $(1-f1) \times (1-f2)$.

In a preferred embodiment, the size of each node that represents a univariate prognostic feature indicates a univariate prognostic value of the univariate prognostic feature that is represented by the node. This means that some nodes of the minimal spanning tree as displayed on a graphical user interface are larger than other nodes of the of the minimal spanning as displayed on the graphical user interface. The nodes of univariate prognostic features that have more prognostic importance are rendered to be larger, whereas the nodes of univariate prognostic features that have less prognostic importance are rendered to be smaller.

In a preferred embodiment, the univariate prognostic value is a Kaplan Meier log rank test p-value or a hazard ratio, preferably a Kaplan Meier log rank test p-value.

In a preferred embodiment, step (c) comprises the steps of

(c1) normalizing the structural feature value and the gene expression feature value;
(c2) calculating a bivariate feature value from the combination of the normalized structural feature value and the normalized gene expression feature value, wherein the graphical indication comprises the bivariate feature value.

In a preferred embodiment, the minimal spanning tree comprises a first node representing the structural feature measured in (a), a second node representing the gene expression feature measured in (b), and an edge that extends between the first node and the second node (i.e. that connects the first node and the second node), wherein the edge represents the bivariate feature for which the bivariate feature value is calculated in (c2).

In a preferred embodiment, the normalizing of the structural feature value and the gene expression feature value uses a function, wherein the function is derived from structural feature values and gene expression feature values and from normalized structural feature values and normalized gene expression feature values of the plurality of patients.

In a preferred embodiment, the normalizing uses rank normalization.

The plurality of patients refers to a cohort of patients exhibiting the cancer. For each of the patients, it is known whether the patient suffered a recurrence of cancer. The plurality preferably comprises more than 10 patients so that a meaningful statistical analysis can be performed. In the study performed by the inventor, 23 prostate cancer patients were included.

In a preferred embodiment, the structural feature is an average distance between first objects and second objects of the tissue sample.

The first objects and/or the second objects are preferably cellular objects, i.e. objects that correspond to cells in the tissue sample. Preferably the first objects correspond to a first type of cells and the second objects correspond to a second type of cells, wherein the second type of cells is different from the first type of cells.

In a further preferred embodiment, step (a) comprises the steps of

(a1) generating the first objects from a first digital image of a first tissue slice of the tissue sample, wherein the first tissue slice has been stained by immunohistochemistry (IHC) with a first antibody that stains cells exhibiting a first characteristic, wherein the first objects correspond to the cells stained by the first antibody;
(a2) generating the second objects from a second digital image of a second tissue slice of the tissue sample, wherein the second tissue slice has been stained by IHC with a second antibody that stains cells exhibiting a second characteristic, wherein the second objects correspond to the cells stained by the second antibody; and
(a3) determining the average distance between the first objects and the second objects of the tissue sample; and wherein step (b) comprises the step of
(b1) determining the gene expression level of the gene from a third tissue slice of the tissue sample, wherein the third tissue slice has been stained for gene expression with a probe of labeled RNA that stains mRNA molecules transcribed from the gene, wherein the gene expression level is determined by determining the amount of staining by the probe of labeled RNA on a third digital image of the third tissue slice, wherein the first tissue slice, the second tissue slice and the third tissue slice are z slices taken from the tissue sample at corresponding positions in the x and y dimensions.

In a preferred embodiment, the first characteristic and the second characteristic are proteins that allow the identification of a certain type of cells. Each of the first and the second characteristic may be one protein such as CD163 or a combination of more than one protein, preferably of two proteins such as CD3 and CD8.

In a preferred embodiment, the gene encodes an immune system related component.

In a preferred embodiment, the graphical user interface is a computer display.

In a preferred embodiment, the graphical user interface is a part of a system, and all steps of the method are performed by the system.

In a preferred embodiment, the method involves a cancer recurrence prediction system and all steps of the method are performed by the cancer recurrence prediction system.

In a preferred embodiment, the cancer is prostate cancer, wherein the cancer recurrence preferably is a recurrence of measurable prostate-specific antigen (PSA) in the patient's blood.

In a preferred embodiment, the cancer is prostate cancer and the univariate prognostic feature is a structural feature selected from the group consisting of

## IHC_DIST_CD163(+)_CD8(+)_STROMA,

## IHC_DIST_CD34(+)_CD163(+)_STROMA,

and

## IHC_DIST_CD163(+)_CD3(+)CD8(-),

The feature "IHC_DIST_CD163(+)_CD8(+)_STROMA" refers to the average distance of CD163 positive cells to CD8 positive cells in stroma.

The feature "IHC_DIST_CD34(+)_CD163(+)_STROMA" refers to the average distance of CD34 positive cells in stroma to CD163 positive cells.

The feature "IHC_DIST_CD163(+)_CD3(+)CD8(-)" refers to the average distance of CD163 positive cells to CD3 positive CD8 negative cells on the whole slide.

The term "stroma" as used herein refers to stroma within a predefined distance to the tumor. The predefined distance to the tumor can be, for example, a 150 $\mu$m threshold.

In a preferred embodiment, the cancer is prostate cancer and the univariate prognostic feature is a gene expression level of a gene selected from the group consisting of CD59, MAGEC2, ATF2, NFKBIA, CXCL13, NUP107, IL12A, JAK2, SYCP1, MAPK1, TBX21, SYK, ANXA1, PMCH, MBL2, C4B, BMI1, CCL5, STAT4, IRF1, CD47, PSEN2, CD96, CTSH, IL17B, DDX43, KLRK1, LGALS3, and PTGDR2.

In a preferred embodiment, the cancer is prostate cancer, the structural feature is an average distance of CD163 positive cells to CD3 positive CD8 negative cells in a prostate tissue sample of the patient, and the gene expression feature is the gene expression level of a MAGEC2 gene. The inventor found that the presence of both a large average distance of the CD163 positive cells to the CD3 positive CD8 negative cells and a low level of gene expression of the MAGEC2 gene indicates a low risk for the prostate cancer recurrence. The MAGEC2 gene encodes melanoma-associated antigen C2.

In a preferred embodiment, the cancer is prostate cancer, the structural feature is an average distance of CD34 positive cells in stroma to CD163 positive cells in a prostate tissue sample of the patient, and the gene expression feature is the gene expression level of a TICAM2 gene. The inventor found that the presence of both a small average distance of the CD34 positive cells in stroma to the CD163 positive cells and a high level of gene expression of the TICAM2 gene indicates a high risk for the prostate cancer recurrence. The TICAM2 gene encodes TIR domain-containing adapter molecule 2.

The terms "large", "small", "low" and "high" refer to a comparison of the average distance or the level of gene expression to a predetermined threshold that was determined from individual values of the plurality of patients. The predetermined threshold may be displayed together with or as a part of the graphical indication displayed in (c).

In a preferred embodiment, the graphical indication illustrates the risk of the patient of the recurrence of the cancer based on statistics of the risks of the plurality of patients.

The disclosure further relates to a method of generating a network of prognostic features for cancer recurrence of a cancer patient, comprising:

(a) measuring *in vitro* structural feature values of a plurality of structural features and gene expression feature values of a plurality of gene expression features of cancer tissue samples of a plurality of patients having the cancer, wherein the gene expression features are gene expression levels of genes;

(b) computing bivariate prognostic features from the structural feature values and the gene expression feature values, wherein each bivariate prognostic feature provides significant prognostic information on the cancer recurrence in the plurality of patients; and

(c) displaying the network of prognostic features on a graphical user interface, wherein the network is a minimal spanning tree that comprises nodes and edges, wherein each edge of the minimal spanning tree connects two nodes, wherein each node of the minimal spanning tree represents a structural feature of the plurality of structural features or a gene expression feature of the plurality of gene expression features, wherein each edge of the minimal spanning tree represents a bivariate prognostic feature computed in step (b), wherein the bivariate prognostic feature is a combination of the two features that are represented by the two nodes connected by the edge, wherein at least one of the two features is a univariate prognostic feature that provides significant prognostic information on the cancer recurrence in the plurality of patients, and wherein the width of each edge indicates a bivariate prognostic value of the bivariate prognostic feature that is represented by the edge.

In a preferred embodiment, a first node of the network represents a structural feature of the plurality of structural features, a second node of the network represents a gene expression feature of the plurality of gene expression features, and an edge that connects the first node and the second node represents one bivariate feature computed in (b).

In a preferred embodiment, the bivariate prognostic value is a Kaplan Meier log rank test p-value or a hazard ratio, preferably a Kaplan Meier log rank test p-value.

In a preferred embodiment, the size of each node that represents a univariate prognostic feature indicates a univariate prognostic value of the univariate prognostic feature that is represented by the node. In a preferred embodiment, the univariate prognostic value is a Kaplan Meier log rank test p-value or a hazard ratio, preferably a Kaplan Meier log rank test p-value.

In a preferred embodiment, each bivariate prognostic feature computed in (b) is computed using a fuzzy logic combination of two feature values including the operators "and" and "not", wherein the two feature values are feature values determined in step (a).

In a preferred embodiment, each bivariate prognostic feature computed in (b) determines a prognostic value on the cancer recurrence in the plurality of patients.

In a preferred embodiment, the prognostic value is computed by using a cut-point value.

In a preferred embodiment, the structural features are average distances of first objects to second objects of the tissue sample.

In a preferred embodiment, the gene encodes an immune system related component.

The graphical user interface may display all or a portion of the network.

In a preferred embodiment, the graphical user interface is a computer display.

In a preferred embodiment, the graphical user interface is a part of a system, and all steps of the method are performed by the system.

In a preferred embodiment, the method involves a cancer recurrence prediction system and all steps of the method are performed by the cancer recurrence prediction system.

In a preferred embodiment, the cancer is prostate cancer, wherein the cancer recurrence preferably is a recurrence of measurable prostate-specific antigen (PSA) in the patient's blood.

Further disclosed is a method for providing a graphical indication of whether a patient will have a recurrence of a cancer that uses univariate and bivariate prognostic features that were generated as part of a minimal spanning tree (MST). The method involves measuring a first value of a first feature and a second value of a second feature. The first value is measured based on an object detected in a digital image of a first tissue slice that has been stained with a protein-specific immunohistochemical (IHC) biomarker. The first tissue slice was cut from a formalin-fixed paraffin-embedded (FFPE) tissue sample of cancer tissue from the patient. The second value is measured based on objects marked with an mRNA-specific probe biomarker detected in a second tissue slice that was cut from the FFPE tissue sample. The first feature is the univariate prognostic feature for the recurrence of the cancer in a cohort of patients exhibiting the cancer. A combination of the first feature and the second feature is the bivariate prognostic feature for the recurrence of the cancer in the cohort of patients. The first feature and the second feature are elements of the minimal spanning tree that is constructed from first features, second features and bivariate prognostic features for the recurrence of the cancer in the cohort. Nodes of the minimal spanning tree represent the first features and the second features, edges of the minimal spanning tree represent the bivariate prognostic features, and weights of the edges represent prognostic significance of the bivariate prognostic features. The graphical indication of the combination of the first value and the second value is displayed on a graphical user interface to illustrate whether the patient will have a recurrence of the cancer.

Further disclosed is a method for generating a network of prognostic features for cancer recurrence of a cohort of cancer patients that involves computing bivariate prognostic features that are the edges of the network. A set of immunohistochemical-based (IHC-based) values of IHC-based features is measured based on a tissue sample of a tumor of each cancer patient in the cohort. A set of gene expression values of gene expression features is measured based on the tissue sample of the tumor of each cancer patient in the cohort. A set of bivariate prognostic features is computed that exhibit significant prognostic value on the cancer recurrence. All or a portion of the network is displayed on a computer display. A first node of the network represents one IHC-based feature of the set of IHC-based features. A second node of the network represents one gene expression feature of the set of gene expression features. An edge of the network that extends between the first node and the second node represents one bivariate prognostic feature of the set of bivariate prognostic features.

Further disclosed is an analysis and display system that generates and displays a score indicative of whether cancer will recur in a patient. In a learning phase, tumor tissue from each one of many patients is obtained and analyzed. For each of these patients, it is known whether the patient suffered a recurrence of cancer, and this cancer recurrence information is loaded into the system. A phenomic feature of the tumor tissue is measured and a corresponding phenomic feature is defined. The phenomic feature may be measured through image analysis of digital images taken of tissue slices stained with IHC-based stains. A genomic feature of the tissue is also measured. This may entail obtaining a probe count indicative of a degree of expression of a particular gene. A bivariate feature is then defined using both the phenomic and genomic information, wherein at least one of the phenomic feature and the genomic feature is a univariate prognostic feature for the recurrence of the cancer. In this way, many phenomic and genomic features can be measured. A bivariate feature can be defined for the relationship between any two of the phenomic and genomic features, wherein at least one of the two features is a univariate prognostic feature for the recurrence of the cancer. A bivariate feature for the relationship between each pair of features is defined. Once all this information has been collected and all the features have been calculated and defined, the system employs a thinning method to eliminate those features that do not have a substantial prognostic value in predicting the recurrence of cancer in a patient. The result of this elimination of unimportant features is a Minimal Spanning Tree (MST). The MST is a network (also called a graph). It includes the features of substantial prognostic importance.

Some of the nodes (bubbles) of the MST network represent phenomic features, and others of the nodes represent genomic features. The edges (arrows) between nodes represent bivariate features. Some of the edges represent bivariate features that are based on both phenomic feature information and genomic feature information. The method of using phenomic feature information along with genomic feature information in the prediction of cancer recurrence allows additional feature measurements to be brought to bear in the determination of the score, as compared to methods that only use genomic information.

A user of the system can cause a rendering of the MST network to be displayed on the display of the system. The nodes of univariate features that have more prognostic importance are rendered to be larger, whereas the nodes of univariate features that have less prognostic importance are rendered to be smaller. Edges in the network that have more prognostic importance are rendered as thicker lines, whereas edges in the network that have less prognostic importance are rendered as thinner lines. The type of bivariate relationship (one of four "fuzzy logic" combinations) that was determined in the learning phase to have the most prognostic importance is indicated in the MST network by the type of arrow or line that is representing the edge.

In a diagnostic phase, a score is to be generated for a new patient. A diagnostic test that involves collecting information on only a relatively small number of the features is developed using the network as displayed on the system. In one example, raw measurement information on only three univariate features need be collected from the patient. One of the univariate features is a phenomic feature, and the other two univariate features are genomic features. A tissue sample is taken from the patient and both the raw phenomic data as well as the raw genomic data is obtained from the sample. From the raw measurement data, a score value for each univariate feature (each univariate feature used in the diagnostic test) is calculated. In the example in which there are three univariate features involved in the diagnostic test, raw measurement data for each of these three univariate features is obtained. From this raw data, a score value for each of the three univariate features is calculated. In addition, the raw data is used to calculate a score value for each of the two bivariate features (edges in the network) between these three univariate features. The overall score is a function of the underlying feature score values. In one example, each of the underlying score values is either a "1" (representing a "yes" vote), or a "0" (representing a "no" vote). There is one score value for each of the three univariate phenomic features, and there is one score value for each of the two bivariate features, for a total of five score values. The function is a majority voting function. The overall score is therefore a majority vote of the five votes provided by the five underlying features. The resulting overall score, which is indicative of whether cancer will recur in the patient, is then displayed on the display of the system.

Multiple different diagnostic tests can be developed by inspecting the network after the learning phase, and by selecting features that have notably high prognostic importance. The example outlined above of a test involving three univariate features and two bivariate features is presented for illustrative purposes. Although an example of the system is presented where the score is to be indicative of whether the patient will suffer a recurrence of prostate cancer, the system has general applicability. For example, the system is usable to generate a score indicative of whether a patient will suffer a recurrence of another type of cancer, such as lung cancer, or breast cancer.

**Examples**

**Example 1**

**Material and Methods**

a. Data collection

Data was collected from a cohort of 23 prostate cancer patients, each of which had a Gleason score of 6-9, a staging of pT2, and had an age of equal to or less than 80 years. In 12 patients, prostate cancer recurrence, measured by PSA recurrence, was observed.

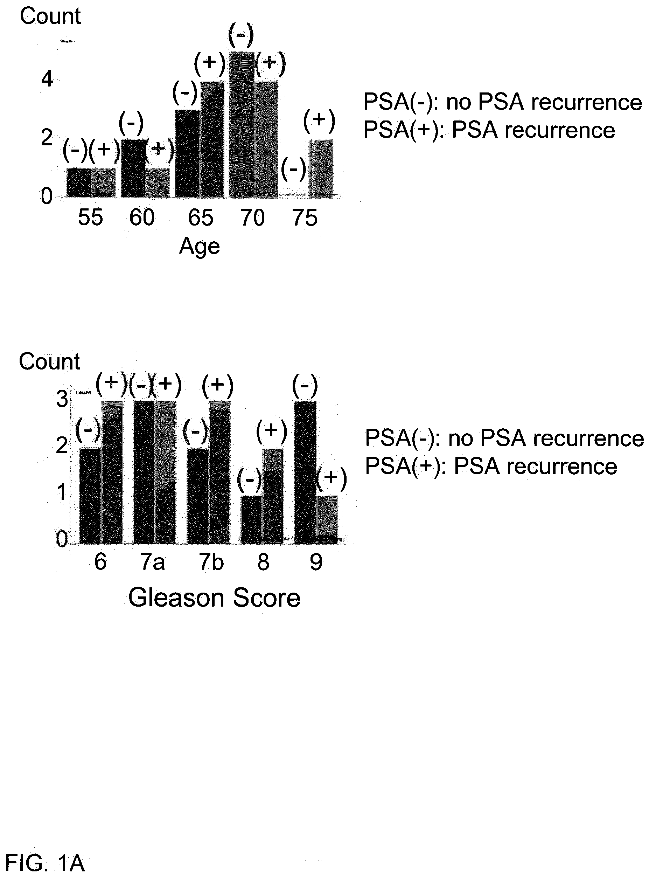

FIG. 1A shows the distribution of age and Gleason score of the patients. The patients are divided into patients with PSA recurrence and patients without PSA recurrence. The columns marked with (+) represent patients with PSA recurrence. The columns marked with (-) represent patients without PSA recurrence.

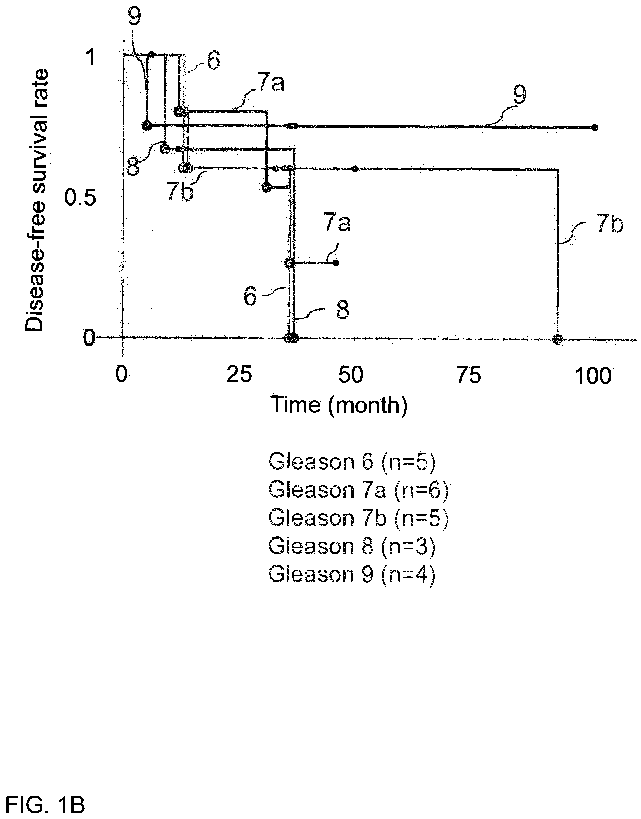

FIG. 1B shows the patients' disease-free survival rate over time depending on their Gleason score.

Clinical parameters of the patients (Gleason score, age, PSA blood value) provided no prognostic value as shown by multi-variate Cox regression analysis:

| Clinical parameter | n | HR | 95% CI | p-value |
|---|---|---|---|---|
| Gleason score 6 | 5 | | | |
| Gleason score 7a | 6 | 0.369 | 0.034 - 4.028 | 0.414 |
| Gleason score 7b | 5 | 0.319 | 0.033 - 3.062 | 0.322 |
| Gleason score 8 | 3 | 0.695 | 0.065 - 7.459 | 0.764 |
| Gleason score 9 | 4 | 0.160 | 0.116 - 418.9 | 0.149 |
| Age | 23 | 1.062 | 0.914 - 1.234 | 0.433 |
| PSA blood value | 23 | 1.016 | 0.984 - 1.049 | 0.325 |

All patients underwent radical prostatectomy. From the resected prostate of each patient, one formalin-fixed paraffin-embedded (FFPE) tissue block comprising tumor tissue was generated for this study. The tissue block was sectioned for IHC and gene expression analysis. The data was obtained from whole slide images.

b. IHC-based analysis

From each tissue block, three consecutive sections were processed with dual stains: CD3/CD8 visualizing T-cells/cytotoxic T-cells; CD68/CD163 visualizing all-macrophages/M2-polarized macrophages; and CK18/p63 visualizing epithelial cells/cells of the basal membrane. One additional fourth section was processed with a single stain: CD34 visualizing endothelial cells (vessels).

Normal prostate glands are characterized by CK18 (cytokeratin 18)-positive luminal cells with at least one p63-positive adjacent basal cell. All other CK18-positive cells are considered as "tumor" cells. For subsequent data mining, potentially prognostic image-based features (digital measurements) such as densities, ratios, and distances of stain-positive cells in specific regions of interest across differently stained sections after co-registration were extracted. The relevant regions of interest were:

- tumor as defined above;

- stroma within a predefined distance to the tumor.

The stroma is defined by a threshold-based segmentation of stroma using a distance-from-tumor map with a 150 $\mu$m threshold. Other stroma is defined as tissue excluding normal glands.

In total, 462 features were computed.

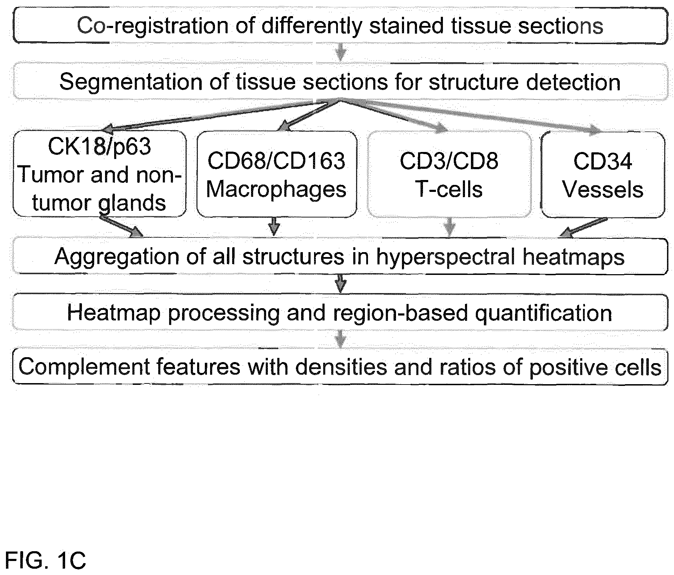

FIG. 1C shows an overview of the immuno-phenomics workflow, i.e. the workflow of the IHC-based analysis.

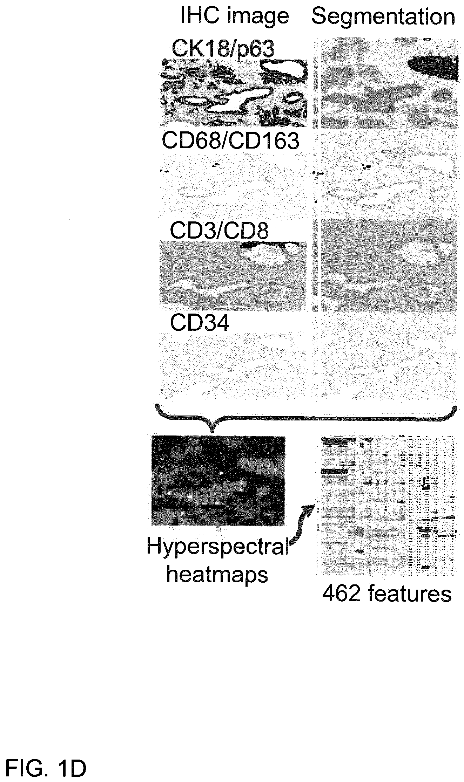

FIG. 1D shows IHC images obtained from tissue sections stained with CK18/p63, CD68/CD163, CD3/CD8 and CD34, respectively (left column), and their segmentation for structure detection (right column). FIG. 1D further shows a hyperspectral heatmap obtained by aggregation of all structures and a table with data on the 462 features that were computed.

For determining the average distance of CD163 positive cells to CD3 positive CD8 negative cells, the distance of every CD163 positive cell on the slide to its four nearest CD3 positive CD8 negative cells is measured. The average distance is calculated from all distances measured this way.

For determining the average distance of CD34 positive cells in stroma to CD163 positive cells, the distance of every CD34 positive cell in stroma on the slide to its four nearest CD163 positive cells is measured. The average distance is calculated from all distances measured this way.

c. Gene expression analysis

Within the tumor region, tissue from a fifth section was processed using the NanoString nCounter PanCancer Immune Profiling Panel (NanoString Technologies, Seattle). The NanoString software provided quantitative measurements for 730 genes with potentially prognostic value.

The gene expression analysis provides gene expression data of 730 immune-related genes. The data is obtained from the same tissue block as the data obtained by the IHC-based analysis.

In the gene expression analysis, mRNA counts for 730 immune-related genes (nCounter PanCancer Immune Profiling Panel) are collected from sections containing tumor tissue only. This step is followed by a normalization of the absolute mRNA counts with respect to 40 housekeeping (HK) genes (nCounter Advanced Analysis Plugin). The normalization comprises the steps of

- background subtraction (geometric means of negative controls)

- multiplication with normalization factor $\mu_{baseline}(HK)/\mu(HK,patient)$, wherein

   $\mu(HK,patient)$ is the geometric mean of 40 selected stable housekeeping genes (geNorm; Vandesompele J et al. (2002) Accurate normalization of real-time quantitative RT-PCR data by geometric averaging of multiple internal control genes. Genome Biol., vol. 3, no. 7) and

   $p_{baseline}(HK)$ is the arithmetic mean of $\mu(HK,patient)$ over all patients

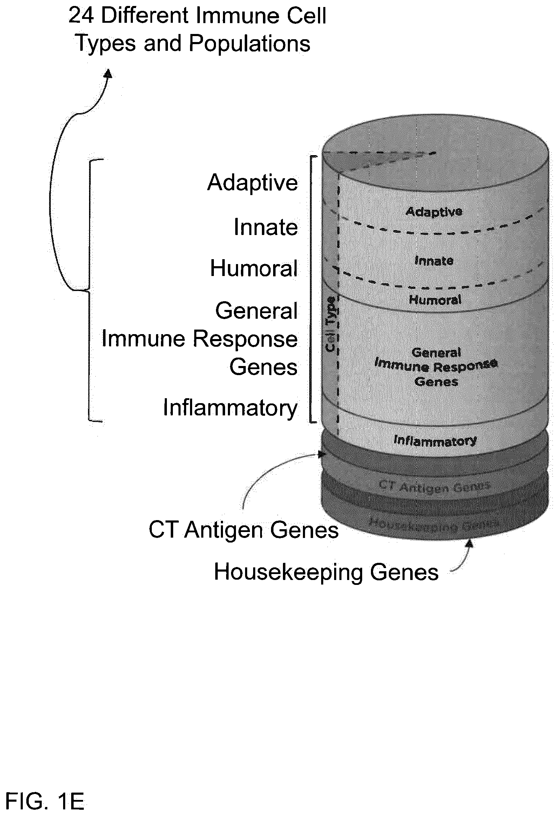

FIG. 1E shows an overview of the roles of the proteins that are encoded by the genes covered by the gene expression analysis (source: www.nanostring.com). The immune system-related proteins cover the following immune response categories:

B Cell Functions
T Cell Functions
NK Cell Functions
Antigen Processing
Adhesion
Cell Cycle
Cell Functions
Chemokines
Complement
Cytokines
Cytotoxicity
Interleukins
Leukocyte Functions
Macrophages Functions
Microglial Functions
Pathogen Defense
Senescence
TLR
TNF Super Family
Transporter Functions

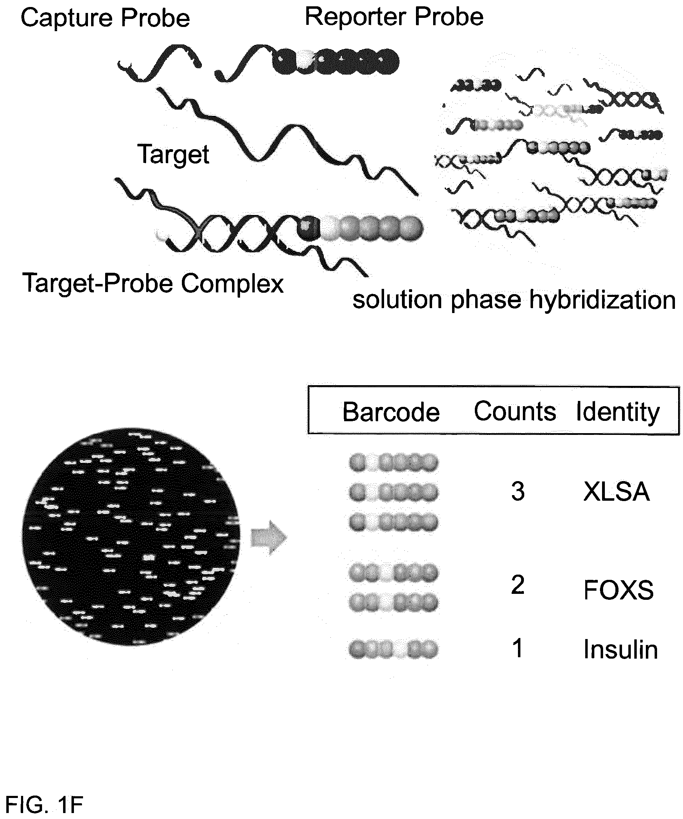

FIG. 1F shows an overview of the workflow of the gene expression analysis using the NanoString nCounter PanCancer Immune Profiling Panel (source: www.nanostring.com). Two probes, namely a Capture Probe and a Reporter Probe, hybridize (i.e. attach) directly to a target molecule in solution. The Reporter Probe carries the fluorescent barcode and the Capture Probe contains a biotin moiety that immobilizes the hybridized complex for data collection. The target molecule is the mRNA of interest. After hybridization, samples are transferred to an nCounter instrument which removes excess probes. Purified target-probe complexes are bound, immobilized and aligned on the imaging surface of the nCounter cartridge. Sample cartridges are scanned by an automated fluorescence microscope. Barcodes are counted for each target molecule and the data are exported as a simple CSV file.

d. Feature normalization

To integrate the IHC-based features and the gene expression features, all features were normalized by the rank percentage [0...1] in the study (rank normalization). The rank percentages are computed by sorting the values of each feature in the study from smallest to largest value, and then replacing the feature value by (position_in_the_sorted_list - 1)/(number_of_samples-1).

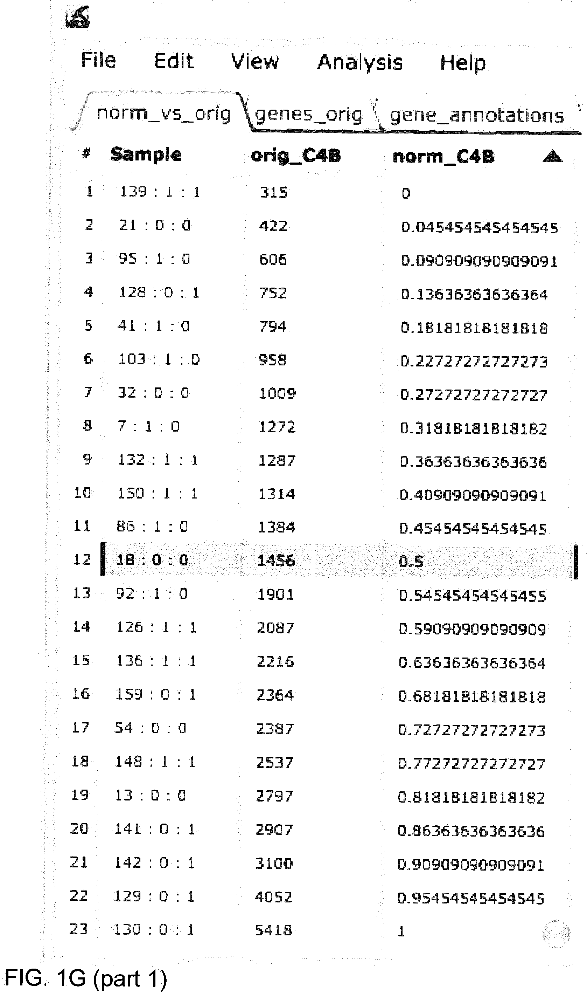

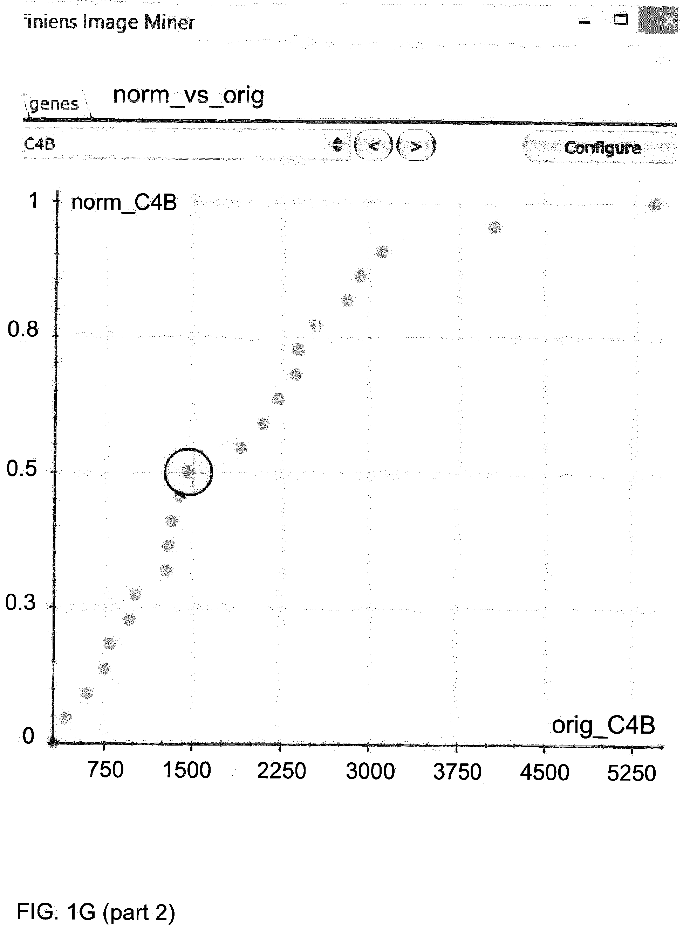

As an example, the normalization of the C4B gene expression measurement is shown in FIG. 1G. Part 1 of FIG. 1G shows original NanoString C4B gene expression measurements (column "orig_C4B") at side with the normalized values (column "norm_C4B") for all 23 patients. Part 2 of FIG. 1G shows the scatterplot of original vs. normalized C4B gene expression measurements.

To determine the rank percentage of a feature value of a new patient, the curve provided by the original feature values versus normalized feature values (rank percentages) of this study was interpolated. For example, using linear interpolation, if a new patient has a C4B feature value of 1678, then the normalized feature value becomes 0.522.

### e. Univariate feature selection

For univariate feature selection, for every IHC-based and gene expression feature, and every potential feature value cut point within a quantile range of 40% to 60%, the negative logarithm (base 10) of the Kaplan Meier log-rank test p-value (log-p-value) using the clinical recurrence data was computed. Those features with a mean log-p-value greater than $-\log10(0.05)$ were considered to be prognostic.

### f. Bivariate feature selection

For bivariate feature selection, all significant univariate features (f1) were combined with all available features (f2). To combine the feature f1 with the feature f2 into a single (potentially prognostic) feature f12, four (fuzzy) logical combinations of f1 and f2 were chosen:

- f1 and f2: $f12 = f1 * f2$
- not f1 and f2: $f12 = (1-f1) * f2$
- f1 and not f2: $f12 = f1 * (1-f2)$
- not f1 and not f2: $f12 = (1-f1) * (1-f2)$

The feature f12 is also designated combination or score.

The selection of significant combinations (scores) was equivalent to the univariate feature selection (selection of scores having a significant ($p<0.05$) mean Kaplan Meier log-rank test p-value within a range of 5 cut-points), with the additional requirement that the log-rank test p-value of the combination f12 is at least a factor 10 smaller than the smallest log-rank test p-value from the univariate analysis of f1 or f2. In this way, only those bivariate models which provide significant benefit compared to the univariate components are kept.

This analysis renders 9746 feature combinations f12 as significant. To reduce the number of prognostic features by another step, based on graph theory, a minimal spanning tree (MST) of the network of bivariate features was constructed using a modified version of Prim's algorithm.

First, all significant bivariate features are sorted according to their log-p-value (descending). The most significant bivariate feature is selected (first in the sorted list) to constitute the basis of the MST. Then the bivariate feature list is iterated from top to bottom, and any bivariate feature f12 is added to the MST if at least f1 or f2 is not yet part of the MST. Additionally, features f12 are added if they are part of the top 75% quantile of all bivariate features.

The MST network is then shown on a graphical user interface using a tree layout algorithm (Graphviz sfdp software).

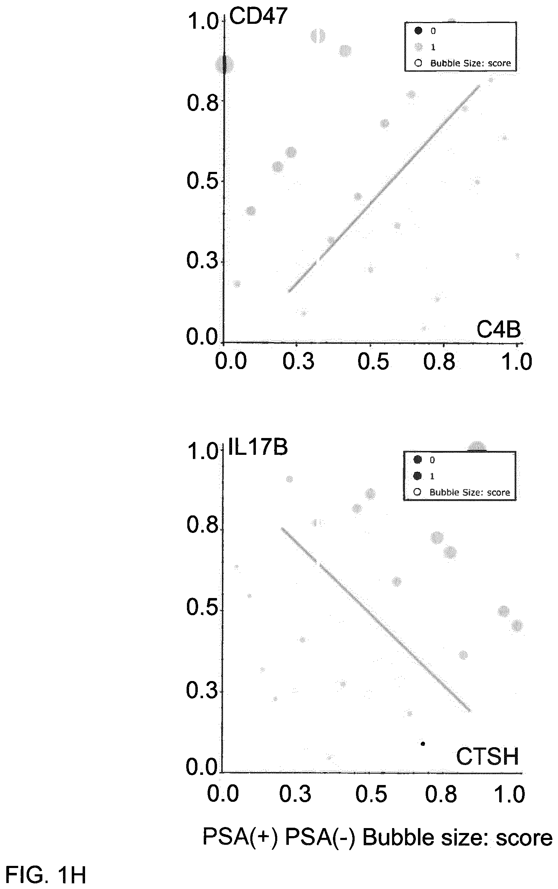

As examples for bivariate feature selection, tests for the combination of C4B with CD47 and for the combination of CTSH with IL17B are shown in FIG. 1H. Lighter bubbles ("0" in the figure legend) correspond to patients without PSA recurrence. Darker bubbles correspond to patients with PSA recurrence ("1" in the figure legend). The bubble size indicates the score. Both combinations were found to provide suitable bivariate features that provide high prognostic value for PSA recurrence prediction (see Results, Table 2, bivariate features no. 01 and no. 04).

### Results

### Univariate features predicting PSA recurrence

Using the method described above, 3 phenomic (IHC-based) and 29 gene expression features, which provide significant prognostic power to predict prostate cancer recurrence, were discovered.

These univariate features are shown in Table 1. A positive sign behind the feature indicates that high features values are favorable for the patients, and the risk of PSA recurrence is low. Accordingly, a negative sign behind the feature indicates that high features values are unfavorable for the patients, and the risk of PSA recurrence is high.

Table 1:

| CD59- | C4B+ |
|-------|------|

(continued)

| MAGEC2- | IHC_Dist_CD163(+)_CD3(+)CD8(-)+ (Average distance of CD163(+) to CD3(+)CD8(-) cells on whole slide) |
|---|---|
| ATF2- | BMI1- |
| NFKBIA- | CCL5- |
| CXCL13+ | STAT4- |
| NUP107- | IRF1- |
| IL12A- | CD47- |
| JAK2- | PSEN2- |
| SYCP1- | CD96- |
| IHC_Dist_CD34(+)_CD163(+)_stroma+ (Average distance of CD34(+) cells to CD163(+) cells in stroma) | CTSH- |
| MAPK1- | IL17B- |
| TBX21- | DDX43- |
| SYK- | KLRK1- |
| ANXA1- | IHC_Dist_CD163(+)_CD8(+)_stroma+ (Average distance of CD163(+) cells to CD8(+) cells in stroma) |
| PMCH- | LGALS3- |
| MBL2- | PTGDR2- |

The phenomic features are related to the distance of CD163(+) cells (associated with M2 macrophages) to CD3(+) cells (associated with T-cells) or CD34(+) cells (associated with vessels). The gene expression features relate to various immune system components, with C4B including the complement system.

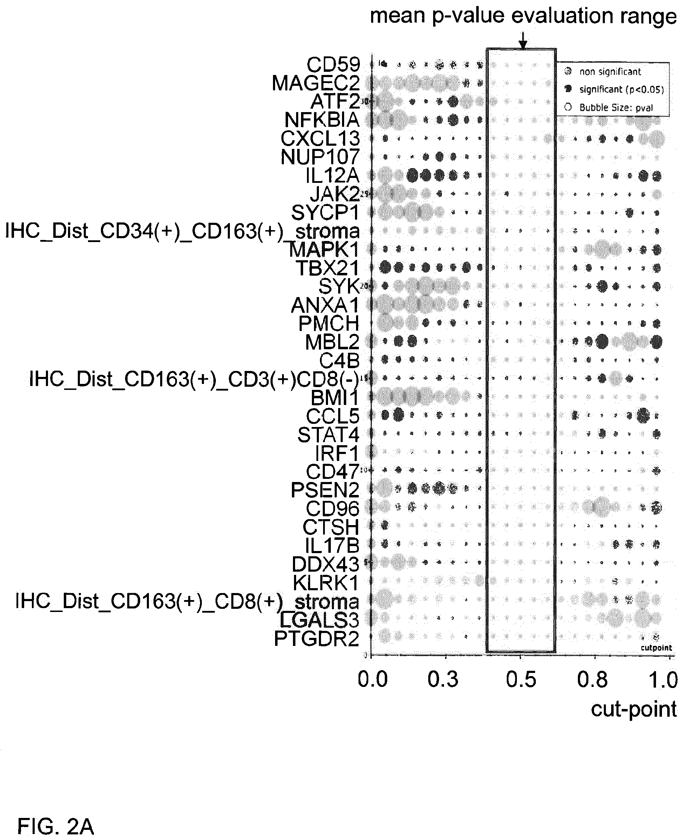

FIG. 2A shows an overview of the most prognostic univariate features including the log-p-value of the feature value cut point indicated with the bubble size. Darker bubbles correspond to non-significant log-p-values. Lighter bubbles correspond to significant log-p-values. Features having a significant ($p < 0.05$) mean Kaplan Meier log-rank test p-value within a range of 5 cut points were selected (box in the center of the figure). In other words, the box in the center of the figure shows the log-p-values that were used for mean calculation to select the features (see methods).

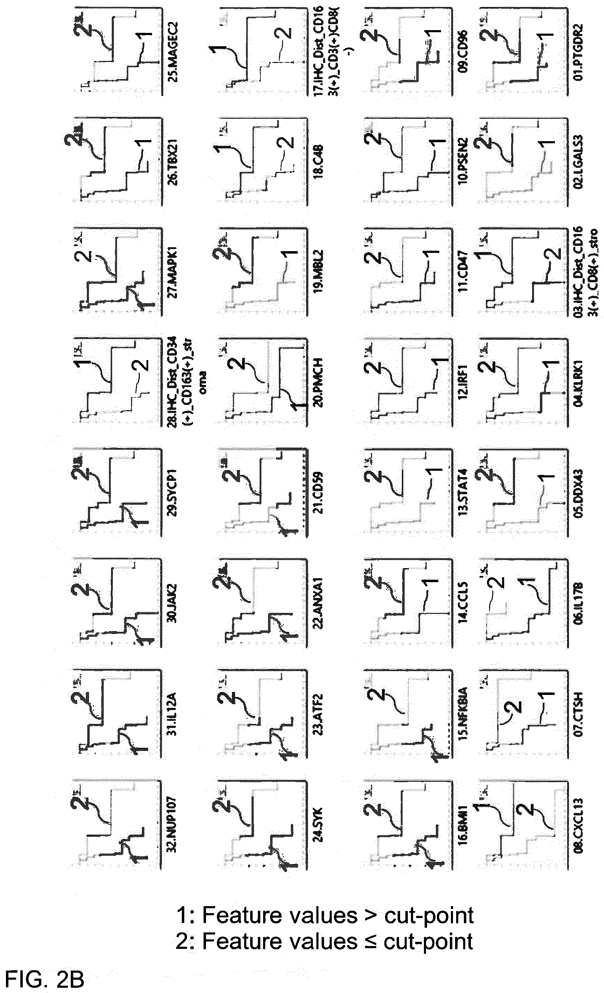

FIG. 2B shows an overview of the Kaplan-Meier curves of all 32 discovered prognostic univariate features. The features represent the best univariate models to predict PSA recurrence.

**Bivariate features predicting PSA recurrence**

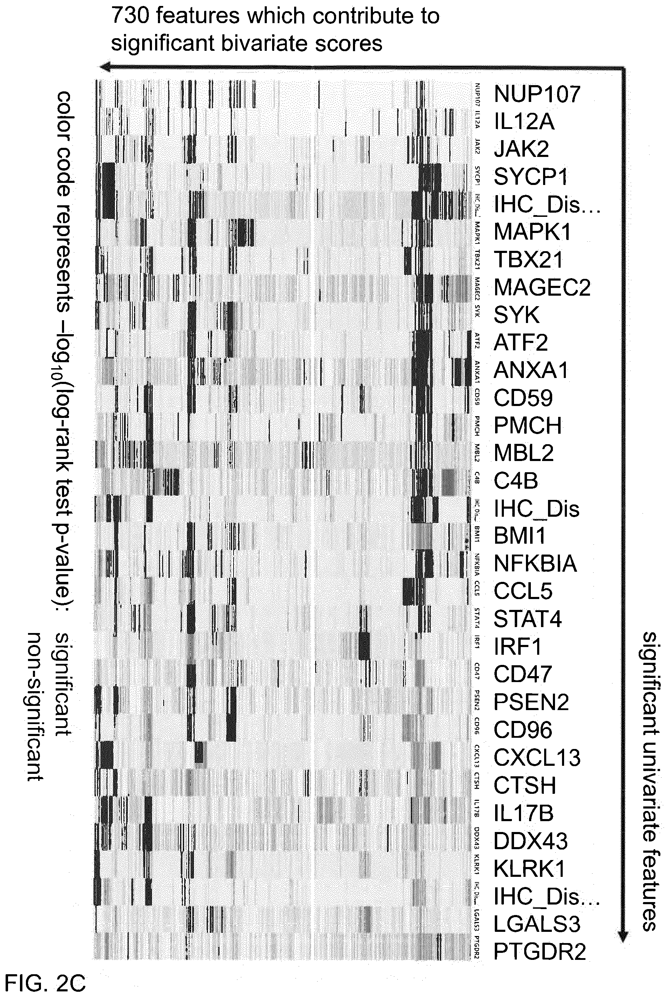

FIG. 2C shows a matrix of the 9746 significant bivariate p-values obtained by the bivariate feature selection described above (see methods).

A network of 22 bivariate features, which provide high prognostic value for PSA recurrence prediction, were extracted (see methods).

These 22 bivariate features are shown in Table 2. A positive sign behind the feature indicates that high features values are favorable for the patients, and the risk of PSA recurrence is low. Accordingly, a negative sign behind the feature indicates that high features values are unfavorable for the patients, and the risk of PSA recurrence is high.

Table 2:

| No. | Bivariate feature |
|---|---|
| 01 | not C4B and CD47 - |
| 02 | not DDX43 and not PSEN2 + |
| 03 | LGALS3 and MAGEC2 - |
| 04 | CTSH and IL17B - |
| 05 | LGALS3 and not IL6R - |

(continued)

| No. | Bivariate feature |
|-----|-------------------|
| 06 | IRF1 and not IL6R - |
| 07 | LGALS3 and not ISG20 - |
| 08 | not CTSH and MAGEA4 + |
| 09 | not C4B and IRF1 - |
| 10 | CD47 and MAGEC2 - |
| 11 | IL17B and SYCP1 - |
| 12 | PSEN2 and not ISG20 - |
| 13 | not STAT4 and MAGEA4 + |
| 14 | ANXA1 and not ISG20 - |
| 15 | not TBX21 and MAGEA4 + |
| 16 | MAGEC2 and STAT4 - |
| 17 | MAGEC2 and SYK - |
| 18 | BMI1 and MAGEC2 + |
| 19 | IHC_Dist_CD163(+) CD3(+)CD8(-) and not MAGEC2 + |
| 20 | not IHC_Dist_CD34(+) CD163(+) stroma and TICAM2 - |
| 21 | ANXA1 and TICAM2 - |
| 22 | not MAPK1 and MAGEA4 + |

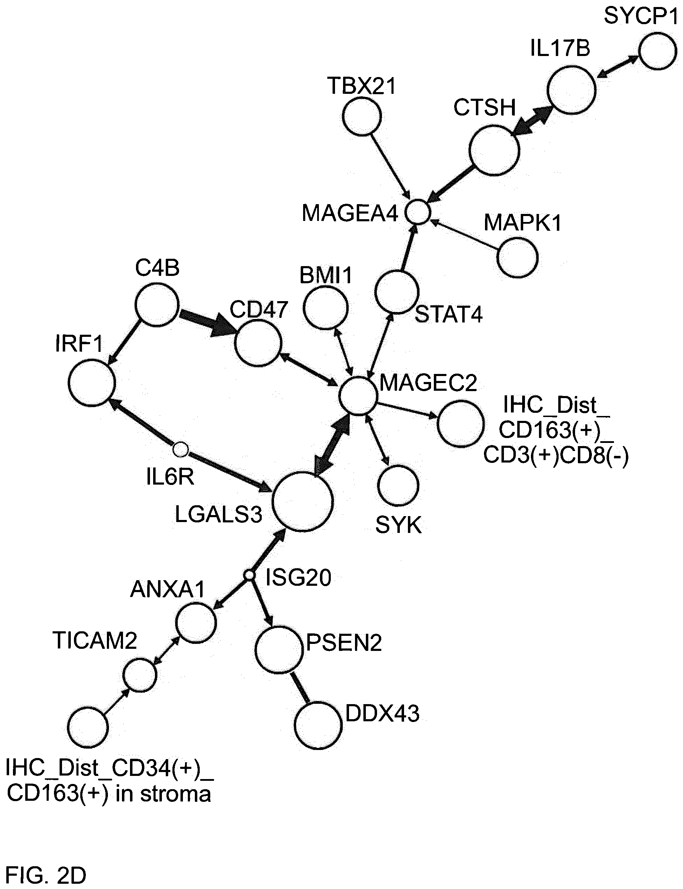

FIG. 2D shows a network including the prognostic bivariate features. The network is an extended Minimal Spanning Tree (MST) of bivariate models (bivariate features) predicting PSA recurrence, wherein the MST was extended by the most significant (75% quantile) bivariate models. The node sizes are related to the univariate prognostic value (average log-p-value). The edge widths are related to the bivariate prognostic value (average log-p-value). In other words, the node size indicates the - log10(univariate p-value) and the edge size (edge width) indicates the - log10(bivariate p-value). A missing arrow head at feature f1 indicates a "not" logical combination when computing the bivariate feature f12, e.g. "not f1 and f2" is rendered as a directed arrow from f1 to f2. Likewise, "f1 and not f2" is rendered as a directed arrow from f2 to f1, "f1 and f2" is rendered as a double-headed arrow, and "not f1 and not f2" is rendered as an arrow without any arrow head, i.e. as a simple line.

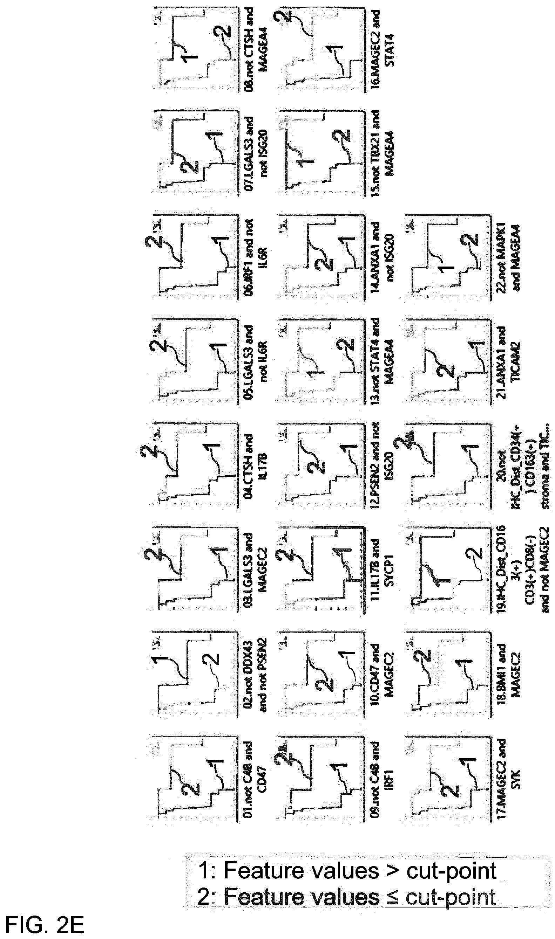

FIG. 2E shows an overview of the Kaplan-Meier curves of all 22 discovered prognostic bivariate features. These bivariate features are included in the network shown in FIG. 2D. The features represent the best bivariate models to predict PSA recurrence.

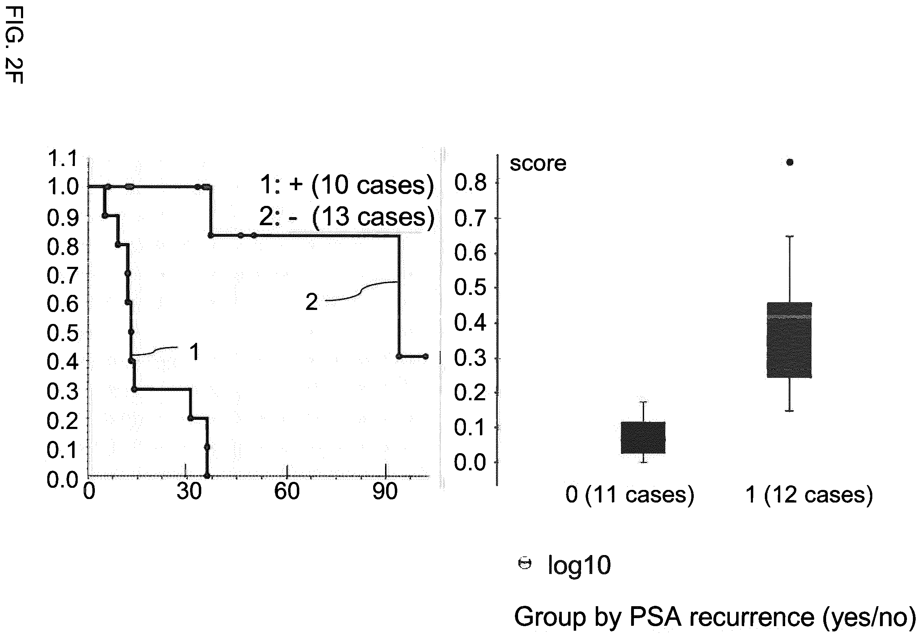

FIG. 2F shows an example stratification using the score (bivariate feature) "not C4B and CD47". A higher score indicates a higher risk of PSA recurrence as shown in the right panel (yes=1, no=0).

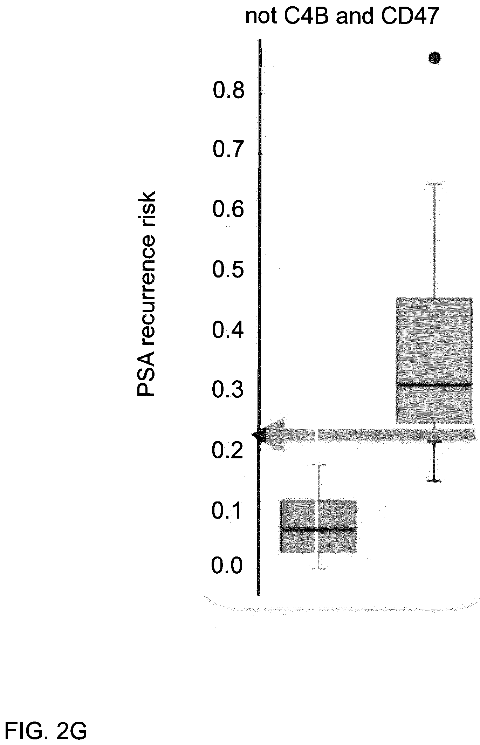

FIG. 2G shows an example of a user interface element that visualizes the PSA recurrence risk of a given patient (horizontal arrow) in the context of the results of this study using the bivariate feature "not C4B and CD47". The left box plot shows the feature values of study patients who did not show PSA recurrence. The right box shows the feature values of study patients who exhibited PSA recurrence.

The MST analysis revealed two bivariate features that are combined immuno-phenomics and gene expression models to predict PSA recurrence. One of the two bivariate features is a large distance from CD163(+) cells to CD3(+) CD8(-) cells and a low level of MAGEC2 expression (Table 2, bivariate feature no. 19). This bivariate feature is a good prognostic factor, i.e. predicts a good prognosis (no PSA recurrence). The other of the two bivariate features is a small distance from CD34(+) cells to CD163(+) cells in stroma and a high level of TICAM2 expression (Table 2, bivariate feature no. 20). This bivariate feature is a poor prognostic factor, i.e. predicts a poor prognosis (PSA recurrence).

The MST graph network further indicates that a high level of MAGEC2 expression and a high level of LGALS3 expression (Table 2, bivariate feature no. 03) is a key component of increased PSA recurrence risk.

The network of bivariate prognostic models (bivariate prognostic features) represents a novel, dual representation of gene network correlation analysis. The analysis also revealed that the combination of immuno-phenomics with gene expression measurements provides spatial context to a powerful prognostic bivariate model.

**Example 2**

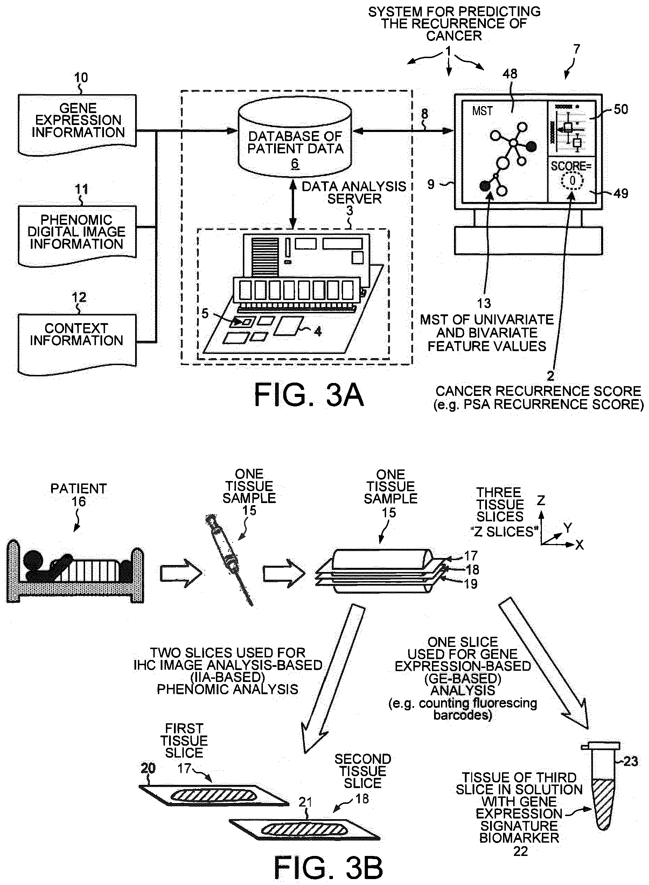

FIG. 3A is a conceptual diagram of a system 1 for predicting cancer recurrence using a prognostic method that analyzes genomic univariate features, as well as phenomic univariate features, as well as bivariate features of the two (of a genomic feature and a phenomic feature), and based at least in part on that analysis outputs a score 2. The score 2 is indicative of whether a patient will suffer a recurrence of cancer. System 1 includes a data analysis server 3. The server 3 has a processor 4 that executes system software 5. The software 5 is stored on the server 3 in a non-transitory processor-readable medium, such as semiconductor memory and/or magnetic disk storage. The server 3 also maintains, and/or provides access to, a database 6 of patient data. The database 6 may be stored on the server 3, or it may be stored remotely such that it is accessible to the server 3. The system 1 further includes a computer 7. The computer 7 is coupled to the server 3, for example by one or more networks or network connections 8. Computer 7 includes a keyboard (not shown) and a display 9. A user of the system 1 uses the computer 7 to enter information into the system 1. Information that the user can enter includes genomic feature information 10, phenomic feature information 11, and context information 12. The genomic feature information can be a set of counts, where each count indicates the degree of expression of a corresponding gene in the tissue of a cancer patient. The phenomic feature information 11 can be digital images taken of tissue of the cancer patient. In addition to this genomic and phenomic information about a patient, context information 12 for the patient is also loaded into the database 6. The context information 12 for a patient includes information about the patient including clinical cancer recurrence data. For each patient of a plurality of patients, the system user uses computer 7 to cause genomic information, phenomic information, and context information to be loaded into the system 1 so that the information is stored in the database 6. In addition to the patients mentioned above, there is also a patient who is being seen in the clinical setting. It is for this patient that the score 2 is to be generated. The system user uses the computer 7 to cause both genomic and phenomic information about this patient to be loaded into the system 1 and to be stored in the database 6.

The system user uses computer 7 to interact with the system 1, and to view information served to the system user by server 3. The server 3 may cause this information to be displayed for viewing on the display 9 of the computer 7. An example of information that can be viewed is a Minimum Spanning Tree (MST) 13 of univariate features and bivariate features, where nodes (bubbles) of the MST represent univariate features, and where edges (interconnecting lines and arrows) of the MST represent bivariate features. The system 1, by virtue of processor 4 executing the system software 5, analyzes the genomic gene expression information and the phenomic digital image information along with the context information, and generates therefrom the score 2. The system 1 then causes the score 2 to be displayed on the display 9 of the computer 7.

One particular phenomic feature that is of particular interest in the prognostic method carried out by the system 1 of FIG. 3A is an average distance. This phenomic feature is referred to as "IHC_Dist_CD163(+)_CD3(+)CD8(-)". IHC-based staining and image analysis are used to identify CD163(+) stained objects in a tissue sample. These objects correspond to M2-type macrophages. IHC-based staining and image analysis is also used to identify CD3(+)CD8(-) objects in the same tissue sample. These objects correspond to non-cytotoxic T-cells in the tissue sample. Each CD163(+) object is then considered, and for that CD163(+) object the average distance (in micrometers) between it and the four nearest identified CD3(+)CD8(-) objects is determined. The average of all these averages for all the CD163(+) objects in the tissue sample is then determined, and this overall average is the raw measurement value for the IHC_Dist_CD163(+) _CD3(+)CD8(-) feature. Additional detail on how this raw measurement value is determined is set forth below.

In addition to analyzing "phenomic features", the system 1 of FIG. 3A also analyzes "genomic features". In one example, a device called nCounter and gene-specific probe pairs of reporter probes and capture probes is used. The nCounter device and the gene-specific probe pairs are commercially available from NanoString Technologies, Inc., of Seattle, Washington. The nCounter device has a high-resolution CCD camera. After the pair of probes have hybridized to their target mRNA molecules in the tissue sample, excess probes (unattached probes) in the solution are removed. The remaining probe/mRNA complexes are then aligned and immobilized via the capture probes. The nCounter device illuminates the probe/mRNA complexes and uses its high-resolution CCD camera to perform optical examination of the probes. In this way, the reporter probe on each individual target mRNA molecule is identified by its barcode to be a reporter probe of a particular type, and the count of this particular reporter probe type is incremented. After all the reporter probes in the sample have been detected and counted in this way, the nCounter device outputs a digital file. Such a digital file is an example of the genomic information 10 (gene expression information 10) that is loaded into the system 1 of FIG. 3A. This digital file includes a count value. The count value indicates the number of times that a reporter probe of a particular type (bearing a particular color-coded barcode) was detected in the sample.

In a specific example of the prognostic method carried out by the system 1 of FIG. 3A, there are two probe pairs that are of particular interest. The first probe pair is usable with the nCounter device to measure the gene expression of the LGALS3 gene. The LGALS3 gene is located in chromosome 14, locus q21-q22. The second probe pair is usable with the nCounter device to measure the expression of the MAGEC2 gene. The MAGEC2 gene is located in chromosome Xq27.2.

The prognostic method carried out by the system 1 of FIG. 3A has a "learning phase" and a "diagnostic phase". In the learning phase, both genomic feature information as well as phenomic feature information from each patient of a plurality of patients is generated, and then analyzed by the system. For each of these patients, information on many different genomic features and on many different phenomic features are typically collected and loaded into the system. The result of the learning phase is that the system 1 has information that is usable to generate a score 2 for a particular new patient in the later "diagnostic phase". This score 2 can be generated for the new patient without having to load but a small amount of genomic feature information and but a small amount of phenomic feature information for the new patient. Based on this relatively small amount of information, the system 1 can generate the score 2. The score 2 indicates whether the new patient will likely suffer cancer recurrence.

FIG. 3B is a diagram that illustrates the "diagnostic phase" operation of the system 1. A tissue sample 15 is obtained (for example, by biopsy) from the new patient 16. The new patient 16 is the patient for whom the score 2 is to be generated. The tissue sample 15 is then sliced into very thin slices. Some of the slices are used to generate phenomic digital image information 11 that is supplied to the system 1 in the diagnostic phase for the new patient. Others of the slices are used to generate gene expression information 10 that is supplied to the system in the diagnostic phase for the new patient. In the illustration of FIG. 3B, the first tissue slice 17 is stained with first pair of IHC stains and is put on a first slide 20. A first high-resolution color digital image of the slice 17 is taken and is supplied as first digital image information to the system. The second tissue slice 18 is stained with another pair of IHC stains and is put on a second slide 21. A second high-resolution color digital image of the slice 18 is taken and is supplied as second digital image information to the system. The digital image information derived from slices 17 and 18 is the raw measurement data used by the system 1 to generate a raw measurement value for the phenomic univariate feature for the new patient. The third tissue slice 19 is used for gene expression-based genomic analysis. The tissue of the third slice 19 is lysed and a first gene-specific probe pair for a first gene is attached, and a second gene-specific probe pair for a second gene is attached. In the specific example set forth below, the first gene is the LGALS3 gene and the second gene is the MAGEC2 gene. The resulting material 22 in the sample capsule 23 is processed by the nCounter device mentioned above, thereby generating a first count indicative of the degree of gene expression of the first gene, and a second count indicative of the degree of gene expression of the second gene. A digital computer file that records these counts is output from the nCounter device and is supplied to the system 1 of FIG. 3A as the gene expression information 10. These counts, as they are recorded in the digital file, are the raw measurement values for the genomic univariate features for the new patient. As is explained in further detail below, the system 1 generates a phenomic univariate feature score value (using the digital image information from the first and second digital images), generates a pair of genomic univariate feature score values (using the count data as output by the nCounter device), and further generates a pair of bivariate feature score values (each bivariate score value is based on both phenomic feature information and on genomic feature information). Based at least in part on these three univariate feature score values and on these two bivariate feature score values, the system 1 generates the overall score 2. The overall score 2 is then displayed on the display 9 of the computer 5.

The "learning phase" of the prognostic method is explained in further detail below by way of an example. In the example, there are twenty-three patients from whom a substantial amount of genomic information and a substantial amount of phenomic information is collected in the learning phase. For each of these twenty-three patients, the clinical cancer recurrence of the patient is known. Namely, whether the patient actually suffered a recurrence of cancer is known and this information is stored as part of the context information for the patient. In addition, if the patient did suffer such recurrence, then the date of that recurrence is known. This information is also stored as part of the context information 12 for the patient.

From each one of these twenty-three patients, a tissue sample is obtained. The resulting tissue sample block is sliced into numerous tissue slices. Some of these tissue slices are used to make raw measurements of various different phenomic univariate features. Information on many different phenomic univariate features is obtained. Others of the tissue slices are used to make raw measurements of various different genomic univariate features. Many different gene-specific probe pairs are employed to obtain gene expression information for many different genes. For a given feature, all the raw measurements for that feature are normalized. One normalization method that can be used is rank percentage normalization. It involves sorting the raw measurement values of the feature from smallest to largest, and then replacing each raw measurement value by a corresponding rank value equal to: (position_in_the_sorted_list - 1)/(number_of_samples - 1). Then, using these normalized rank values, as well as the known clinical cancer recurrence information for the corresponding patients, a Kaplan-Meier plot analysis is performed for every possible cut-point within a quantile range of 40% to 60%. This results in a Kaplan-Meier log-rank test p-value for every cut-point. The -log of each p-value is determined, thereby generating a set of -log-p-values. A univariate feature that has a mean of these -log-p-values greater than $-\log10(0.05)$ is considered to be prognostic. The cut-point having the largest -log-p-value is determined to be the cut-point for the univariate feature, and univariate feature value is the -log-p-value determined for that cut-point.

Next, the normalized rank values for each significant univariate feature are combined with the normalized rank values of each other available feature in order to calculate a bivariate feature value (a "-log(p-value)" for the bivariate feature). To combine a first feature (denoted f1) with a second feature (denoted f2) in order to obtain a single (potentially prognostic) bivariate feature (denoted f12), four fuzzy logical combinations of the corresponding rank values of the two features are calculated. Those four fuzzy logical combinations are: 1) f12 = f1*f2 (denoted "f1 and f2" of "SM1"); 2) f12 = (1-f1)*f2 (denoted "not f1 and f2" or "SM2"); 3) f12 = f1*(1-f2) (denoted "f1 and not f2" or "SM3"); and 4) f12=(1-f1)*(1-f2) (denoted "not f1 and not f2" or "SM4"). The determination of which fuzzy logical combination is considered significant is the same as the selection of significant univariate features as described above, except that here the determination of the significant bivariate features has the additional requirement that the log-rank-test p-value of the combination f12 must be at least a factor of ten times smaller than the smallest log-rank-test p-value from the univariate analysis of f1 or f2. For each bivariate feature, one of the four possible fuzzy logical combinations is determined to be the most significant, and the -log(p-value) of that combination is determined to be the bivariate feature value (the -log(p-value) for the bivariate feature).

To reduce the number of prognostic features by another step, the univariate feature values and the bivariate feature values as determined above are fashioned into a network (into a graph). The determined univariate feature values (-log(p-values)) are the bubbles (nodes) of the network. The determined bivariate feature values (-log(p-values)) are the arrows (edges) of the network.

A modified version of Prim's algorithm is then used to trim the network and thereby to obtain an extended Minimal Spanning Tree (MST). In this version of Prim's algorithm, all significant bivariate features are first sorted into descending order according to their -log(p-values). The most significant bivariate feature is then selected (first in the sorted list) to be a starting node of the MST. Then the bivariate feature list is iterated from top to end, and any bivariate feature f12 is added to the MST if at least f1 or f2 is not yet part of the MST. Additionally, f12 bivariate features are added if they are part of the top 75% quantile of all bivariate features. Any univariate features representing a bubble at an end of a bivariate feature in the tree, that is not present in the tree, is added to the tree. A tree layout method is then used to render a diagram of the extended MST. In one example, the open source graph visualization software tool called "Graphviz sfdp" is used to generate a visual rendering of the MST. The user of the system 1 of FIG. 3A can use the computer 7 to cause the rendered extended MST diagram to be displayed on the display 9 as shown in simplified form in FIG. 3A.

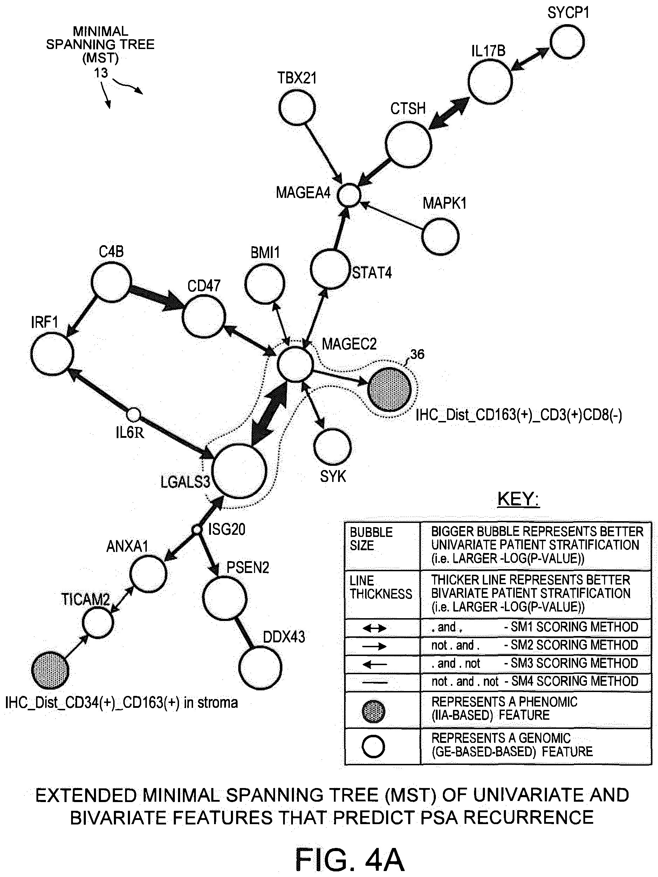

FIG. 4A is a diagram of the extended MST 13 as it is rendered on display 9 of the computer 7. In the diagram, there is a bubble (node) for each univariate feature. The size of the bubble (node) indicates the prognostic significance of the univariate feature, namely a larger bubble indicates a larger -log(p-value), whereas a smaller bubble indicates a smaller -log(p-value). The larger the bubble, the more significant the univariate feature. In the diagram, a genomic univariate feature is denoted by its corresponding bubble being unshaded, whereas a phenomic univariate feature is denoted by its corresponding bubble being shaded. Genomic univariate features are displayed so that they can be visually distinguished from phenomic univariate features. In the diagram, the thickness of the arrow (edge) representing a bivariate feature indicates the prognostic significance of the bivariate feature, namely a thicker line represents a larger bivariate feature value (-log(p-value) for the bivariate feature), whereas a thinner line represents a smaller bivariate feature value (-log(p-value) for the bivariate feature). The thicker the line, the more prognostic significance the bivariate feature has. For each bivariate feature shown, the extended MST indicates which one of the four fuzzy logic combinations it was that was considered to be the most significant. As indicated by the key at the lower right of FIG. 4A, the lack of an arrow head where an edge reaches a bubble indicates a "not" of the univariate feature represented by the bubble. A bivariate prognostic feature is considered to have significant prognostic value if the Kaplan-Meier p-value for the cohort of patients using the bivariate prognostic feature and a selected cut-point is less than 5%.

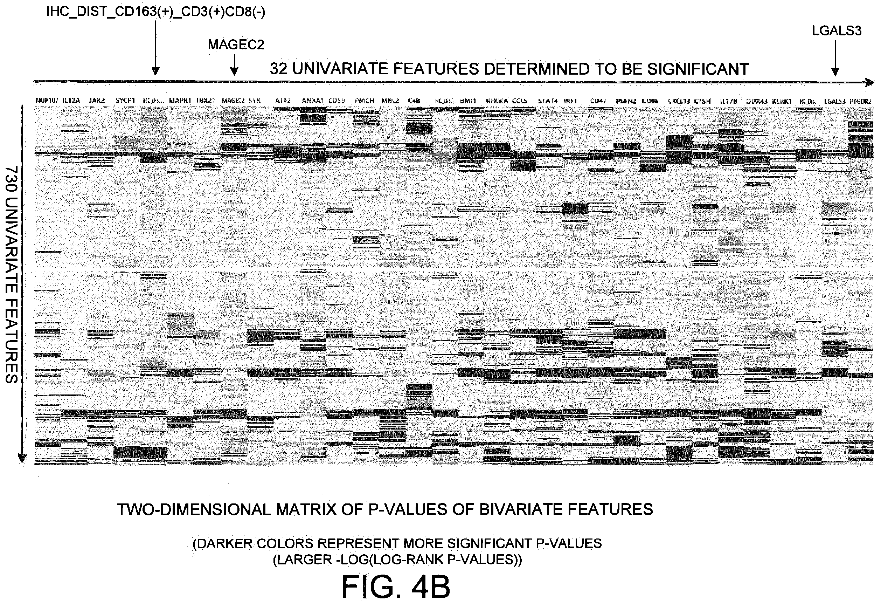

FIG. 4B is a portion of a two-dimensional matrix of the bivariate feature values. A bivariate feature value here is the -log(p-value) for the bivariate feature. The univariate features (including both phenomic and genomic features) that are determined to be significant are listed across the top of the matrix. Note that there is a column for the phenomic feature "IHC_Dist_CD163(+)_CD3(+)CD8(-)", and there is a column for the genomic feature "MAGEC2", and there is a column for the genomic feature "LGALS3". For each of the univariate features, there is also a corresponding row in the matrix. In this example, 730 univariate features were measuring in the learning phase, so there is one row in the matrix for each of the 730 univariate features. The thin rectangular intersection block of the matrix that appears in the column of one univariate feature and in the row of another univariate feature is shaded with a color. The darker the color of the intersection block of a bivariate feature, the more significant the bivariate feature is. Using the method described above, the most significant bivariate features are determined.

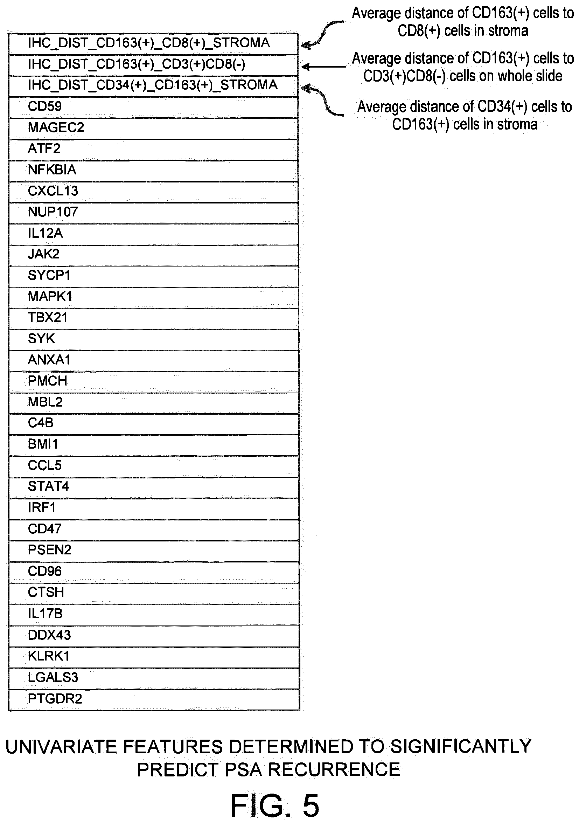

FIG. 5 is a table that sets forth the thirty-two univariate features that are determined to be most significant. A feature was determined to be significant if it had a significant (p<0.05) mean Kaplan Meier log-rank test p-value within a range of five cut-points.

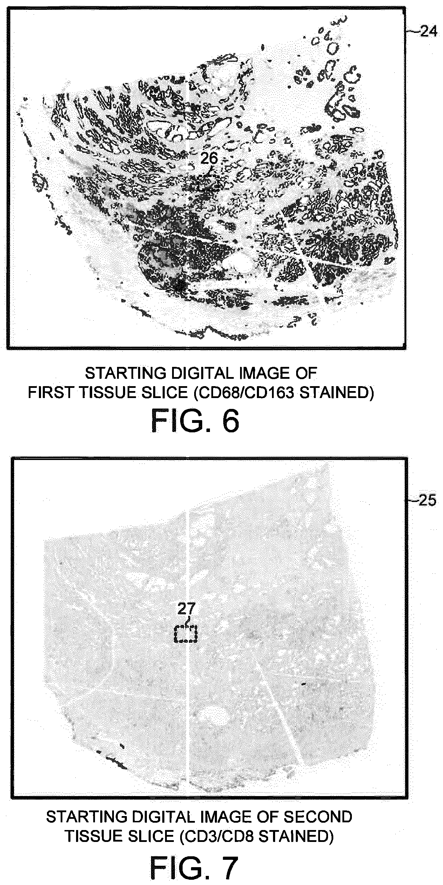

FIGS. 6-10 set forth more detail about how the "IHC_Dist_CD163(+)_ CD3(+)CD8(-)" phenomic feature raw measurement data is obtained. Two consecutive tissue slices of the same tissue sample are stained with IHC-based stains in different ways. The first tissue slice is duplex-stained with a CD68 antibody stain and a CD163 antibody stain. The CD68 stain may, for example, be a stain referred to as #M087601-2, available from Dako North America, Inc., 6392 Via Real, Carpinteria, California 93013. The CD1623 stain may, for example, be a stain referred to as #760-4437, available from Ventana Medical Systems, Inc., 1910 Innovation Park Drive, Tucson, Arizona 85755. Due to this double staining, individual tumoricidal M1 type macrophages appear red when the slice is viewed under magnification, and individual tumorigenic M2 type macrophages appear brown when the slice is viewed under magnification. After staining, the slice is placed on a slide. A high-resolution color digital image 24 is then taken of the stained slice. FIG. 6 is a grayscale version of the high-resolution digital image 24.

The second tissue slice is also duplex stained, but this slice is stained with a CD3 antibody stain and a CD8 antibody stain. Due to this double staining, individual non-cytotoxic T-cells appear red when the slice is viewed under magnification, and individual cytotoxic T-cells appear brown when the slice is viewed under magnification. After staining, the slice is placed on a slide. A high-resolution color digital image 25 is then taken of the stained slice. FIG. 7 is a grayscale version of the high-resolution digital image 25.

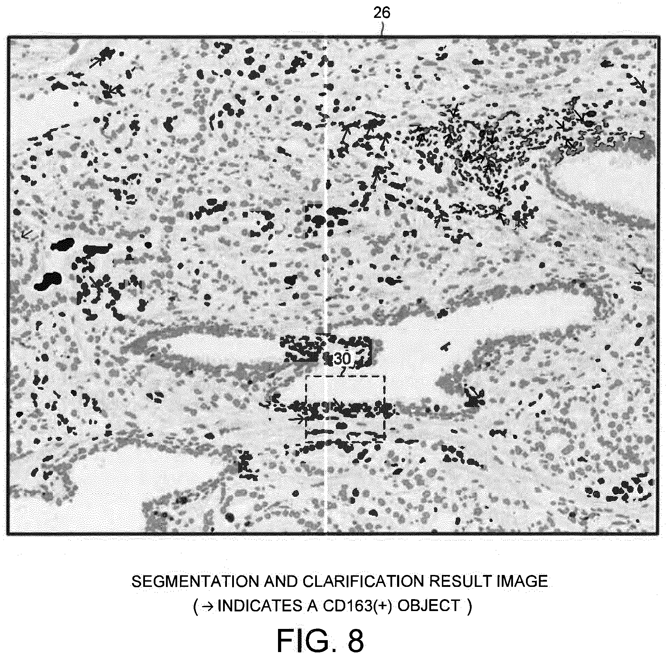

FIG. 8 is an expanded view of a portion 26 of the first digital image 24 of FIG. 6. The system 1 performs image analysis on the first digital image, thereby identifying CD163(+)-stained objects. The arrows in FIG. 8 identify the CD163(+)-stained objects. The location in the X-Y dimension of the center of each detected CD163(+) object is logged. The CD163(+)-stained objects of FIG. 8 correspond to M2 macrophages.

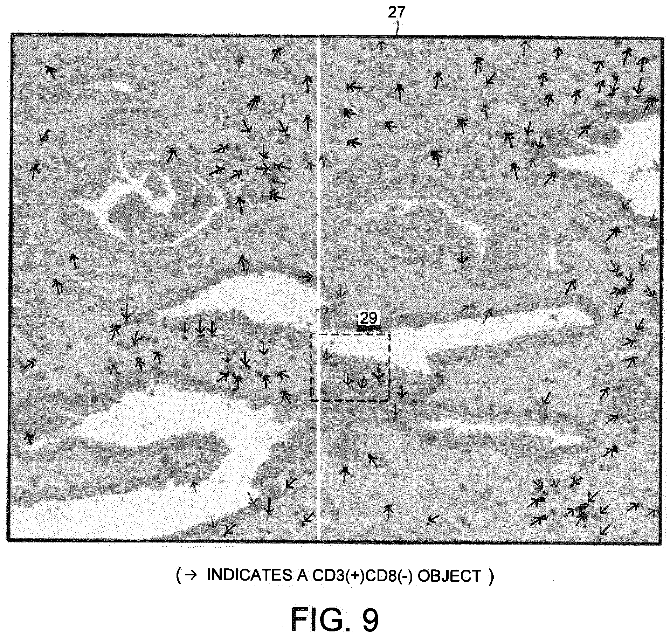

FIG. 9 is an expanded view of a portion 27 of the second digital image 25 of FIG. 7. The portion 27 of the second digital image 25 is same X-Y dimension as is the portion 26 of the first digital image 24. The tissue represented in the two portions 27 and 26 are, however, slightly offset in the Z dimension. The system 1 performs image analysis on the second digital image 25, thereby identifying CD3(+)CD8(-) objects. The arrows in FIG. 9 identify the identified CD3(+)CD8(-) objects. The location in the X-Y dimension of the center of each detected CD3(+)CD8(-) object is logged. Individual CD3(+)CD8(-) objects correspond to individual non-cytotoxic T-cells.

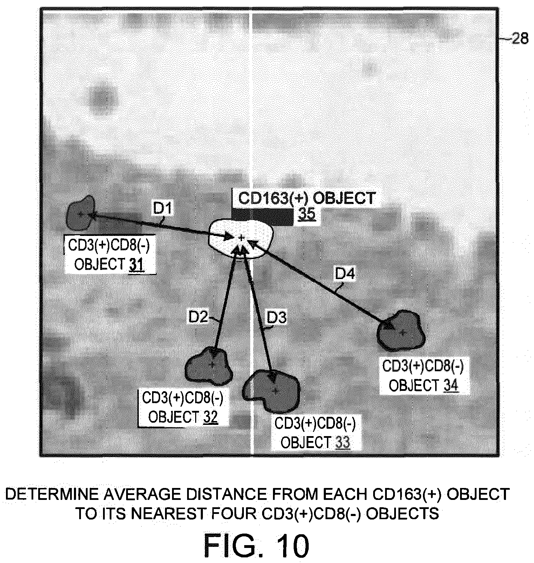

FIG. 10 is an illustrative diagram that shows how the average distance from a CD163(+) object to its nearest four CD3(+)CD8(-) objects is determined. The X-Y image block portion identified by reference numeral 28 in FIG. 10 represents the same X-Y block of tissue as does block 29 in FIG. 9 and as does block 30 in FIG. 8. The CD3(+)CD8(-) objects 31-34 are determined to be the four such objects that are the closest (in the X-Y dimension) to the CD163(+) object 35. The distances D1, D2, D3 and D4 are determined from the logged center locations of the CD3(+)CD8(-) objects 31-34 and the CD163(+) object 35. The average of the distances D1, D2, D3 and D4 in micrometers is determined and recorded. This process is repeated for all the CD163(+) object detected in the first digital image 24. All these averages are in turn averaged in order to obtain one overall average. This one overall average number (in units of micrometers) is the "IHC_Dist_CD163(+)_CD3(+)CD8(-)" phenomic feature raw measurement value for the patient from whom the first and second tissue slices were taken.

Inspection of the extended MST 13 of FIG. 4A indicates that a phenomic univariate feature can be advantageously used in the diagnostic phase along with two genomic univariate features, and along with two associated bivariate features, to generate the score 2. The dashed line 36 in FIG. 4A encircles these five features. The MST 13 is viewable by the system user during and after the learning phase so that the system user can review the results of the learning phase, and can identify the features that the learning phase identified as being significant. This information is usable to design a diagnostic clinical test (for example, a test to predict cancer recurrence) that is effective, and yet only employs a relatively small number of features.

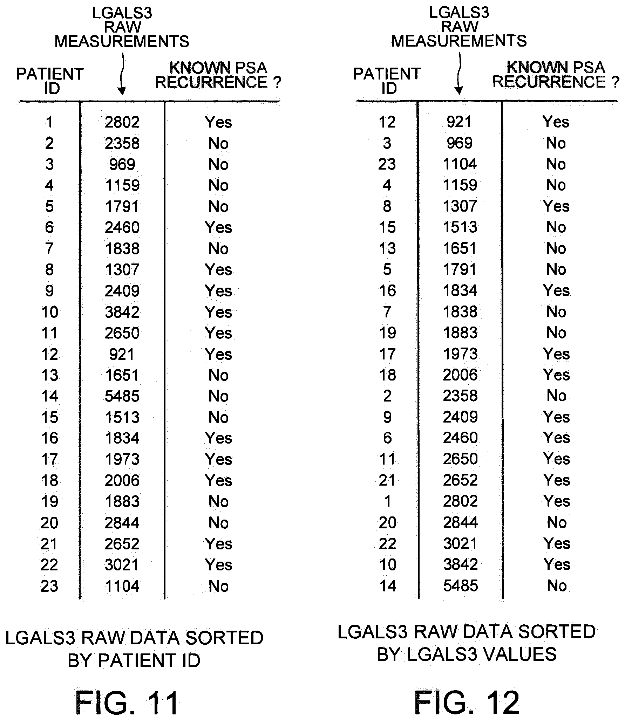

FIGS. 11-37 are a sequence of diagrams that set forth how the raw measurement values for the three univariate features (the LGALS3 feature, the MAGEC2 feature, and the IHC_DIST_CD163(+)_CD3(+)CD8(-) feature) is processed in preparation for the "diagnostic phase".

FIG. 11 shows the LGALS3 raw measurement data values. As mentioned above, the LGALS3 feature is a genomic feature, so the listed raw measurement values are counts. For each patient of the twenty-three patients, there is a raw measurement count. The right column sets forth the known cancer recurrence information for the associated patient. For example, the patient identified with patient ID of "4" was known not to suffer cancer recurrence, so the indicated value is "No". The LGALS3 raw measurement count for this patient is "1159", as indicated by the corresponding entry for patient "4" in the middle column of the table.

FIG. 12 shows how the information in the rows of FIG. 11 is reordered (i.e., is "sorted") so that the top row is for the patient having the smallest raw measurement count, and so that the bottom row is for the patient having the largest raw measurement count.

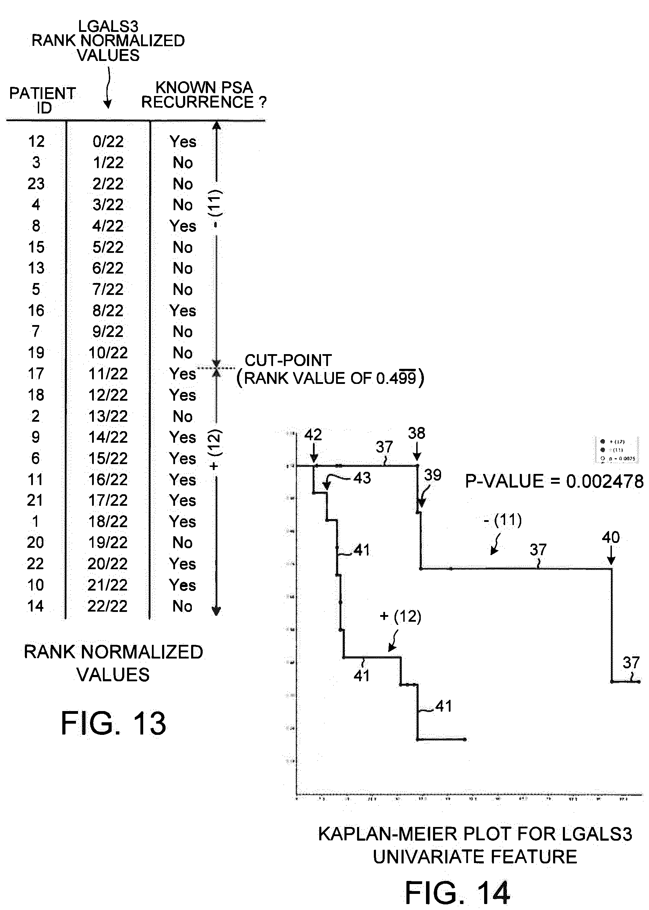

FIG. 13 shows how the raw measurement count values of FIG. 12 are then normalized into "rank normalized values". The smallest raw count value is replaced with the value 0/22, the next smallest raw count value is replaced with the value 1/22, and so forth. The denominator of the replacement values is the number of patients supplying the data (in this case, twenty-three) minus one. For the univariate features, a predetermined fixed cut-point value of 0.499 repeating is used. Accordingly, there are eleven patients in the group of patients above the cut-point value, and there are twelve patients the group of patients below the cut-point value. The p-value for this grouping is calculated to be 0.002478.

FIG. 14 is a Kaplan-Meier plot for the LGALS3 data of FIG. 13. The horizontal axis represents time. The upper line 37 represents the group of eleven patients above the cut-point in FIG. 13. This group of patients is estimated by the grouping to be patients who will not suffer cancer recurrence. At the time indicated by arrow 38, however, one of these patients did suffer recurrence. The upper line 37 of the Kaplan-Meier plot therefore drops downward an amount to reflect the number of patients suffering recurrence at this time. Then later, at the time indicated by arrow 39, another of the patients in this group suffered recurrence. The upper line 37 therefore drops downward further. No more of the patients of this group suffered recurrence until the time indicated by arrow 40, at which point another patient suffered recurrence. The upper line 37 therefore drops downward at again. The lower line 41 represents the group of twelve patients below the cut-point in FIG.

13. This group of patients is estimated by the grouping to be patients who will suffer recurrence. None of the patients of this group suffered recurrence until the time indicated by arrow 42. At this time a patient in this second group suffered recurrence, so the bottom line 41 drops downward an amount to reflect the number of patients suffering recurrence at that time. Similarly, at the time indicated by arrow 43 another of the patents of this second group sufferance recurrence, and therefore the lower line 41 drops downward again. If the grouping of patients as reflected by the cut-point were perfect, then the upper line 41 would extend horizontally from left to right over time, without ever dropping, because none of the patients represented by that upper line 37 would ever have suffered cancer recurrence. As to the bottom line 41, by the end of time at that right of the plot, that bottom line 41 would reach the very bottom of the plot because all the patients of the second group as represented by the bottom line 41 would have suffered cancer recurrence at some time. The actual Kaplan-Meier plot for the predetermined 0.499 cut-point and patient grouping of FIG. 13 is shown in FIG. 14.

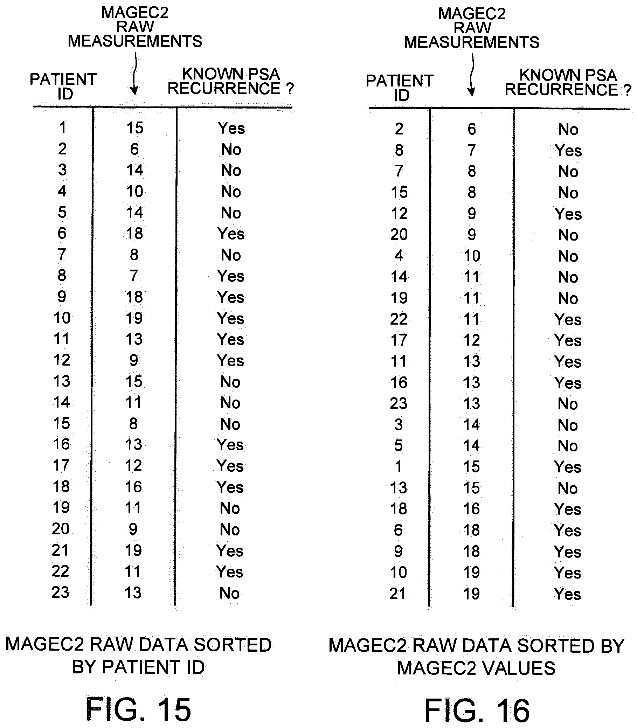

FIG. 15 shows the MAGEC2 raw measurement data values for the twenty-three patients being studied in the learning phase.

FIG. 16 shows the information in the rows of FIG. 15 in reordered form (i.e., "sorted") so that the top row is for the patient having the smallest raw measurement count, and so that the bottom row is for the patient having the largest raw measurement count.

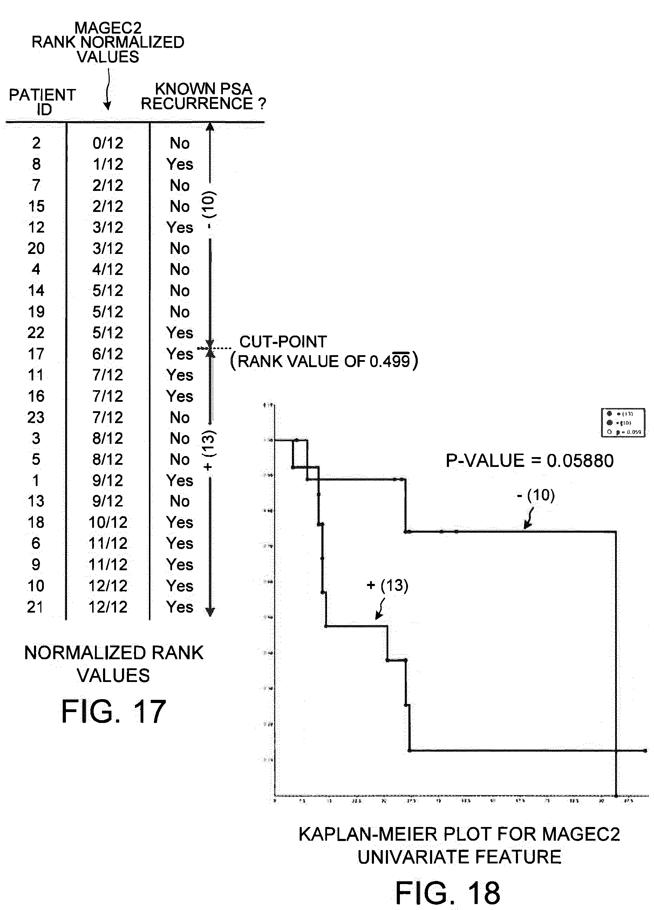

FIG. 17 shows how the ranked raw measurement count values of FIG. 16 are then normalized into rank normalized values. The cut-point value is the same predetermined cut-point value of 0.499 repeating as in FIG. 13.

FIG. 18 is a Kaplan-Meier plot for the MAGEC2 data of FIG. 17. For the predetermined cut-point value of 0.499 repeating as shown in FIG. 17, there are ten patients in a first group and there are thirteen patients in a second group. The p-value for this grouping is 0.05880.

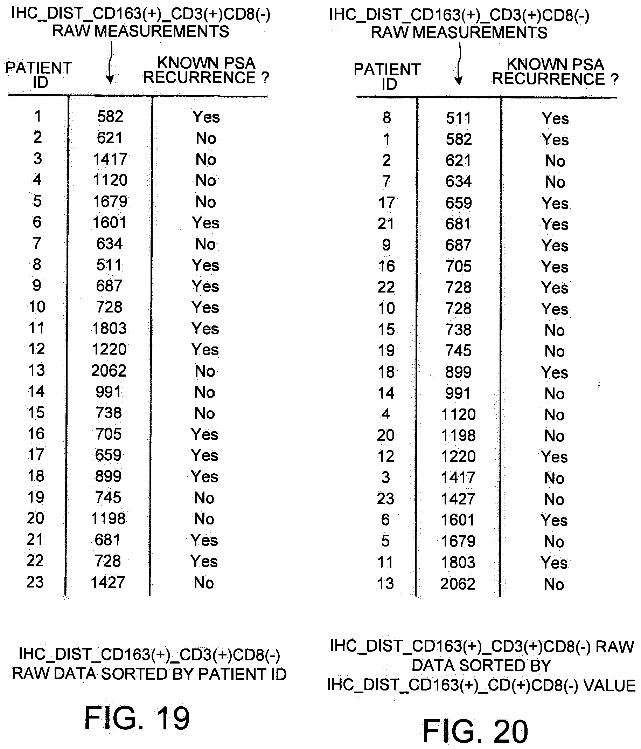

FIG. 19 shows the IHC_DIST_CD163(+)_CD3(+)CD8(-) raw measurement data values for the twenty-three patients being studied in the learning phase. These values are distances in micrometers.

FIG. 20 shows the information in the rows of FIG. 19 in reordered form (i.e., "sorted").

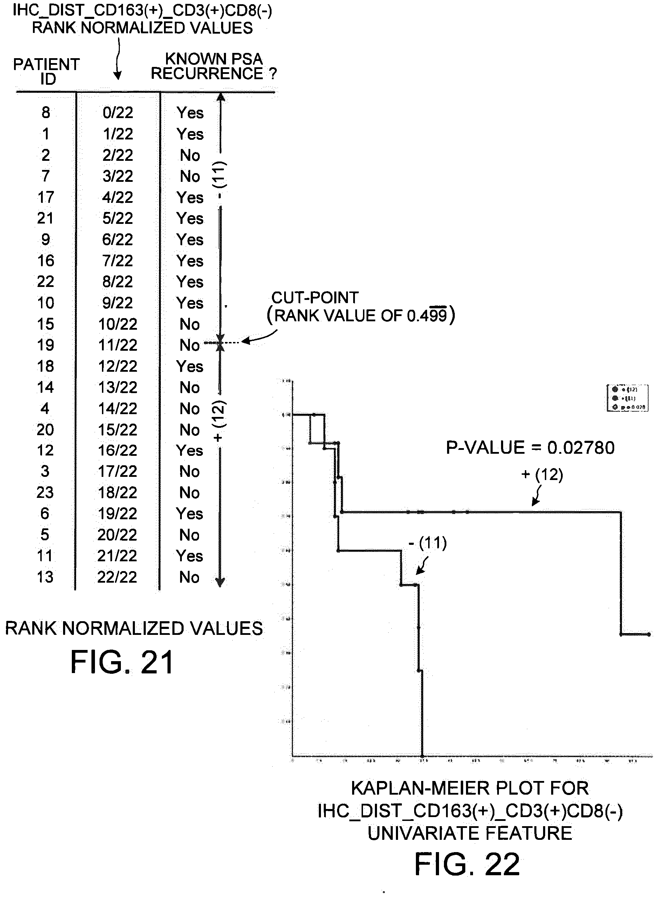

FIG. 21 shows how the ranked raw measurement count values of FIG. 20 are then normalized. The cut-point value is the same predetermined cut-point value of 0.499 repeating as in FIG. 17 and FIG. 13. The p-value for this grouping is 0.02780.

FIG. 22 is a Kaplan-Meier plot for the data of FIG. 21. For the predetermined cut-point value of 0.499 repeating as shown in FIG. 21, there are twelve patients in a first group and there are eleven patients in a second group. The p-value for this grouping is 0.02780.

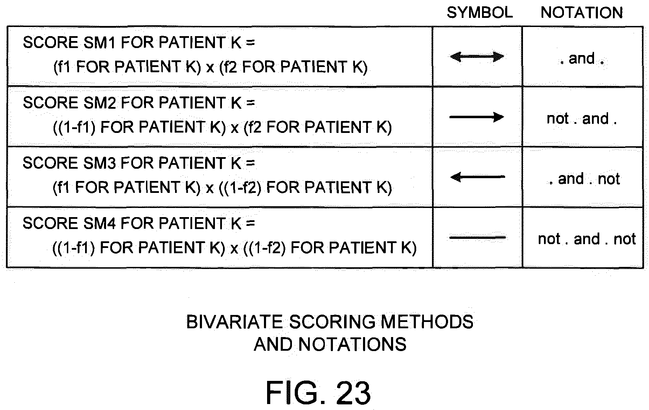

FIG. 23 is a table that shows the four "fuzzy logic" bivariate scoring methods. These scoring methods are denoted SM1, SM2, SM3 and SM4.

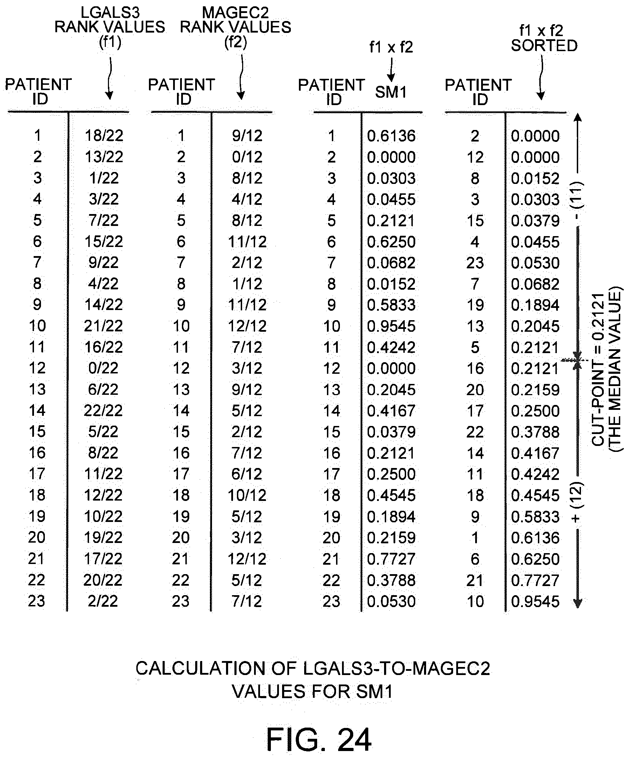

FIG. 24 is a diagram that shows how LGALS3-to-MAGEC2 normalized rank values are calculated using the first bivariate scoring method SM1. The leftmost two columns of the diagram set forth the normalized rank values for LGALS3. These are the normalized LGALS3 values of FIG. 13, except that they have been reordered according to patient ID. The next two leftmost columns of the diagram set forth the normalized rank values for MAGEC2. These are the normalized MAGEC2 values of FIG. 17, except that they have been reordered according to patient ID. For each patient ID, the normalized value in the LGALS3 column for that patient ID is multiplied by the normalized value in the MAGEC2 column for that patient ID. The resulting values are indicated in the column denoted "f1 x f2". The normalized LGALS3 value is simply multiplied by the normalized MAGEC2 value because the scoring method is SM1, and according to the table of FIG. 23, the f1 value for the patient is multiplied by the f2 value for patient K, where the variable K goes from one (for the patient having the patient ID of "1") to twenty-three (for the patient having the patient ID of "23"). The rows of the "f1 x f2" column are then reordered (i.e., "sorted") so that the top row has the smallest "f1 x f2" value, and so that the bottom row has the largest "f1 x f2" value. For the predetermined cut-point at the median value of 0.2121, there is a first group of eleven patients, a second group of twelve patients. For this patient grouping, the corresponding p-value is 0.0009008.

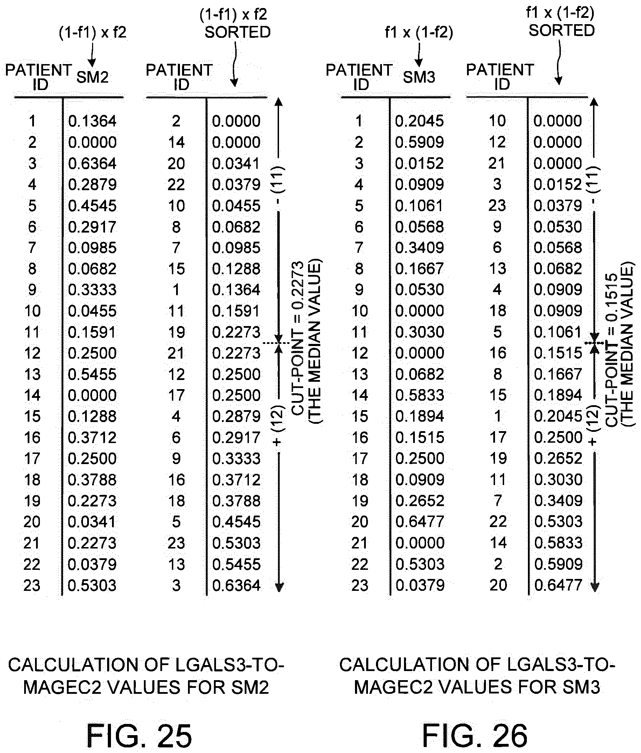

FIG. 25 is a diagram that shows how the cut-point and p-value for the LGALS3-to-MAGEC2 bivariate relationship is determined when the second bivariate scoring method SM2 is used. The cut-point is predetermined to be the median value of 0.2273. The same process as set forth above in connection with FIG. 24 is performed, except that the "f1 x f2" column of FIG. 24 is replaced with a "(1-f1) x f2" column. The values in this column are then reordered so that the smallest SM2 value is at the top of the reordered column, and so that the largest SM2 value is at the bottom of the reordered column. This reordered information is the rightmost column in FIG. 25 that is labeled "LGALS3-to-MAGEC2 normalized rank values for SM2". For the predetermined cut-point at the median value of 0.2273, there is a first group of eleven patients and a second group of twelve patients. For this patient grouping, the corresponding p-value is 0.9806.

FIG. 26 is a diagram that shows show how the cut-point and p-value for the LGALS3-to-MAGEC2 bivariate relationship is determined when the second bivariate scoring method SM3 is used. Again, the cut-point is predetermined to be the median value of 0.1515. For the predetermined cut-point at the median value of 0.1515, there is a first group of eleven patients and a second group of twelve patients. For this patient grouping, the corresponding p-value is 0.8034.

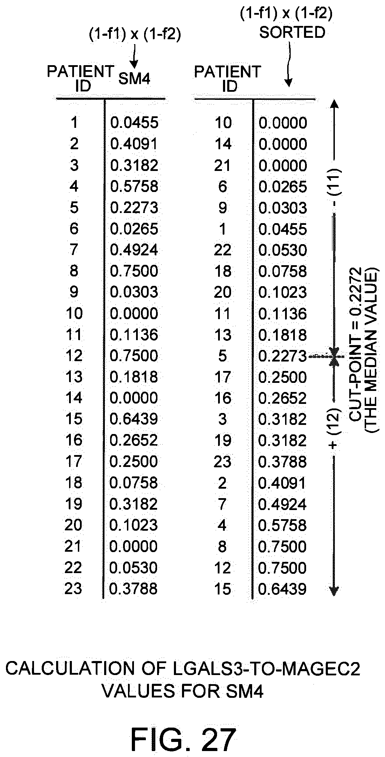

FIG. 27 is a diagram that shows show how the cut-point and p-value for the LGALS3-to-MAGEC2 bivariate relationship is determined when the second bivariate scoring method SM4 is used. Again, the cut-point is predetermined to be the median value of 0.2272. For the predetermined cut-point at the median value of 0.2272, there is a first group of eleven patients and a second group of twelve patients. For this patient grouping, the corresponding p-value is 0.006845.

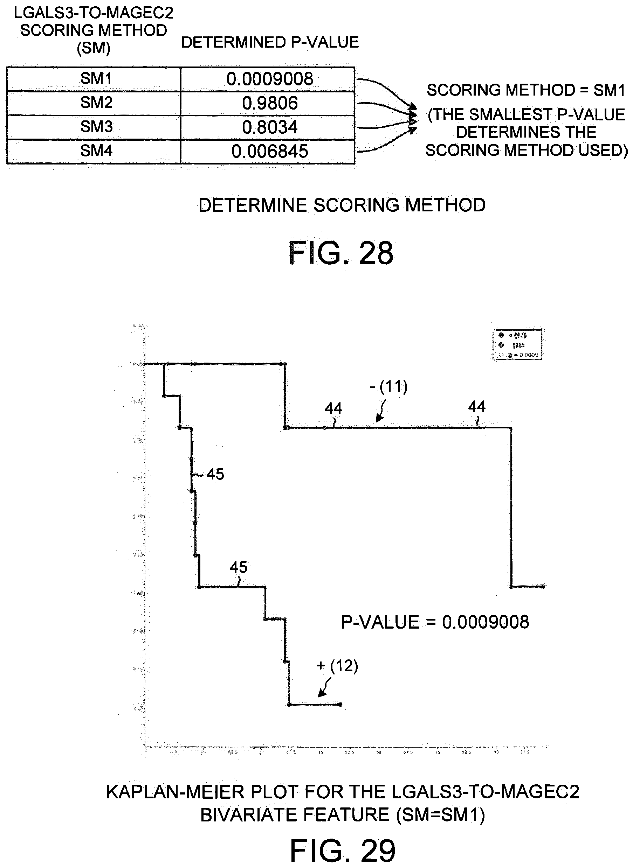

FIG. 28 is a table showing the p-values determined for the LGALS3-to-MAGEC2 bivariate relationship for each of the four scoring methods SM1, SM2, SM3 and SM4. Scoring method SM1 resulted in the smallest p-value, so scoring method SM1 is selected to be the scoring method used.

FIG. 29 is a Kaplan-Meier plot for the LGALS3-to-MAGEC2 bivariate relationship when the SM1 scoring method is used. As indicated by the diagram of FIG. 21 for the SM1 scoring method, the cut-point value of 0.2273 divides the patients into a first group of eleven patients and a second group of twelve patients. The first group is represented by the line 44 in the Kaplan-Meier plot of FIG. 29. The second group is represented by the line 45 in the Kaplan-Meier plot of FIG. 29.

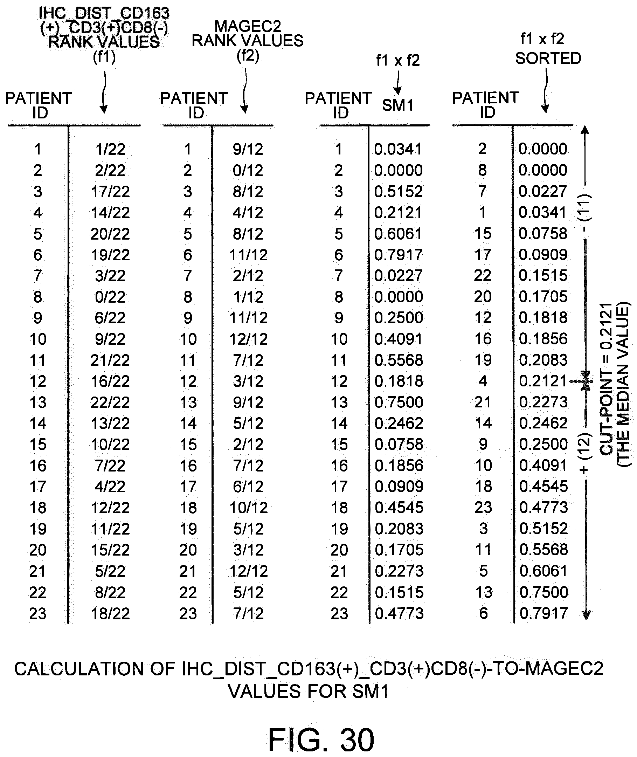

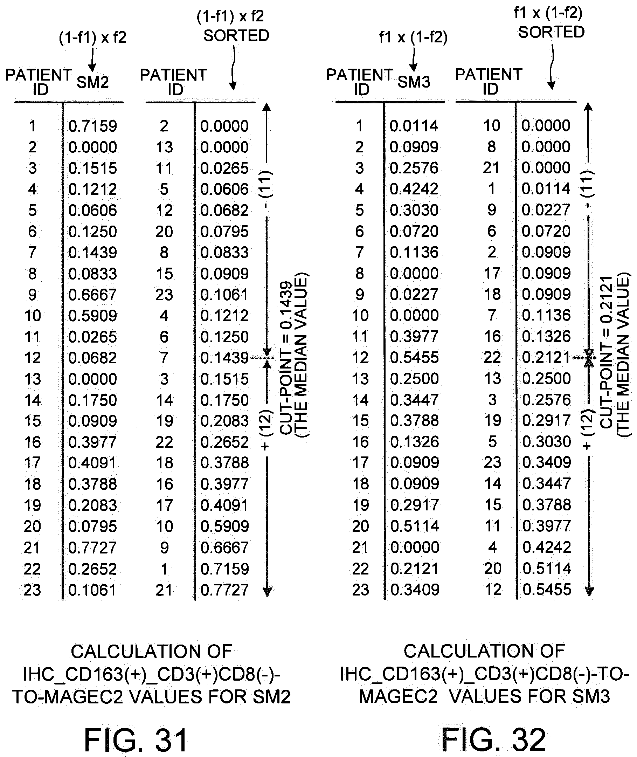

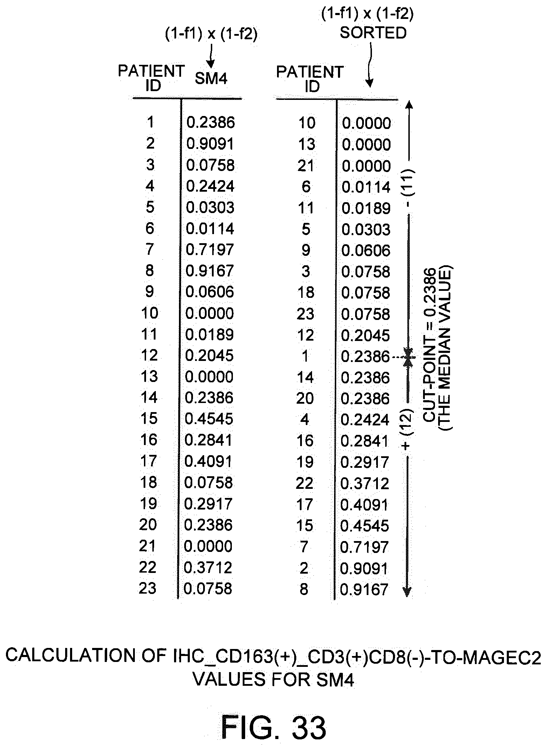

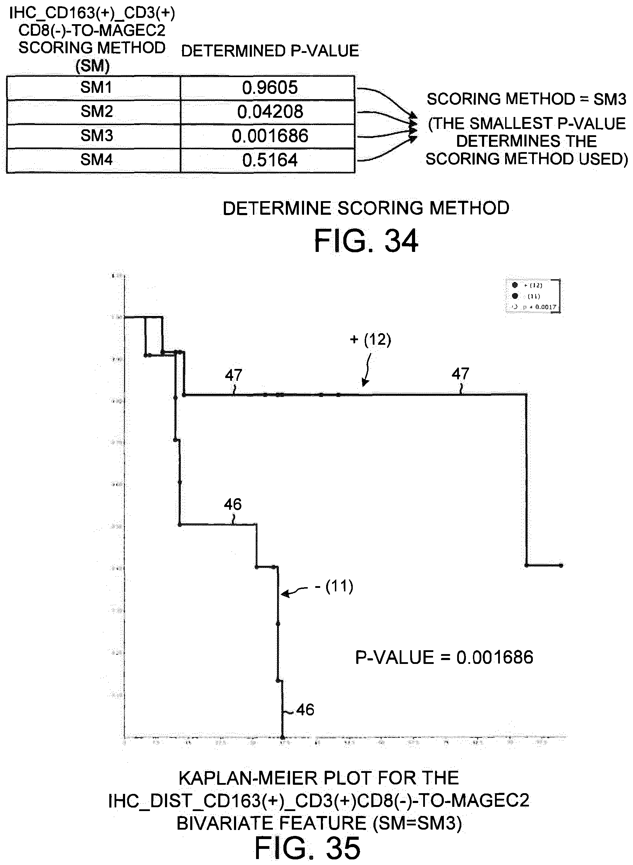

The same process described above for the LGALS3-to-MAGEC2 bivariate relationship in connection with FIGS. 24-29 is performed for the IHC_Dist_CD163(+)_CD3(+)CD8(-)-to-MAGEC2 bivariate relationship. FIG. 30 shows how the cut-point value and the p-value for the SM1 scoring method are determined. The cut-point is predetermined to be the median value of 0.2121. FIG. 31 shows how the cut-point value and the p-value for the SM2 scoring method are determined. The cut-point is predetermined to be the median value of 0.1439. FIG. 32 shows how the cut-point value and the p-value for the SM3 scoring method are determined. The cut-point is predetermined to be the median value of 0.2121. FIG. 33 shows how the cut-point value and the p-value for the SM4 scoring method are determined. The cut-point is predetermined to be the median value of 0.2386. FIG. 34 is a chart showing the p-values determined for each of the four scoring methods SM1, SM2, SM3 and SM4. The chart reveals that the smallest p-value is obtained when the SM3 scoring method is used. The SM3 scoring method is therefore determined to be the method used. FIG. 35 is a Kaplan-Meier plot for the IHC_Dist_CD163(+)_CD3(+)CD8(-)-to-MAGEC2 bivariate relationship when the SM3 scoring method is used. The predetermined median value cut-point of 0.2121 divides the twenty-three patients into a first group of eleven patients and a second group of twelve patients. The first group is represented by the line 46 in the Kaplan-Meier plot of FIG. 35. The second group is represented by the line 47 in the Kaplan-Meier plot of FIG. 35.

Accordingly, the "learning phase" results in the generation of both a cut-point value and a rank normalized value list for each of the three univariate features, and for each of the two bivariate features. This information is stored in the database 6 for later use in the diagnostic phase.

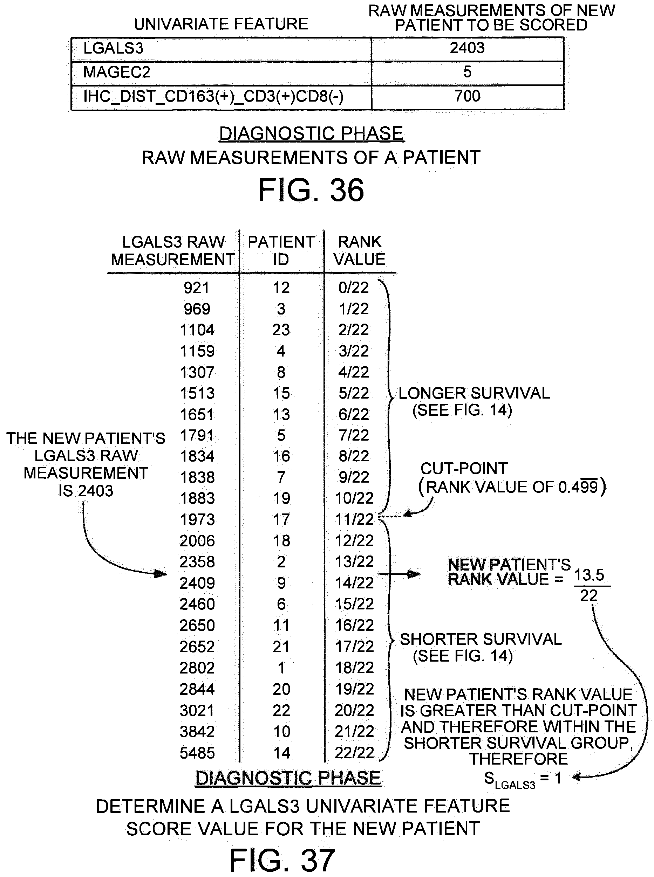

Operation of the system 1 of FIG. 3A in the "diagnostic phase" is described below in connection with FIGS. 36-43. The new patient for whom the score 2 is to be determined is seen in a clinical setting, and a tissue sample is obtained from the patient. The tissue sample is sliced and analyzed as explained above in connection with FIG. 3B. The first and second tissue slices are stained and imaged and analyzed by the system so as to generate an IHC_Dist_CD163(+)_CD3(+)CD8(-)-to-MAGEC2 raw measurement value. The third tissue slice is lysed and two pairs of probes are applied. The nCounter device is used to count probes, thereby generating two count values. These two count values are loaded into the system as the LGALS3 raw measurement value and the MAGEC2 raw measurement value. The three raw measurement values for the new patient are set forth in FIG. 36.

FIG. 37 shows the ordered rank values, the corresponding LGALS3 raw measurement values, and the cut-point value of FIG. 13 as determined in the learning phase for the LGALS3 univariate feature. Patient rank values that are greater than the cut-point value are for patients in the shorter survival group, whereas patient rank values that are smaller than the cut-point value are for patients in the longer survival group. For each patient ID, the LGALS3 raw measurement value is listed. The LGALS3 raw measurement value of 2403 for the new patient is compared to this list of raw measurement values obtained in the learning phase. As illustrated in FIG. 37, the 2403 value for the new patient is a value between the 2358 value for patient number 2 and the 2409 value for patient number 9. A rank value for the raw score of the new patient is then determined. The rank value corresponding to the 2403 raw measurement value is between the rank value for patient number 2 (who has a raw measurement value of 2358) and patient number 9 (who has a raw measurement value of 2409). The rank value for the new patient must therefore be between the rank value of 13/22 for patient number 2 and the rank value of 14/22 for patient number 9. In the present example, because the rank value for the new patient is between these two values, a rank value for the new patient is determined in simplified fashion to be a rank value of 13.5/22 which is midway between the two rank values of 13/22 and 14/22. This rank value of 13.5/22. This rank value of 13.5/22 for LGALS3 is stored for later use in the determining a bivariate feature score. If the rank value for the new patient is greater than the cut-point value, then an LGALS3 univariate feature score SLGALS3 has a value of "1", otherwise the score SLGALS3 has a value of "0". In the case of the raw measurement value being 2403, the corresponding rank value of 13.5/22 is greater than the cut-point value of 0.499, so the SLGALS3 score value is "1".

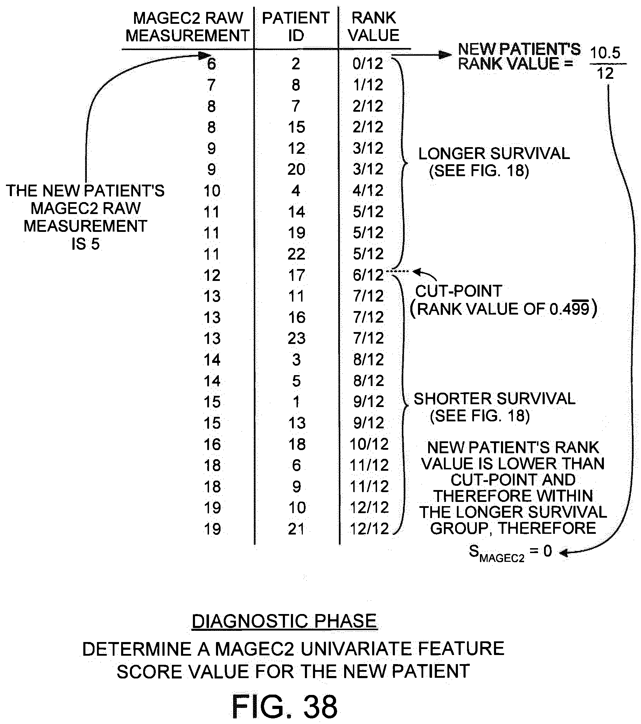

FIG. 38 shows the ordered rank values and the cut-point value determined in the learning phase for the MAGEC2 univariate feature. The same process described above in connection with LGALS3 and FIG. 37 is performed here for MAGEC2. The new patient's MAGEC2 raw measurement value of 5 points to a location in the list of raw MAGEC2 measurements that is above (smaller than) the raw measurement value of 6 for patient number 2. The rank value of the new patent is therefore determined to be 0/12. This rank value of 0/12 for MAGEC2 is stored for later use in the determining of bivariate feature scores. Because the rank value of 0/12 for the new patient is smaller than the cut-point value of 0.499, the rank value for the new patient is within the set of rank values for the "longer survival" group of patients. The SMAGEC2 score value is therefore determined to be "0".

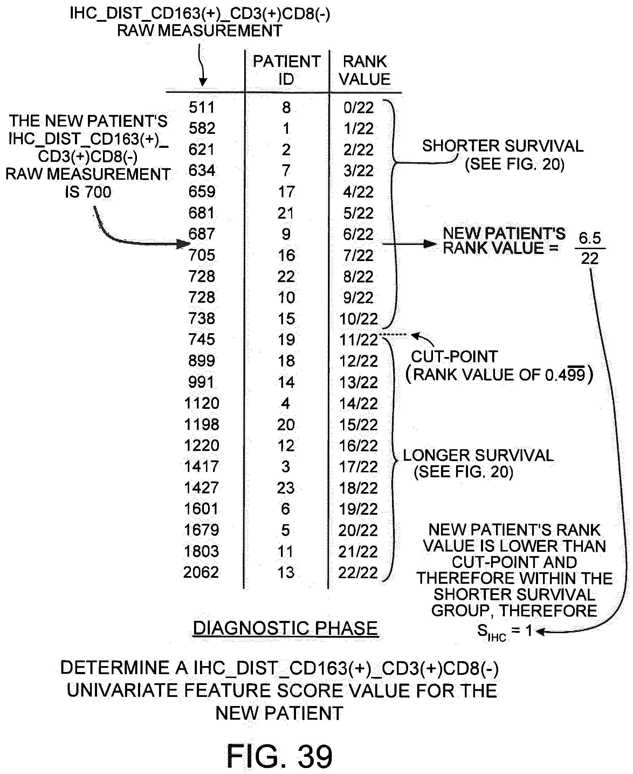

FIG. 39 shows the ordered rank values and the cut-point value determined in the learning phase for the IHC_Dist_CD163(+)_CD3(+)CD8(-) univariate feature. The same process described above in connection with LGALS3 and FIG. 37 is performed here for IHC_Dist_CD163(+)_CD3(+)CD8(-). The new patient's raw measurement value of 700 points to a location in the ordered list between patient number 9 and patient number 16. Patient number 9 has a rank value of 6/22 and patient number 16 has a rank value of 7/22, so the rank value for the new patient is determined to be rank value midway between these two values, namely 6./5/22. As can be seen from FIG. 39, rank values below the cut-point indicate relatively longer survival instances, as compared with rank value above the cut-point which indicate relatively shorter survival instances. In the case of the IHC_Dist_CD163(+)_CD3(+)CD8(-) univariate feature, therefore, being below (greater than) the cut-point value indicates relatively longer survival, whereas being above (smaller than) the cut-point value indicates relatively shorter survival. Because the new patient's raw measurement value of 700 corresponds to a rank value of 6.5/22, and because this rank value is smaller than the cut-point value of 0.499, the SIHC score value is determined to be "1".

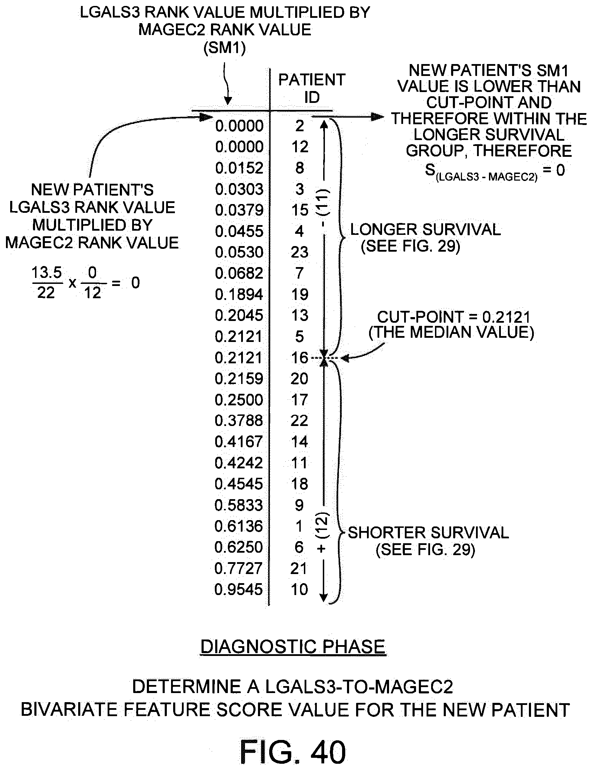

FIG. 40 shows how a LGALS3-to-MAGEC2 bivariate feature score value for the new patient is determined. Because the SM1 scoring method was determined in the diagnostic phase to the scoring method for the LGALS3-to-MAGEC2 bivariate relationship that results in the lowest p-value, the SM1 scoring method is used. The rank normalized list of rank values of FIG. 24 for the SM1 scoring method is replicated in FIG. 40. The rank value for the new patient is determined using the 13.5/22 rank value for LGALS3 as determined in connection with FIG. 37 and the 0/12 rank value for MAGEC2 as determined in connection with FIG. 38. As illustrated in FIG. 40, the SM1 equation is applied to these two rank values, resulting in a SM1 rank value for the LGALS3-to-MAGEC2 bivariate relationship of 0.0000. As can be seen from FIG. 40, this 0.0000 rank value is located at the top of the ordered list of rank values of FIG. 40. In the ordered list of FIG. 40, rank values that are smaller than the 0.2121 cut-point value are associated with the longer survival group of patients, and rank values that are larger than the 0.2121 cut-point value are associated with the shorter survival group of patients. Because the LGALS3-to-MAGEC2 SM1 value of 0.0000 is smaller than the 0.2121 cut-point value, it is within the longer survival group of patients, and the S(LGALS3-MAGEC2) score value is determined to be "0".

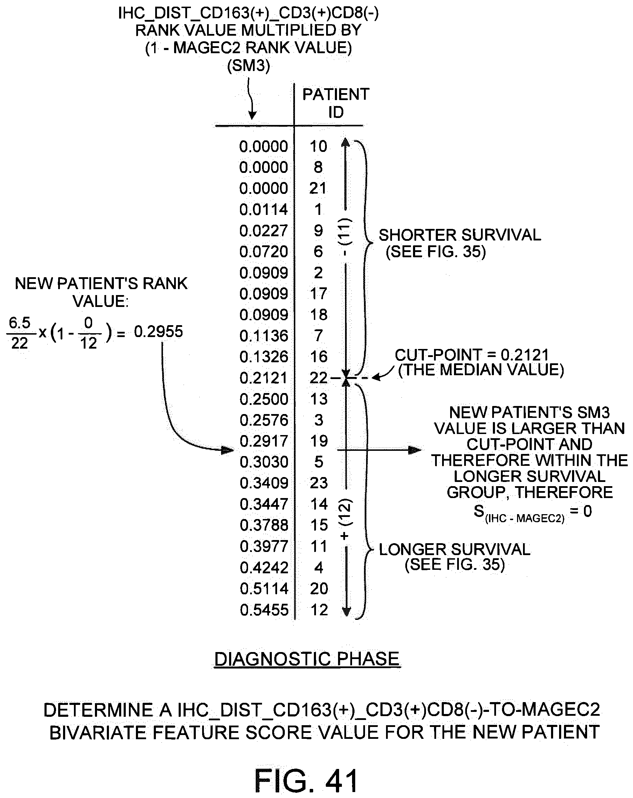

FIG. 41 shows how a IHC_Dist_CD163(+)_CD3(+)CD8(-)-to-MAGEC2 bivariate feature score value for the new patient is determined. The same process described above in connection with FIG. 40 is used. Because the SM3 scoring method was determined in the diagnostic phase (see FIG. 34) to be the scoring method having the smallest p-value, the SM3 scoring method is used. The list of rank values of FIG. 32 for the SM3 scoring method is replicated in FIG. 41. Rank values that are smaller than the cut-point value of 0.2121 are for patients in the shorter survival group of patients, whereas rank values that are larger than the cut-point value of 0.2121 are for patients in the longer survival group of patients. The rank value for the new patient is determined using the IHC rank value of 6.5/22 (see FIG. 39) and the MAGEC2 rank value of 0/12 (see FIG. 38). The SM3 equation is applied to these two rank values, resulting in an SM3 rank value for the bivariate relationship of 0.2955. As can be seen from FIG. 41, this 0.2955 rank value is located between the rank value for patient number 9 and the rank value for patient number 6. Because the 0.2955 rank for the new patient is larger than the 0.2121 cut-point value, it is within the longer survival group of patients. The S(IHC-MAGEC2) score value is therefore determined to be "0".

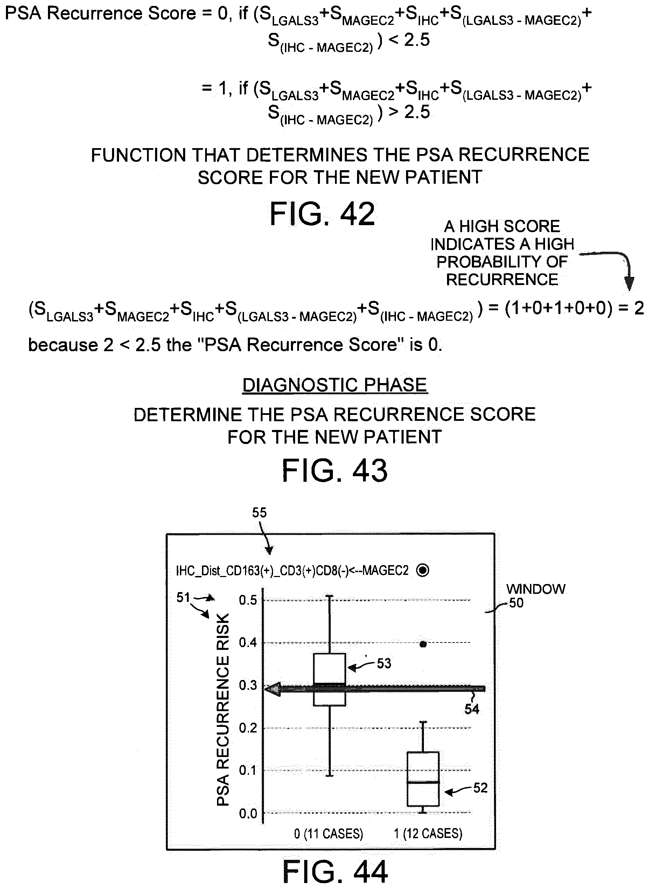

The score 2 generated by the system 1 of FIG. 3A for the new patient is a function of the three univariate feature score values SLGALS3, SMAGEC2 and SIHC, and of the two bivariate feature score values S(LGALS3 - MAGEC2) and S(IHC - MAGEC2). In the present example, this function is a majority voting function (see FIG. 42). Each of the five feature score values is a number which is either 0 or 1. The five feature score values are summed, and if the sum is greater than 2.5 then the overall score (the score 2 of FIG. 1) is determined to be "1", otherwise the overall score (the score 2 of FIG. 1) is determined to be "0". An overall score of "0" indicates that cancer recurrence is determined to be unlikely. An overall score of "1" indicates that cancer recurrence is determined to be likely.

FIG. 43 shows how the function of FIG. 42 is applied in the case of the new patient whose score 2 is being determined. The sum of the five features score values is 2, and this sum is smaller than 2.5, so score 2 as determined by the system 1 of FIG. 1 is "0". This score value of "0" is displayed on the display 9 of the computer 7 of FIG. 1. In the example of FIG. 2, three windows 48, 49 and 50 are presented on the display 9. The extended MST 13 is rendered in window 48. Text stating "SCORE = 0" is rendered in window 49. A graphical user interface visualization element 51 is rendered in window 50.

FIG. 44 is a diagram that shows the graphical user interface visualization element 51 in further detail. The visualization element 51 visualizes PSA recurrence risk of the new patient with an arrow 54. The visualization is presented in the context of the bivariate feature IHC_DST_CD163(+)_CD3(+)CD8(-)-to-MAGEC2 (IHC_DST_CD163(+)_CD3(+) CD8(-) and not MAGEC2). Box 52 depicts the rank values of the twelve learning phase patients whose rank values appear below the cut-point value in FIG. 41, whereas box 53 depicts the rank values of the eleven learning phase patients whose rank values appear above the cut-point value in FIG. 41. The textual note "IHC and not MAGEC2" 55 is a graphical indication of the scoring method of the bivariate feature, which in this case is scoring method SM3. The user of the system 1 of FIG. 1 can view the extended MST 13 on display 9 of the computer 7, and then use the computer's mouse or other pointing tool to select a feature of the extended MST. This selected feature may, for example, be the bivariate prognostic feature mentioned above. As a result of this selection, the system causes the diagram of FIG. 44 to be presented to the user in window 50 at the same time that the extended MST is presented in the window 48. The diagram in window 50 provides more detailed underlying data about the selected feature that is shown at a higher level of abstraction (as a node or edge) in the extended MST 13.

## Claims

1. A method for determining a risk of a cancer patient of a cancer recurrence, the method comprising the steps of:

(a) receiving raw structural feature measurement data of cancer tissue samples of a plurality of patients having the cancer onto a system;

(b) defining a structural feature based at least in part on the raw structural feature measurement data received in (a), wherein the structural feature includes a list of rank values;

(c) receiving raw gene expression feature measurement data of the cancer tissue samples of the plurality of patients onto the system;

(d) defining a gene expression feature based at least in part on the raw gene expression feature measurement data received in (c), wherein the gene expression feature includes a list of rank values;

(e) defining a bivariate prognostic feature by combining rank values of the list of rank values of the structural feature with rank values of the list of rank values of the gene expression feature thereby generating a list of rank values for the bivariate prognostic feature, wherein the defining of the bivariate prognostic feature in (e) further involves determining and storing a cut-point value for the bivariate prognostic feature;

(f1) measuring *in vitro* a structural feature measurement data value of a structural feature of a cancer tissue sample of the patient;

(f2) receiving the structural feature measurement data value onto the system, wherein the structural feature measurement data value is data obtained by analyzing a digital image of a first portion of tissue of the tissue sample;

(g1) measuring *in vitro* a gene expression feature measurement data value of a gene expression feature of the tissue sample, wherein the gene expression feature is a gene expression level of a gene;

wherein a combination of the structural feature and the gene expression feature is the bivariate prognostic feature for the recurrence of the cancer in the plurality of patients having the cancer defined in (e), wherein the structural feature and the gene expression feature are

elements of a minimal spanning tree that comprises nodes and edges, wherein each edge of the minimal spanning tree connects two nodes, wherein the minimal spanning tree is constructed from structural features, gene expression features and bivariate prognostic features for the recurrence of the cancer in the plurality of patients,

wherein each node of the minimal spanning tree represents a structural feature or a gene expression feature, wherein each edge of the minimal spanning tree represents a bivariate prognostic feature, wherein the bivariate prognostic feature is a combination of the two features that are represented by the two nodes connected by the edge, wherein at least one of the two features is a univariate prognostic feature for the recurrence of the cancer in the plurality of patients, and

wherein the width of each edge indicates a bivariate prognostic value of the bivariate prognostic feature that is represented by the edge;

(g2) receiving the gene expression feature measurement data value onto the system, wherein the gene expression feature measurement data value is data obtained by analyzing a second portion of the tissue of the tissue sample;

(h) calculating a score value for the bivariate prognostic feature based at least in part on the structural feature measurement data value received in (f2), the gene expression feature measurement data value received in (g2), and the cut-point value of (e), wherein the receiving of (f2) and the receiving of (g2) and the calculating in (h) are all per- formed after the defining of the bivariate prognostic feature in (e);

(i) determining a score by evaluating a function, wherein the function is a function of the score value calculated in (h), wherein (a) through (i) are performed by the system, and wherein the score determined in (i) is indicative of whether the cancer patient will have a recurrence of the cancer; and

(j) displaying a graphical indication of the combination of the structural feature measurement data value and the gene expression feature measurement data value on a graphical user interface to illustrate the risk of the patient of the recurrence of the cancer,
wherein the structural feature is an average distance between first objects and second objects of the tissue sample,
wherein the first objects and the second objects correspond to two different cell types and the average distance between the first objects and the second objects is determined by analyzing one or more digital images of one or more tissue slices of the tissue sample that have been stained by immunohistochemistry (IHC) using protein-specific IHC biomarkers that allow identifying the two different cell types,
wherein the gene expression level of the gene is a measurement of expression of mRNA transcribed from the gene in the tissue sample by using an mRNA-specific probe biomarker,
wherein all steps of the method are performed by the system.

2. The method of claim 1, wherein the bivariate prognostic value is a Kaplan Meier log rank test p-value or a hazard ratio.

3. The method of claim 1 or 2, wherein the size of each node that represents a univariate prognostic feature indicates a univariate prognostic value of the univariate prognostic feature that is represented by the node, wherein the univariate prognostic value preferably is a Kaplan Meier log rank test p- value or a hazard ratio.

4. The method of any of claims 1 to 3,

wherein step (f1) comprises the steps of
(f1-1) generating the first objects from a first digital image of a first tissue slice of the tissue sample, wherein the first tissue slice has been stained by immunohistochemistry (IHC) with a first antibody that stains cells exhibiting a first characteristic, wherein the first objects correspond to the cells stained by the first antibody;
(f1-2) generating the second objects from a second digital image of a second tissue slice of the tissue sample, wherein the second tissue slice has been stained by IHC with a second antibody that stains cells exhibiting a second characteristic, wherein the second objects correspond to the cells stained by the second antibody; and
(f1-3) determining the average distance between the first objects and the second objects of the tissue sample; and
wherein step (g1) comprises the step of
(g1-1) determining the gene expression level of the gene from a third tissue slice of the tissue sample, wherein the third tissue slice has been stained for gene expression with a probe of labeled RNA that stains mRNA molecules transcribed from the gene, wherein the gene expression level is determined by determining the amount of staining by the probe of labeled RNA on a third digital image of the third tissue slice, wherein the first tissue slice, the second tissue slice and the third tissue slice are z slices taken from the tissue sample at corresponding positions in the x and y dimensions.

5. The method of any of claims 1 to 4, wherein the gene encodes an immune system related component.

6. The method of any of claims 1 to 5, wherein the graphical user interface is a computer display.

7. The method of any of claims 1 to 6, wherein the cancer is prostate cancer, wherein the cancer recurrence preferably is a recurrence of measurable prostate-specific antigen (PSA) in the patient's blood.

**Patentansprüche**

1. Verfahren zum Bestimmen eines Risikos eines Krebspatienten für ein Wiederauftreten des Krebses, das Verfahren umfassend die Schritte:

(a) Empfangen von Rohmessdaten eines Strukturmerkmals von Krebsgewebeproben einer Vielzahl von Patienten, die den Krebs aufweisen, auf einem System;
(b) Definieren eines Strukturmerkmals, das mindestens teilweise auf den in (a) empfangenen Rohmessdaten des Strukturmerkmals basiert, wobei das Strukturmerkal eine Liste von Rangwerten umfasst;
(c) Empfangen von Rohmessdaten eines Genexpressionsmerkmals der Krebsgewebeproben der Vielzahl von Patienten auf dem System;
(d) Definieren eines Genexpressionsmerkmales, das mindestens teilweise auf den in (c) empfangenen Rohmessdaten des Genexpressionsmerkmales basiert, wobei das Genexpressionsmerkmal eine Liste von Rangwerten umfasst;
(e) Definieren eines bivariaten Prognosemerkmals durch ein Kombinieren von Rangwerten aus der Liste von Rangwerten des Strukturmerkmals mit Rangwerten aus der Liste von Rangwerten des Genexpressionsmerkmales, wodurch eine Liste von Rangwerten für das bivariate Prognosemerkmal erzeugt wird, wobei das Definieren des bivariaten Prognosemerkmals in (e) ferner ein Bestimmen und ein Speichern eines Trennstellenwerts für das bivariate Prognosemerkmal umfasst;

(f1) Messen, *in vitro,* eines Strukturmerkmalsmessdatenwerts eines Strukturmerkmals einer Krebsgewebeprobe des Patienten;

(f2) Empfangen des Strukturmerkmalsmessdatenwerts auf dem System, wobei der Strukturmerkmalsmessdatenwert Daten sind, die durch ein Analysieren eines digitalen Bilds eines ersten Gewebeabschnitts der Gewebeprobe erhalten werden;

(g1) Messen, *in vitro,* eines Genexpressionsmerkmalsmessdatenwerts eines Genexpressionsmerkmals der Gewebeprobe, wobei das Genexpressionsmerkmal ein Genexpressionsgrad eines Gens ist;

wobei eine Kombination aus dem Strukturmerkmal und dem Genexpressionsmerkmal das bivariate Prognosemerkmal für das Wiederauftreten des Krebses bei der Vielzahl von Patienten ist, die den in (e) definierten Krebs ausweisen, wobei das Strukturmerkmal und das Genexpressionsmerkmal sind Elemente eines minimalen Spannbaums, der Knoten und Kanten umfasst, wobei jede Kante des minimalen Spannbaums zwei Knoten verbindet, wobei der minimale Spannbaum aus Strukturmerkmalen, Genexpressionsmerkmalen und bivariaten Prognosemerkmalen für das Wiederauftreten des Krebses bei der Vielzahl von Patienten aufgebaut ist,
wobei jeder Knoten des minimalen Spannbaums ein Strukturmerkmal oder ein Genexpressionsmerkmal darstellt,
wobei jede Kante des minimalen Spannbaums ein bivariates Prognosemerkmal darstellt, wobei das bivariate Prognosemerkmal eine Kombination aus den zwei Merkmalen ist, die durch die zwei Knoten dargestellt werden, die durch die Kante verbunden sind, wobei mindestens eines der zwei Merkmale ein univariates Prognosemerkmal für das Wiederauftreten des Krebses bei der Vielzahl von Patienten ist, und wobei die Breite jeder Kante einen bivariaten Prognosewert des bivariaten Prognosemerkmals angibt, das durch die Kante dargestellt wird;

(g2) Empfangen des Genexpressionsmerkmalsmessdatenwerts auf dem System, wobei der Genexpressionsmerkmalsmessdatenwert Daten sind, die durch das Analysieren eines zweiten Gewebeabschnitts der Gewebeprobe erhalten werden;

(h) Berechnen eines Punktzahlwerts für das bivariate Prognosemerkmal, der mindestens teilweise auf dem in (f2) empfangenen Strukturmerkmalsmessdatenwert, dem in (g2) empfangenen Genexpressionsmerkmalsmessdatenwert und dem Trennstellenwert von (e) basiert, wobei das Empfangen von (f2) und das Empfangen von (g2) und das Berechnen in (h) alle nach dem Definieren des bivariaten Prognosemerkmals in (e) durchgeführt werden;

(i) Bestimmen einer Punktzahl durch ein Auswerten einer Funktion, wobei die Funktion eine Funktion des in (h) berechneten Punktzahlwerts ist, wobei (a) bis einschließlich (i) durch das System durchgeführt werden und wobei die in (i) bestimmte Punktzahl angibt, ob der Krebspatient ein Wiederauftreten des Krebses aufweisen wird; und

(j) Anzeigen einer grafischen Angabe der Kombination aus dem Strukturmerkmalsmessdatenwert und dem Genexpressionsmerkmalsmessdatenwert auf einer grafischen Benutzeroberfläche, um das Risiko des Patienten für ein Wiederauftreten des Krebses zu veranschaulichen,

wobei das Strukturmerkmal ein durchschnittlicher Abstand zwischen ersten Objekten und zweiten Objekten der Gewebeprobe ist,
wobei die ersten Objekte und die zweiten Objekte zwei unterschiedlichen Zelltypen entsprechen und der durchschnittliche Abstand zwischen den ersten Objekten und den zweiten Objekten durch das Analysieren eines oder mehrerer digitaler Bilder eines oder mehrerer Gewebeschnitte der Gewebeprobe bestimmt wird, die durch Immunhistochemie (IHC) unter Verwendung proteinspezifischer IHC-Biomarker gefärbt wurden, die ein Identifizieren der zwei unterschiedlichen Zelltypen ermöglichen,
wobei der Genexpressionsgrad des Gens eine Messung der Expression von mRNA ist, die von dem Gen in der Gewebeprobe durch Verwenden eines mRNA-spezifischen Sondenbiomarkers transkribiert wird,
wobei alle Schritte des Verfahrens durch das System durchgeführt werden.

**2.** Verfahren nach Anspruch 1, wobei der bivariate Prognosewert ein p-Wert des Kaplan-Meier-Log-Rang-Tests oder ein Risikoverhältnis ist.

**3.** Verfahren nach Anspruch 1 oder 2, wobei die Größe jedes Knotens, der ein univariates Prognosemerkmal darstellt, einen univariaten Prognosewert des univariaten Prognosemerkmals angibt, das durch den Knoten dargestellt wird, wobei der univariate Prognosewert vorzugsweise ein p-Wert des Kaplan-Meier-Log-Rang-Tests oder ein Risikoverhältnis ist.

4. Verfahren nach einem der Ansprüche 1 bis 3, wobei Schritt (f1) die Schritte umfasst

(f1-1) Erzeugen der ersten Objekte aus einem ersten digitalen Bild einer ersten Gewebescheibe der Gewebeprobe, wobei die erste Gewebescheibe durch Immunhistochemie (IHC) mit einem ersten Antikörper gefärbt wurde, der Zellen färbt, die eine erste Eigenschaft vorweisen, wobei die ersten Objekte den Zellen entsprechen, die durch den ersten Antikörper gefärbt werden;

(f1-2) Erzeugen der zweiten Objekte aus einem zweiten digitalen Bild einer zweiten Gewebescheibe der Gewebeprobe, wobei die zweite Gewebescheibe durch IHC mit einem zweiten Antikörper gefärbt wurde, der Zellen färbt, die eine zweite Eigenschaft vorweisen, wobei die zweiten Objekte den Zellen entsprechen, die durch den zweiten Antikörper gefärbt werden; und

(f1-3) Bestimmen des durchschnittlichen Abstands zwischen den ersten Objekten und den zweiten Objekten der Gewebeprobe; und

wobei Schritt (g1) den Schritt umfasst

(g1-1) Bestimmen des Genexpressionsgrads des Gens aus einer dritten Gewebescheibe der Gewebeprobe, wobei die dritte Gewebescheibe auf Genexpression mit einer Sonde von markierter RNA gefärbt wurde, die von dem Gen transkribierte mRNA-Moleküle färbt, wobei der Genexpressionsgrad durch das Bestimmen eines Ausmaßes der Färbung durch die Sonde von markierter RNA auf einem dritten digitalen Bild der dritten Gewebescheibe bestimmt wird, wobei die erste Gewebescheibe, die zweite Gewebescheibe und die dritte Gewebescheibe Z-Scheiben sind, die aus der Gewebeprobe an entsprechenden Positionen in der x- und y-Dimension entnommen wurden.

5. Verfahren nach einem der Ansprüche 1 bis 4, wobei das Gen eine mit dem Immunsystem in Zusammenhang stehende Komponente codiert.

6. Verfahren nach einem der Ansprüche 1 bis 5, wobei die grafische Benutzeroberfläche ein Computerbildschirm ist.

7. Verfahren nach einem der Ansprüche 1 bis 6, wobei der Krebs Prostatakrebs ist, wobei das Wiederauftreten des Krebses vorzugsweise ein Wiederauftreten des messbaren Prostata-spezifischen Antigens (PSA) in dem Blut des Patienten ist.

## Revendications

1. Procédé permettant de déterminer un risque de récidive de cancer d'un patient cancéreux, le procédé comprenant les étapes consistant à :

(a) recevoir des données brutes de mesure de caractéristique structurelle d'échantillons de tissu cancéreux d'une pluralité de patients atteints de cancer sur un système ;

(b) définir une caractéristique structurelle en fonction au moins en partie des données brutes de mesure de caractéristique structurelle reçues en (a), dans lequel la caractéristique structurelle comporte une liste de valeurs de classement ;

(c) recevoir des données brutes de mesure de caractéristique d'expression génique des échantillons de tissu cancéreux de la pluralité de patients sur le système ;

(d) définir une caractéristique d'expression génique en fonction au moins en partie des données brutes de mesure de caractéristique d'expression génique reçues en (c), dans lequel la caractéristique d'expression génique comporte une liste de valeurs de classement ;

(e) définir une caractéristique pronostique bivariée en combinant des valeurs de classement de la liste de valeurs de classement de la caractéristique structurelle avec des valeurs de classement de la liste de valeurs de classement de la caractéristique d'expression génique en générant de ce fait une liste de valeurs de classement pour la caractéristique pronostique bivariée, dans lequel la définition de la caractéristique pronostique bivariée en (e) implique en outre la détermination et le stockage d'une valeur de point de coupure pour la caractéristique pronostique bivariée ;

(f1) mesurer *in vitro* une valeur de données de mesure de caractéristique structurelle d'une caractéristique structurelle d'un échantillon de tissu cancéreux du patient ;

(f2) recevoir la valeur de données de mesure de caractéristique structurelle sur le système, dans lequel la valeur de données de mesure de caractéristique structurelle est des données obtenues en analysant une image numérique d'une première partie de tissu de l'échantillon de tissu ;

(g1) mesurer *in vitro* une valeur de données de mesure de caractéristique d'expression génique d'une caractéristique d'expression génique de l'échantillon de tissu, dans lequel la caractéristique d'expression génique est un niveau d'expression génique d'un gène ;

dans lequel une combinaison de la caractéristique structurelle et de la caractéristique d'expression génique est la caractéristique pronostique bivariée pour la récidive du cancer dans la pluralité de patients atteints de cancer définie en (e), dans lequel la caractéristique structurelle et la caractéristique d'expression génique sont

des éléments d'un arbre couvrant minimal qui comprend des nœuds et des bords, dans lequel chaque bord de l'arbre couvrant minimal relie deux nœuds, dans lequel l'arbre couvrant minimal est construit à partir de caractéristiques structurales, de caractéristiques d'expression génique et de caractéristiques pronostiques bivariées pour la récidive du cancer dans la pluralité de patients,

dans lequel chaque nœud de l'arbre couvrant minimal représente une caractéristique structurelle ou une caractéristique d'expression génique,

dans lequel chaque bord de l'arbre couvrant minimal représente une caractéristique pronostique bivariée, dans lequel la caractéristique pronostique bivariée est une association des deux caractéristiques qui sont représentées par les deux nœuds reliés par le bord, dans lequel au moins l'une des deux caractéristiques est une caractéristique pronostique univariée pour la récidive du cancer dans la pluralité de patients, et

dans lequel la largeur de chaque bord indique une valeur pronostique bivariée de la caractéristique pronostique bivariée qui est représentée par le bord ;

(g2) recevoir la valeur de données de mesure de caractéristique d'expression génique sur le système, dans lequel la valeur de données de mesure de caractéristique d'expression génique est des données obtenues en analysant une seconde partie du tissu de l'échantillon de tissu ;

(h) calculer une valeur de score pour la caractéristique pronostique bivariée en fonction au moins en partie de la valeur de données de mesure de caractéristique structurelle reçue en (f2), de la valeur de données de mesure de caractéristique d'expression génique reçue en (g2), et de la valeur de point de coupure de (e), dans lequel la réception de (f2) et la réception de (g2) et le calcul en (h) sont tous mis en œuvre après la définition de la caractéristique pronostique bivariée en (e) ;

(i) déterminer un score en évaluant une fonction, dans lequel la fonction est une fonction de la valeur de score calculée en (h), dans lequel (a) à (i) sont mis en œuvre par le système, et dans lequel le score déterminé en (i) indique si le patient cancéreux aura une récidive du cancer ; et

(j) afficher une indication graphique de la combinaison de la valeur de données de mesure de caractéristique structurelle et de la valeur de données de mesure de caractéristique d'expression génique sur une interface graphique utilisateur afin d'illustrer le risque de la récidive du cancer pour le patient,

dans lequel la caractéristique structurelle est une distance moyenne entre des premiers objets et des seconds objets de l'échantillon de tissu,

dans lequel les premiers objets et les seconds objets correspondent à deux types de cellule différents et la distance moyenne entre les premiers objets et les seconds objets est déterminée en analysant une ou plusieurs images numériques d'une ou plusieurs coupes de tissu de l'échantillon de tissu qui ont été colorées par immuno-histochimie (IHC) à l'aide de biomarqueurs IHC spécifiques à une protéine qui permettent l'identification des deux types de cellule différents,

dans lequel le niveau d'expression génique du gène est une mesure d'expression d'ARNm transcrit à partir du gène dans l'échantillon de tissu en utilisant un biomarqueur à sonde spécifique à l'ARNm,

dans lequel toutes les étapes du procédé sont mises en œuvre par le système.

2. Procédé selon la revendication 1, dans lequel la valeur pronostique bivariée est une valeur p de test de log-rank de Kaplan-Meier ou un rapport de risques instantanés.

3. Procédé selon la revendication 1 ou 2, dans lequel la taille de chaque noeud qui représente une caractéristique pronostique univariée indique une valeur pronostique univariée de la caractéristique pronostique univariée qui est représentée par le noeud, dans lequel la valeur pronostique univariée est de préférence une valeur p de test de log-rank de Kaplan-Meier ou un rapport de risques instantanés.

**4.** Procédé selon l'une quelconque des revendications 1 à 3, dans lequel l'étape (f1) comprend les étapes consistant à

(f1-1) générer les premiers objets à partir d'une première image numérique d'une première coupe de tissu de l'échantillon de tissu, dans lequel la première coupe de tissu a été colorée par immuno-histochimie (IHC) avec un premier anticorps qui colore des cellules présentant une première caractéristique, dans lequel les premiers objets correspondent aux cellules colorées par le premier anticorps ;
(f1-2) générer les seconds objets à partir d'une deuxième image numérique d'une deuxième coupe de tissu de l'échantillon de tissu, dans lequel la deuxième coupe de tissu a été colorée par IHC avec un second anticorps qui colore des cellules présentant une seconde caractéristique, dans lequel les seconds objets correspondent aux cellules colorées par le second anticorps ; et
(f1-3) déterminer la distance moyenne entre les premiers objets et les seconds objets de l'échantillon de tissu ; et dans lequel l'étape (g1) comprend l'étape consistant à
(g1-1) déterminer le niveau d'expression génique du gène à partir d'une troisième coupe de tissu de l'échantillon de tissu, dans lequel la troisième coupe de tissu a été colorée pour expression génique avec une sonde d'ARN marqué qui colore des molécules d'ARNm transcrites à partir du gène, dans lequel le niveau d'expression génique est déterminé en déterminant la quantité de coloration par la sonde d'ARN marqué sur une troisième image numérique de la troisième coupe de tissu, dans lequel la première coupe de tissu, la deuxième coupe de tissu et la troisième coupe de tissu sont des coupes z prélevées de l'échantillon de tissu au niveau de positions correspondantes dans les dimensions x et y.

**5.** Procédé selon l'une quelconque des revendications 1 à 4, dans lequel le gène code pour un composant apparenté au système immunitaire.

**6.** Procédé selon l'une quelconque des revendications 1 à 5, dans lequel l'interface graphique utilisateur est un dispositif d'affichage d'ordinateur.

**7.** Procédé selon l'une quelconque des revendications 1 à 6, dans lequel le cancer est un cancer de la prostate, dans lequel la récidive de cancer est de préférence une récidive d'antigène prostatique spécifique (PSA) mesurable dans le sang du patient.

FIG. 1A

Gleason 6 (n=5)
Gleason 7a (n=6)
Gleason 7b (n=5)
Gleason 8 (n=3)
Gleason 9 (n=4)

FIG. 1B

Co-registration of differently stained tissue sections

Segmentation of tissue sections for structure detection

| CK18/p63 Tumor and non-tumor glands | CD68/CD163 Macrophages | CD3/CD8 T-cells | CD34 Vessels |

Aggregation of all structures in hyperspectral heatmaps

Heatmap processing and region-based quantification

Complement features with densities and ratios of positive cells

FIG. 1C

IHC image     Segmentation
CK18/p63

CD68/CD163

CD3/CD8

CD34

Hyperspectral
heatmaps

462 features

FIG. 1D

FIG. 1E

FIG. 1F

File     Edit     View     Analysis     Help

norm_vs_orig \ genes_orig ( gene_annotations

| # | Sample | orig_C4B | norm_C4B ▲ |
|---|--------|----------|------------|
| 1 | 139 : 1 : 1 | 315 | 0 |
| 2 | 21 : 0 : 0 | 422 | 0.045454545454545 |
| 3 | 95 : 1 : 0 | 606 | 0.090909090909091 |
| 4 | 128 : 0 : 1 | 752 | 0.13636363636364 |
| 5 | 41 : 1 : 0 | 794 | 0.18181818181818 |
| 6 | 103 : 1 : 0 | 958 | 0.22727272727273 |
| 7 | 32 : 0 : 0 | 1009 | 0.27272727272727 |
| 8 | 7 : 1 : 0 | 1272 | 0.31818181818182 |
| 9 | 132 : 1 : 1 | 1287 | 0.36363636363636 |
| 10 | 150 : 1 : 1 | 1314 | 0.40909090909091 |
| 11 | 86 : 1 : 0 | 1384 | 0.45454545454545 |
| 12 | 18 : 0 : 0 | 1456 | 0.5 |
| 13 | 92 : 1 : 0 | 1901 | 0.54545454545455 |
| 14 | 126 : 1 : 1 | 2087 | 0.59090909090909 |
| 15 | 136 : 1 : 1 | 2216 | 0.63636363636364 |
| 16 | 159 : 0 : 1 | 2364 | 0.68181818181818 |
| 17 | 54 : 0 : 0 | 2387 | 0.72727272727273 |
| 18 | 148 : 1 : 1 | 2537 | 0.77272727272727 |
| 19 | 13 : 0 : 0 | 2797 | 0.81818181818182 |
| 20 | 141 : 0 : 1 | 2907 | 0.86363636363636 |
| 21 | 142 : 0 : 1 | 3100 | 0.90909090909091 |
| 22 | 129 : 0 : 1 | 4052 | 0.95454545454545 |
| 23 | 130 : 0 : 1 | 5418 | 1 |

FIG. 1G (part 1)

FIG. 1G (part 2)

PSA(+) PSA(-) Bubble size: score

FIG. 1H

FIG. 2A

1: Feature values > cut-point
2: Feature values ≤ cut-point

FIG. 2B

FIG. 2C

FIG. 2D

1: Feature values > cut-point
2: Feature values ≤ cut-point

FIG. 2E

FIG. 2F

1: + (10 cases)
2: - (13 cases)

score

0 (11 cases)    1 (12 cases)

⊖ log10

Group by PSA recurrence (yes/no)

EP 3 533 883 B1

FIG. 2G

FIG. 3A

FIG. 3B

EXTENDED MINIMAL SPANNING TREE (MST) OF UNIVARIATE AND
BIVARIATE FEATURES THAT PREDICT PSA RECURRENCE

FIG. 4A

IHC_DIST_CD163(+)_CD3(+)CD8(-)

MAGEC2

LGALS3

32 UNIVARIATE FEATURES DETERMINED TO BE SIGNIFICANT

730 UNIVARIATE FEATURES

TWO-DIMENSIONAL MATRIX OF P-VALUES OF BIVARIATE FEATURES

(DARKER COLORS REPRESENT MORE SIGNIFICANT P-VALUES
(LARGER -LOG(LOG-RANK P-VALUES))

FIG. 4B

| | |
|---|---|
| IHC_DIST_CD163(+)_CD8(+)_STROMA | ← Average distance of CD163(+) cells to CD8(+) cells in stroma |
| IHC_DIST_CD163(+)_CD3(+)CD8(-) | ← Average distance of CD163(+) cells to CD3(+)CD8(-) cells on whole slide |
| IHC_DIST_CD34(+)_CD163(+)_STROMA | ← Average distance of CD34(+) cells to CD163(+) cells in stroma |
| CD59 | |
| MAGEC2 | |
| ATF2 | |
| NFKBIA | |
| CXCL13 | |
| NUP107 | |
| IL12A | |
| JAK2 | |
| SYCP1 | |
| MAPK1 | |
| TBX21 | |
| SYK | |
| ANXA1 | |
| PMCH | |
| MBL2 | |
| C4B | |
| BMI1 | |
| CCL5 | |
| STAT4 | |
| IRF1 | |
| CD47 | |
| PSEN2 | |
| CD96 | |
| CTSH | |
| IL17B | |
| DDX43 | |
| KLRK1 | |
| LGALS3 | |
| PTGDR2 | |

UNIVARIATE FEATURES DETERMINED TO SIGNIFICANTLY
PREDICT PSA RECURRENCE

# FIG. 5

STARTING DIGITAL IMAGE OF
FIRST TISSUE SLICE (CD68/CD163 STAINED)

## FIG. 6

STARTING DIGITAL IMAGE OF SECOND
TISSUE SLICE (CD3/CD8 STAINED)

## FIG. 7

26

SEGMENTATION AND CLARIFICATION RESULT IMAGE
(→ INDICATES A CD163(+) OBJECT )

FIG. 8

( → INDICATES A CD3(+)CD8(-) OBJECT )

FIG. 9

DETERMINE AVERAGE DISTANCE FROM EACH CD163(+) OBJECT
TO ITS NEAREST FOUR CD3(+)CD8(-) OBJECTS

# FIG. 10

| PATIENT ID | LGALS3 RAW MEASUREMENTS | KNOWN PSA RECURRENCE ? |
|:---:|:---:|:---:|
| 1 | 2802 | Yes |
| 2 | 2358 | No |
| 3 | 969 | No |
| 4 | 1159 | No |
| 5 | 1791 | No |
| 6 | 2460 | Yes |
| 7 | 1838 | No |
| 8 | 1307 | Yes |
| 9 | 2409 | Yes |
| 10 | 3842 | Yes |
| 11 | 2650 | Yes |
| 12 | 921 | Yes |
| 13 | 1651 | No |
| 14 | 5485 | No |
| 15 | 1513 | No |
| 16 | 1834 | Yes |
| 17 | 1973 | Yes |
| 18 | 2006 | Yes |
| 19 | 1883 | No |
| 20 | 2844 | No |
| 21 | 2652 | Yes |
| 22 | 3021 | Yes |
| 23 | 1104 | No |

LGALS3 RAW DATA SORTED
BY PATIENT ID

## FIG. 11

| PATIENT ID | LGALS3 RAW MEASUREMENTS | KNOWN PSA RECURRENCE ? |
|:---:|:---:|:---:|
| 12 | 921 | Yes |
| 3 | 969 | No |
| 23 | 1104 | No |
| 4 | 1159 | No |
| 8 | 1307 | Yes |
| 15 | 1513 | No |
| 13 | 1651 | No |
| 5 | 1791 | No |
| 16 | 1834 | Yes |
| 7 | 1838 | No |
| 19 | 1883 | No |
| 17 | 1973 | Yes |
| 18 | 2006 | Yes |
| 2 | 2358 | No |
| 9 | 2409 | Yes |
| 6 | 2460 | Yes |
| 11 | 2650 | Yes |
| 21 | 2652 | Yes |
| 1 | 2802 | Yes |
| 20 | 2844 | No |
| 22 | 3021 | Yes |
| 10 | 3842 | Yes |
| 14 | 5485 | No |

LGALS3 RAW DATA SORTED
BY LGALS3 VALUES

## FIG. 12

LGALS3
RANK NORMALIZED
VALUES

| PATIENT ID | | KNOWN PSA RECURRENCE ? |
|---|---|---|
| 12 | 0/22 | Yes |
| 3 | 1/22 | No |
| 23 | 2/22 | No |
| 4 | 3/22 | No |
| 8 | 4/22 | Yes |
| 15 | 5/22 | No |
| 13 | 6/22 | No |
| 5 | 7/22 | No |
| 16 | 8/22 | Yes |
| 7 | 9/22 | No |
| 19 | 10/22 | No |
| 17 | 11/22 | Yes |
| 18 | 12/22 | Yes |
| 2 | 13/22 | No |
| 9 | 14/22 | Yes |
| 6 | 15/22 | Yes |
| 11 | 16/22 | Yes |
| 21 | 17/22 | Yes |
| 1 | 18/22 | Yes |
| 20 | 19/22 | No |
| 22 | 20/22 | Yes |
| 10 | 21/22 | Yes |
| 14 | 22/22 | No |

- (11)

+ (12)

CUT-POINT
(RANK VALUE OF $0.4\overline{99}$)

RANK NORMALIZED
VALUES

FIG. 13

P-VALUE = 0.002478

- (11)

+ (12)

KAPLAN-MEIER PLOT FOR LGALS3
UNIVARIATE FEATURE

FIG. 14

56

| PATIENT ID | MAGEC2 RAW MEASUREMENTS | KNOWN PSA RECURRENCE ? |
|---|---|---|
| 1 | 15 | Yes |
| 2 | 6 | No |
| 3 | 14 | No |
| 4 | 10 | No |
| 5 | 14 | No |
| 6 | 18 | Yes |
| 7 | 8 | No |
| 8 | 7 | Yes |
| 9 | 18 | Yes |
| 10 | 19 | Yes |
| 11 | 13 | Yes |
| 12 | 9 | Yes |
| 13 | 15 | No |
| 14 | 11 | No |
| 15 | 8 | No |
| 16 | 13 | Yes |
| 17 | 12 | Yes |
| 18 | 16 | Yes |
| 19 | 11 | No |
| 20 | 9 | No |
| 21 | 19 | Yes |
| 22 | 11 | Yes |
| 23 | 13 | No |

MAGEC2 RAW DATA SORTED BY PATIENT ID

# FIG. 15

| PATIENT ID | MAGEC2 RAW MEASUREMENTS | KNOWN PSA RECURRENCE ? |
|---|---|---|
| 2 | 6 | No |
| 8 | 7 | Yes |
| 7 | 8 | No |
| 15 | 8 | No |
| 12 | 9 | Yes |
| 20 | 9 | No |
| 4 | 10 | No |
| 14 | 11 | No |
| 19 | 11 | No |
| 22 | 11 | Yes |
| 17 | 12 | Yes |
| 11 | 13 | Yes |
| 16 | 13 | Yes |
| 23 | 13 | No |
| 3 | 14 | No |
| 5 | 14 | No |
| 1 | 15 | Yes |
| 13 | 15 | No |
| 18 | 16 | Yes |
| 6 | 18 | Yes |
| 9 | 18 | Yes |
| 10 | 19 | Yes |
| 21 | 19 | Yes |

MAGEC2 RAW DATA SORTED BY MAGEC2 VALUES

# FIG. 16

MAGEC2
RANK NORMALIZED
VALUES

| PATIENT ID | MAGEC2 RANK NORMALIZED VALUES | KNOWN PSA RECURRENCE ? |
|---|---|---|
| 2 | 0/12 | No |
| 8 | 1/12 | Yes |
| 7 | 2/12 | No |
| 15 | 2/12 | No |
| 12 | 3/12 | Yes |
| 20 | 3/12 | No |
| 4 | 4/12 | No |
| 14 | 5/12 | No |
| 19 | 5/12 | No |
| 22 | 5/12 | Yes |
| 17 | 6/12 | Yes |
| 11 | 7/12 | Yes |
| 16 | 7/12 | Yes |
| 23 | 7/12 | No |
| 3 | 8/12 | No |
| 5 | 8/12 | No |
| 1 | 9/12 | Yes |
| 13 | 9/12 | No |
| 18 | 10/12 | Yes |
| 6 | 11/12 | Yes |
| 9 | 11/12 | Yes |
| 10 | 12/12 | Yes |
| 21 | 12/12 | Yes |

− (10)

+ (13)

CUT-POINT
(RANK VALUE OF 0.4$\overline{99}$)

NORMALIZED RANK
VALUES

# FIG. 17

P-VALUE = 0.05880

− (10)

+ (13)

KAPLAN-MEIER PLOT FOR MAGEC2
UNIVARIATE FEATURE

# FIG. 18

IHC_DIST_CD163(+)_CD3(+)CD8(-)
RAW MEASUREMENTS

| PATIENT ID | | KNOWN PSA RECURRENCE ? |
|---|---|---|
| 1 | 582 | Yes |
| 2 | 621 | No |
| 3 | 1417 | No |
| 4 | 1120 | No |
| 5 | 1679 | No |
| 6 | 1601 | Yes |
| 7 | 634 | No |
| 8 | 511 | Yes |
| 9 | 687 | Yes |
| 10 | 728 | Yes |
| 11 | 1803 | Yes |
| 12 | 1220 | Yes |
| 13 | 2062 | No |
| 14 | 991 | No |
| 15 | 738 | No |
| 16 | 705 | Yes |
| 17 | 659 | Yes |
| 18 | 899 | Yes |
| 19 | 745 | No |
| 20 | 1198 | No |
| 21 | 681 | Yes |
| 22 | 728 | Yes |
| 23 | 1427 | No |

IHC_DIST_CD163(+)_CD3(+)CD8(-)
RAW DATA SORTED BY PATIENT ID

# FIG. 19

IHC_DIST_CD163(+)_CD3(+)CD8(-)
RAW MEASUREMENTS

| PATIENT ID | | KNOWN PSA RECURRENCE ? |
|---|---|---|
| 8 | 511 | Yes |
| 1 | 582 | Yes |
| 2 | 621 | No |
| 7 | 634 | No |
| 17 | 659 | Yes |
| 21 | 681 | Yes |
| 9 | 687 | Yes |
| 16 | 705 | Yes |
| 22 | 728 | Yes |
| 10 | 728 | Yes |
| 15 | 738 | No |
| 19 | 745 | No |
| 18 | 899 | Yes |
| 14 | 991 | No |
| 4 | 1120 | No |
| 20 | 1198 | No |
| 12 | 1220 | Yes |
| 3 | 1417 | No |
| 23 | 1427 | No |
| 6 | 1601 | Yes |
| 5 | 1679 | No |
| 11 | 1803 | Yes |
| 13 | 2062 | No |

IHC_DIST_CD163(+)_CD3(+)CD8(-) RAW
DATA SORTED BY
IHC_DIST_CD163(+)_CD(+)CD8(-) VALUE

# FIG. 20

IHC_DIST_CD163(+)_CD3(+)CD8(-)
RANK NORMALIZED VALUES

| PATIENT ID | | KNOWN PSA RECURRENCE ? |
|---|---|---|
| 8 | 0/22 | Yes |
| 1 | 1/22 | Yes |
| 2 | 2/22 | No |
| 7 | 3/22 | No |
| 17 | 4/22 | Yes |
| 21 | 5/22 | Yes |
| 9 | 6/22 | Yes |
| 16 | 7/22 | Yes |
| 22 | 8/22 | Yes |
| 10 | 9/22 | Yes |
| 15 | 10/22 | No |
| 19 | 11/22 | No |
| 18 | 12/22 | Yes |
| 14 | 13/22 | No |
| 4 | 14/22 | No |
| 20 | 15/22 | No |
| 12 | 16/22 | Yes |
| 3 | 17/22 | No |
| 23 | 18/22 | No |
| 6 | 19/22 | Yes |
| 5 | 20/22 | No |
| 11 | 21/22 | Yes |
| 13 | 22/22 | No |

− (11)

+ (12)

CUT-POINT
(RANK VALUE OF 0.4$\overline{99}$)

RANK NORMALIZED VALUES

## FIG. 21

P-VALUE = 0.02780

+ (12)

− (11)

KAPLAN-MEIER PLOT FOR
IHC_DIST_CD163(+)_CD3(+)CD8(-)
UNIVARIATE FEATURE

## FIG. 22

| | SYMBOL | NOTATION |
|---|---|---|
| SCORE SM1 FOR PATIENT K =<br>(f1 FOR PATIENT K) x (f2 FOR PATIENT K) | ◄——► | . and . |
| SCORE SM2 FOR PATIENT K =<br>((1-f1) FOR PATIENT K) x (f2 FOR PATIENT K) | ——► | not . and . |
| SCORE SM3 FOR PATIENT K =<br>(f1 FOR PATIENT K) x ((1-f2) FOR PATIENT K) | ◄—— | . and . not |
| SCORE SM4 FOR PATIENT K =<br>((1-f1) FOR PATIENT K) x ((1-f2) FOR PATIENT K) | —— | not . and . not |

BIVARIATE SCORING METHODS
AND NOTATIONS

# FIG. 23

| LGALS3 RANK VALUES (f1) PATIENT ID | | MAGEC2 RANK VALUES (f2) PATIENT ID | | f1 x f2 PATIENT ID | SM1 | f1 x f2 SORTED PATIENT ID | |
|---|---|---|---|---|---|---|---|
| 1 | 18/22 | 1 | 9/12 | 1 | 0.6136 | 2 | 0.0000 |
| 2 | 13/22 | 2 | 0/12 | 2 | 0.0000 | 12 | 0.0000 |
| 3 | 1/22 | 3 | 8/12 | 3 | 0.0303 | 8 | 0.0152 |
| 4 | 3/22 | 4 | 4/12 | 4 | 0.0455 | 3 | 0.0303 |
| 5 | 7/22 | 5 | 8/12 | 5 | 0.2121 | 15 | 0.0379 |
| 6 | 15/22 | 6 | 11/12 | 6 | 0.6250 | 4 | 0.0455 |
| 7 | 9/22 | 7 | 2/12 | 7 | 0.0682 | 23 | 0.0530 |
| 8 | 4/22 | 8 | 1/12 | 8 | 0.0152 | 7 | 0.0682 |
| 9 | 14/22 | 9 | 11/12 | 9 | 0.5833 | 19 | 0.1894 |
| 10 | 21/22 | 10 | 12/12 | 10 | 0.9545 | 13 | 0.2045 |
| 11 | 16/22 | 11 | 7/12 | 11 | 0.4242 | 5 | 0.2121 |
| 12 | 0/22 | 12 | 3/12 | 12 | 0.0000 | 16 | 0.2121 |
| 13 | 6/22 | 13 | 9/12 | 13 | 0.2045 | 20 | 0.2159 |
| 14 | 22/22 | 14 | 5/12 | 14 | 0.4167 | 17 | 0.2500 |
| 15 | 5/22 | 15 | 2/12 | 15 | 0.0379 | 22 | 0.3788 |
| 16 | 8/22 | 16 | 7/12 | 16 | 0.2121 | 14 | 0.4167 |
| 17 | 11/22 | 17 | 6/12 | 17 | 0.2500 | 11 | 0.4242 |
| 18 | 12/22 | 18 | 10/12 | 18 | 0.4545 | 18 | 0.4545 |
| 19 | 10/22 | 19 | 5/12 | 19 | 0.1894 | 9 | 0.5833 |
| 20 | 19/22 | 20 | 3/12 | 20 | 0.2159 | 1 | 0.6136 |
| 21 | 17/22 | 21 | 12/12 | 21 | 0.7727 | 6 | 0.6250 |
| 22 | 20/22 | 22 | 5/12 | 22 | 0.3788 | 21 | 0.7727 |
| 23 | 2/22 | 23 | 7/12 | 23 | 0.0530 | 10 | 0.9545 |

- (11)

CUT-POINT = 0.2121 (THE MEDIAN VALUE)

+ (12)

CALCULATION OF LGALS3-TO-MAGEC2 VALUES FOR SM1

FIG. 24

### FIG. 25

(1-f1) x f2

| PATIENT ID | SM2 |
|---|---|
| 1 | 0.1364 |
| 2 | 0.0000 |
| 3 | 0.6364 |
| 4 | 0.2879 |
| 5 | 0.4545 |
| 6 | 0.2917 |
| 7 | 0.0985 |
| 8 | 0.0682 |
| 9 | 0.3333 |
| 10 | 0.0455 |
| 11 | 0.1591 |
| 12 | 0.2500 |
| 13 | 0.5455 |
| 14 | 0.0000 |
| 15 | 0.1288 |
| 16 | 0.3712 |
| 17 | 0.2500 |
| 18 | 0.3788 |
| 19 | 0.2273 |
| 20 | 0.0341 |
| 21 | 0.2273 |
| 22 | 0.0379 |
| 23 | 0.5303 |

(1-f1) x f2 SORTED

| PATIENT ID | |
|---|---|
| 2 | 0.0000 |
| 14 | 0.0000 |
| 20 | 0.0341 |
| 22 | 0.0379 |
| 10 | 0.0455 |
| 8 | 0.0682 |
| 7 | 0.0985 |
| 15 | 0.1288 |
| 1 | 0.1364 |
| 11 | 0.1591 |
| 19 | 0.2273 |
| 21 | 0.2273 |
| 12 | 0.2500 |
| 17 | 0.2500 |
| 4 | 0.2879 |
| 6 | 0.2917 |
| 9 | 0.3333 |
| 16 | 0.3712 |
| 18 | 0.3788 |
| 5 | 0.4545 |
| 23 | 0.5303 |
| 13 | 0.5455 |
| 3 | 0.6364 |

CUT-POINT = 0.2273 (THE MEDIAN VALUE) · (11) + (12)

CALCULATION OF LGALS3-TO-MAGEC2 VALUES FOR SM2

## FIG. 25

### FIG. 26

f1 x (1-f2)

| PATIENT ID | SM3 |
|---|---|
| 1 | 0.2045 |
| 2 | 0.5909 |
| 3 | 0.0152 |
| 4 | 0.0909 |
| 5 | 0.1061 |
| 6 | 0.0568 |
| 7 | 0.3409 |
| 8 | 0.1667 |
| 9 | 0.0530 |
| 10 | 0.0000 |
| 11 | 0.3030 |
| 12 | 0.0000 |
| 13 | 0.0682 |
| 14 | 0.5833 |
| 15 | 0.1894 |
| 16 | 0.1515 |
| 17 | 0.2500 |
| 18 | 0.0909 |
| 19 | 0.2652 |
| 20 | 0.6477 |
| 21 | 0.0000 |
| 22 | 0.5303 |
| 23 | 0.0379 |

f1 x (1-f2) SORTED

| PATIENT ID | |
|---|---|
| 10 | 0.0000 |
| 12 | 0.0000 |
| 21 | 0.0000 |
| 3 | 0.0152 |
| 23 | 0.0379 |
| 9 | 0.0530 |
| 6 | 0.0568 |
| 13 | 0.0682 |
| 4 | 0.0909 |
| 18 | 0.0909 |
| 5 | 0.1061 |
| 16 | 0.1515 |
| 8 | 0.1667 |
| 15 | 0.1894 |
| 1 | 0.2045 |
| 17 | 0.2500 |
| 19 | 0.2652 |
| 11 | 0.3030 |
| 7 | 0.3409 |
| 22 | 0.5303 |
| 14 | 0.5833 |
| 2 | 0.5909 |
| 20 | 0.6477 |

CUT-POINT = 0.1515 (THE MEDIAN VALUE) · (11) + (12)

CALCULATION OF LGALS3-TO-MAGEC2 VALUES FOR SM3

## FIG. 26

| PATIENT ID | (1-f1) x (1-f2) SM4 | PATIENT ID | (1-f1) x (1-f2) SORTED |
|---|---|---|---|
| 1 | 0.0455 | 10 | 0.0000 |
| 2 | 0.4091 | 14 | 0.0000 |
| 3 | 0.3182 | 21 | 0.0000 |
| 4 | 0.5758 | 6 | 0.0265 |
| 5 | 0.2273 | 9 | 0.0303 |
| 6 | 0.0265 | 1 | 0.0455 |
| 7 | 0.4924 | 22 | 0.0530 |
| 8 | 0.7500 | 18 | 0.0758 |
| 9 | 0.0303 | 20 | 0.1023 |
| 10 | 0.0000 | 11 | 0.1136 |
| 11 | 0.1136 | 13 | 0.1818 |
| 12 | 0.7500 | 5 | 0.2273 |
| 13 | 0.1818 | 17 | 0.2500 |
| 14 | 0.0000 | 16 | 0.2652 |
| 15 | 0.6439 | 3 | 0.3182 |
| 16 | 0.2652 | 19 | 0.3182 |
| 17 | 0.2500 | 23 | 0.3788 |
| 18 | 0.0758 | 2 | 0.4091 |
| 19 | 0.3182 | 7 | 0.4924 |
| 20 | 0.1023 | 4 | 0.5758 |
| 21 | 0.0000 | 8 | 0.7500 |
| 22 | 0.0530 | 12 | 0.7500 |
| 23 | 0.3788 | 15 | 0.6439 |

CUT-POINT = 0.2272 (THE MEDIAN VALUE)

- (11)

+ (12)

CALCULATION OF LGALS3-TO-MAGEC2
VALUES FOR SM4

FIG. 27

LGALS3-TO-MAGEC2
SCORING METHOD
(SM)  DETERMINED P-VALUE

| LGALS3-TO-MAGEC2 SCORING METHOD (SM) | DETERMINED P-VALUE |
|---|---|
| SM1 | 0.0009008 |
| SM2 | 0.9806 |
| SM3 | 0.8034 |
| SM4 | 0.006845 |

SCORING METHOD = SM1
(THE SMALLEST P-VALUE
DETERMINES THE
SCORING METHOD USED)

DETERMINE SCORING METHOD

FIG. 28

- (11)
44    44
~45
45
P-VALUE = 0.0009008
+ (12)

KAPLAN-MEIER PLOT FOR THE LGALS3-TO-MAGEC2
BIVARIATE FEATURE (SM=SM1)

FIG. 29

| IHC_DIST_CD163(+)_CD3(+)CD8(-) RANK VALUES (f1) | | MAGEC2 RANK VALUES (f2) | | f1 x f2 | | f1 x f2 SORTED | |
|---|---|---|---|---|---|---|---|
| PATIENT ID | | PATIENT ID | | PATIENT ID | SM1 | PATIENT ID | |
| 1 | 1/22 | 1 | 9/12 | 1 | 0.0341 | 2 | 0.0000 |
| 2 | 2/22 | 2 | 0/12 | 2 | 0.0000 | 8 | 0.0000 |
| 3 | 17/22 | 3 | 8/12 | 3 | 0.5152 | 7 | 0.0227 |
| 4 | 14/22 | 4 | 4/12 | 4 | 0.2121 | 1 | 0.0341 |
| 5 | 20/22 | 5 | 8/12 | 5 | 0.6061 | 15 | 0.0758 |
| 6 | 19/22 | 6 | 11/12 | 6 | 0.7917 | 17 | 0.0909 |
| 7 | 3/22 | 7 | 2/12 | 7 | 0.0227 | 22 | 0.1515 |
| 8 | 0/22 | 8 | 1/12 | 8 | 0.0000 | 20 | 0.1705 |
| 9 | 6/22 | 9 | 11/12 | 9 | 0.2500 | 12 | 0.1818 |
| 10 | 9/22 | 10 | 12/12 | 10 | 0.4091 | 16 | 0.1856 |
| 11 | 21/22 | 11 | 7/12 | 11 | 0.5568 | 19 | 0.2083 |
| 12 | 16/22 | 12 | 3/12 | 12 | 0.1818 | 4 | 0.2121 |
| 13 | 22/22 | 13 | 9/12 | 13 | 0.7500 | 21 | 0.2273 |
| 14 | 13/22 | 14 | 5/12 | 14 | 0.2462 | 14 | 0.2462 |
| 15 | 10/22 | 15 | 2/12 | 15 | 0.0758 | 9 | 0.2500 |
| 16 | 7/22 | 16 | 7/12 | 16 | 0.1856 | 10 | 0.4091 |
| 17 | 4/22 | 17 | 6/12 | 17 | 0.0909 | 18 | 0.4545 |
| 18 | 12/22 | 18 | 10/12 | 18 | 0.4545 | 23 | 0.4773 |
| 19 | 11/22 | 19 | 5/12 | 19 | 0.2083 | 3 | 0.5152 |
| 20 | 15/22 | 20 | 3/12 | 20 | 0.1705 | 11 | 0.5568 |
| 21 | 5/22 | 21 | 12/12 | 21 | 0.2273 | 5 | 0.6061 |
| 22 | 8/22 | 22 | 5/12 | 22 | 0.1515 | 13 | 0.7500 |
| 23 | 18/22 | 23 | 7/12 | 23 | 0.4773 | 6 | 0.7917 |

- (11)

CUT-POINT = 0.2121 (THE MEDIAN VALUE)

+ (12)

CALCULATION OF IHC_DIST_CD163(+)_CD3(+)CD8(-)-TO-MAGEC2 VALUES FOR SM1

FIG. 30

66

FIG. 31 — (1-f1) x f2 ; (1-f1) x f2 SORTED

| PATIENT ID | SM2 | PATIENT ID | (1-f1) x f2 SORTED |
|---|---|---|---|
| 1 | 0.7159 | 2 | 0.0000 |
| 2 | 0.0000 | 13 | 0.0000 |
| 3 | 0.1515 | 11 | 0.0265 |
| 4 | 0.1212 | 5 | 0.0606 |
| 5 | 0.0606 | 12 | 0.0682 |
| 6 | 0.1250 | 20 | 0.0795 |
| 7 | 0.1439 | 8 | 0.0833 |
| 8 | 0.0833 | 15 | 0.0909 |
| 9 | 0.6667 | 23 | 0.1061 |
| 10 | 0.5909 | 4 | 0.1212 |
| 11 | 0.0265 | 6 | 0.1250 |
| 12 | 0.0682 | 7 | 0.1439 |
| 13 | 0.0000 | 3 | 0.1515 |
| 14 | 0.1750 | 14 | 0.1750 |
| 15 | 0.0909 | 19 | 0.2083 |
| 16 | 0.3977 | 22 | 0.2652 |
| 17 | 0.4091 | 18 | 0.3788 |
| 18 | 0.3788 | 16 | 0.3977 |
| 19 | 0.2083 | 17 | 0.4091 |
| 20 | 0.0795 | 10 | 0.5909 |
| 21 | 0.7727 | 9 | 0.6667 |
| 22 | 0.2652 | 1 | 0.7159 |
| 23 | 0.1061 | 21 | 0.7727 |

CUT-POINT = 0.1439 (THE MEDIAN VALUE)

CALCULATION OF IHC_CD163(+)_CD3(+)CD8(-)-TO-MAGEC2 VALUES FOR SM2

**FIG. 31**

FIG. 32 — f1 x (1-f2) ; f1 x (1-f2) SORTED

| PATIENT ID | SM3 | PATIENT ID | f1 x (1-f2) SORTED |
|---|---|---|---|
| 1 | 0.0114 | 10 | 0.0000 |
| 2 | 0.0909 | 8 | 0.0000 |
| 3 | 0.2576 | 21 | 0.0000 |
| 4 | 0.4242 | 1 | 0.0114 |
| 5 | 0.3030 | 9 | 0.0227 |
| 6 | 0.0720 | 6 | 0.0720 |
| 7 | 0.1136 | 2 | 0.0909 |
| 8 | 0.0000 | 17 | 0.0909 |
| 9 | 0.0227 | 18 | 0.0909 |
| 10 | 0.0000 | 7 | 0.1136 |
| 11 | 0.3977 | 16 | 0.1326 |
| 12 | 0.5455 | 22 | 0.2121 |
| 13 | 0.2500 | 13 | 0.2500 |
| 14 | 0.3447 | 3 | 0.2576 |
| 15 | 0.3788 | 19 | 0.2917 |
| 16 | 0.1326 | 5 | 0.3030 |
| 17 | 0.0909 | 23 | 0.3409 |
| 18 | 0.0909 | 14 | 0.3447 |
| 19 | 0.2917 | 15 | 0.3788 |
| 20 | 0.5114 | 11 | 0.3977 |
| 21 | 0.0000 | 4 | 0.4242 |
| 22 | 0.2121 | 20 | 0.5114 |
| 23 | 0.3409 | 12 | 0.5455 |

CUT-POINT = 0.2121 (THE MEDIAN VALUE)

CALCULATION OF IHC_CD163(+)_CD3(+)CD8(-)-TO-MAGEC2 VALUES FOR SM3

**FIG. 32**

|  | (1-f1) x (1-f2) | | (1-f1) x (1-f2) SORTED |
| PATIENT ID | SM4 | PATIENT ID | |
| --- | --- | --- | --- |
| 1 | 0.2386 | 10 | 0.0000 |
| 2 | 0.9091 | 13 | 0.0000 |
| 3 | 0.0758 | 21 | 0.0000 |
| 4 | 0.2424 | 6 | 0.0114 |
| 5 | 0.0303 | 11 | 0.0189 |
| 6 | 0.0114 | 5 | 0.0303 |
| 7 | 0.7197 | 9 | 0.0606 |
| 8 | 0.9167 | 3 | 0.0758 |
| 9 | 0.0606 | 18 | 0.0758 |
| 10 | 0.0000 | 23 | 0.0758 |
| 11 | 0.0189 | 12 | 0.2045 |
| 12 | 0.2045 | 1 | 0.2386 |
| 13 | 0.0000 | 14 | 0.2386 |
| 14 | 0.2386 | 20 | 0.2386 |
| 15 | 0.4545 | 4 | 0.2424 |
| 16 | 0.2841 | 16 | 0.2841 |
| 17 | 0.4091 | 19 | 0.2917 |
| 18 | 0.0758 | 22 | 0.3712 |
| 19 | 0.2917 | 17 | 0.4091 |
| 20 | 0.2386 | 15 | 0.4545 |
| 21 | 0.0000 | 7 | 0.7197 |
| 22 | 0.3712 | 2 | 0.9091 |
| 23 | 0.0758 | 8 | 0.9167 |

- (11)

+ (12)

CUT-POINT = 0.2386 (THE MEDIAN VALUE)

CALCULATION OF IHC_CD163(+)_CD3(+)CD8(-)-TO-MAGEC2 VALUES FOR SM4

FIG. 33

IHC_CD163(+)_CD3(+)
CD8(-)-TO-MAGEC2
SCORING METHOD    DETERMINED P-VALUE
     (SM)

| SM | DETERMINED P-VALUE |
|---|---|
| SM1 | 0.9605 |
| SM2 | 0.04208 |
| SM3 | 0.001686 |
| SM4 | 0.5164 |

SCORING METHOD = SM3
(THE SMALLEST P-VALUE
DETERMINES THE
SCORING METHOD USED)

DETERMINE SCORING METHOD

# FIG. 34

+ (12)

47                              47

46

- (11)

P-VALUE = 0.001686

46

KAPLAN-MEIER PLOT FOR THE
IHC_DIST_CD163(+)_CD3(+)CD8(-)-TO-MAGEC2
BIVARIATE FEATURE (SM=SM3)

# FIG. 35

| UNIVARIATE FEATURE | RAW MEASUREMENTS OF NEW PATIENT TO BE SCORED |
|---|---|
| LGALS3 | 2403 |
| MAGEC2 | 5 |
| IHC_DIST_CD163(+)_CD3(+)CD8(-) | 700 |

DIAGNOSTIC PHASE
RAW MEASUREMENTS OF A PATIENT

## FIG. 36

| LGALS3 RAW MEASUREMENT | PATIENT ID | RANK VALUE | |
|---|---|---|---|
| 921 | 12 | 0/22 | |
| 969 | 3 | 1/22 | |
| 1104 | 23 | 2/22 | |
| 1159 | 4 | 3/22 | |
| 1307 | 8 | 4/22 | |
| 1513 | 15 | 5/22 | LONGER SURVIVAL |
| 1651 | 13 | 6/22 | (SEE FIG. 14) |
| 1791 | 5 | 7/22 | |
| 1834 | 16 | 8/22 | |
| 1838 | 7 | 9/22 | CUT-POINT |
| 1883 | 19 | 10/22 | $\left(\text{RANK VALUE OF } 0.4\overline{99}\right)$ |
| 1973 | 17 | 11/22 | |
| 2006 | 18 | 12/22 | |
| 2358 | 2 | 13/22 | NEW PATIENT'S |
| 2409 | 9 | 14/22 | RANK VALUE = $\dfrac{13.5}{22}$ |
| 2460 | 6 | 15/22 | |
| 2650 | 11 | 16/22 | |
| 2652 | 21 | 17/22 | SHORTER SURVIVAL |
| 2802 | 1 | 18/22 | (SEE FIG. 14) |
| 2844 | 20 | 19/22 | NEW PATIENT'S RANK VALUE |
| 3021 | 22 | 20/22 | IS GREATER THAN CUT-POINT |
| 3842 | 10 | 21/22 | AND THEREFORE WITHIN THE SHORTER SURVIVAL GROUP, |
| 5485 | 14 | 22/22 | THEREFORE |

THE NEW PATIENT'S LGALS3 RAW MEASUREMENT IS 2403

$S_{LGALS3} = 1$

DIAGNOSTIC PHASE
DETERMINE A LGALS3 UNIVARIATE FEATURE
SCORE VALUE FOR THE NEW PATIENT

## FIG. 37

| MAGEC2 RAW MEASUREMENT | PATIENT ID | RANK VALUE |
|---|---|---|
| 6 | 2 | 0/12 |
| 7 | 8 | 1/12 |
| 8 | 7 | 2/12 |
| 8 | 15 | 2/12 |
| 9 | 12 | 3/12 |
| 9 | 20 | 3/12 |
| 10 | 4 | 4/12 |
| 11 | 14 | 5/12 |
| 11 | 19 | 5/12 |
| 11 | 22 | 5/12 |
| 12 | 17 | 6/12 |
| 13 | 11 | 7/12 |
| 13 | 16 | 7/12 |
| 13 | 23 | 7/12 |
| 14 | 3 | 8/12 |
| 14 | 5 | 8/12 |
| 15 | 1 | 9/12 |
| 15 | 13 | 9/12 |
| 16 | 18 | 10/12 |
| 18 | 6 | 11/12 |
| 18 | 9 | 11/12 |
| 19 | 10 | 12/12 |
| 19 | 21 | 12/12 |

THE NEW PATIENT'S MAGEC2 RAW MEASUREMENT IS 5

NEW PATIENT'S RANK VALUE = $\dfrac{10.5}{12}$

LONGER SURVIVAL (SEE FIG. 18)

CUT-POINT (RANK VALUE OF $0.4\overline{99}$)

SHORTER SURVIVAL (SEE FIG. 18)

NEW PATIENT'S RANK VALUE IS LOWER THAN CUT-POINT AND THEREFORE WITHIN THE LONGER SURVIVAL GROUP, THEREFORE

$S_{MAGEC2} = 0$

## DIAGNOSTIC PHASE
DETERMINE A MAGEC2 UNIVARIATE FEATURE SCORE VALUE FOR THE NEW PATIENT

# FIG. 38

IHC_DIST_CD163(+)_CD3(+)CD8(-)
RAW MEASUREMENT

| | PATIENT ID | RANK VALUE |
|---|---|---|
| 511 | 8 | 0/22 |
| 582 | 1 | 1/22 |
| 621 | 2 | 2/22 |
| 634 | 7 | 3/22 |
| 659 | 17 | 4/22 |
| 681 | 21 | 5/22 |
| 687 | 9 | 6/22 |
| 705 | 16 | 7/22 |
| 728 | 22 | 8/22 |
| 728 | 10 | 9/22 |
| 738 | 15 | 10/22 |
| 745 | 19 | 11/22 |
| 899 | 18 | 12/22 |
| 991 | 14 | 13/22 |
| 1120 | 4 | 14/22 |
| 1198 | 20 | 15/22 |
| 1220 | 12 | 16/22 |
| 1417 | 3 | 17/22 |
| 1427 | 23 | 18/22 |
| 1601 | 6 | 19/22 |
| 1679 | 5 | 20/22 |
| 1803 | 11 | 21/22 |
| 2062 | 13 | 22/22 |

THE NEW PATIENT'S
IHC_DIST_CD163(+)_
CD3(+)CD8(-)
RAW MEASUREMENT
IS 700

SHORTER SURVIVAL
(SEE FIG. 20)

NEW PATIENT'S RANK VALUE = $\dfrac{6.5}{22}$

CUT-POINT
(RANK VALUE OF $0.4\overline{99}$)

LONGER SURVIVAL
(SEE FIG. 20)

NEW PATIENT'S RANK
VALUE IS LOWER THAN
CUT-POINT AND
THEREFORE WITHIN THE
SHORTER SURVIVAL
GROUP, THEREFORE
$S_{IHC} = 1$

DIAGNOSTIC PHASE

DETERMINE A IHC_DIST_CD163(+)_CD3(+)CD8(-)
UNIVARIATE FEATURE SCORE VALUE FOR THE
NEW PATIENT

## FIG. 39

LGALS3 RANK VALUE MULTIPLIED BY
MAGEC2 RANK VALUE
(SM1)

| | PATIENT ID |
|---|---|
| 0.0000 | 2 |
| 0.0000 | 12 |
| 0.0152 | 8 |
| 0.0303 | 3 |
| 0.0379 | 15 |
| 0.0455 | 4 |
| 0.0530 | 23 |
| 0.0682 | 7 |
| 0.1894 | 19 |
| 0.2045 | 13 |
| 0.2121 | 5 |
| 0.2121 | 16 |
| 0.2159 | 20 |
| 0.2500 | 17 |
| 0.3788 | 22 |
| 0.4167 | 14 |
| 0.4242 | 11 |
| 0.4545 | 18 |
| 0.5833 | 9 |
| 0.6136 | 1 |
| 0.6250 | 6 |
| 0.7727 | 21 |
| 0.9545 | 10 |

NEW PATIENT'S SM1 VALUE IS LOWER THAN CUT-POINT AND THEREFORE WITHIN THE LONGER SURVIVAL GROUP, THEREFORE

$$S_{(LGALS3 - MAGEC2)} = 0$$

- (11)

LONGER SURVIVAL (SEE FIG. 29)

CUT-POINT = 0.2121 (THE MEDIAN VALUE)

NEW PATIENT'S LGALS3 RANK VALUE MULTIPLIED BY MAGEC2 RANK VALUE

$$\frac{13.5}{22} \times \frac{0}{12} = 0$$

+ (12)

SHORTER SURVIVAL (SEE FIG. 29)

DIAGNOSTIC PHASE

DETERMINE A LGALS3-TO-MAGEC2
BIVARIATE FEATURE SCORE VALUE FOR THE NEW PATIENT

# FIG. 40

IHC_DIST_CD163(+)_CD3(+)CD8(-)
RANK VALUE MULTIPLIED BY
(1 - MAGEC2 RANK VALUE)
(SM3)

| | PATIENT ID |
|---|---|
| 0.0000 | 10 |
| 0.0000 | 8 |
| 0.0000 | 21 |
| 0.0114 | 1 |
| 0.0227 | 9 |
| 0.0720 | 6 |
| 0.0909 | 2 |
| 0.0909 | 17 |
| 0.0909 | 18 |
| 0.1136 | 7 |
| 0.1326 | 16 |
| 0.2121 | 22 |
| 0.2500 | 13 |
| 0.2576 | 3 |
| 0.2917 | 19 |
| 0.3030 | 5 |
| 0.3409 | 23 |
| 0.3447 | 14 |
| 0.3788 | 15 |
| 0.3977 | 11 |
| 0.4242 | 4 |
| 0.5114 | 20 |
| 0.5455 | 12 |

- (11)

SHORTER SURVIVAL
(SEE FIG. 35)

NEW PATIENT'S RANK VALUE:

$$\frac{6.5}{22} \times \left(1 - \frac{0}{12}\right) = 0.2955$$

CUT-POINT = 0.2121
(THE MEDIAN VALUE)

NEW PATIENT'S SM3
VALUE IS LARGER THAN
CUT-POINT AND
THEREFORE WITHIN THE
LONGER SURVIVAL
GROUP, THEREFORE
$S_{(IHC - MAGEC2)} = 0$

+ (12)

LONGER SURVIVAL
(SEE FIG. 35)

DIAGNOSTIC PHASE

DETERMINE A IHC_DIST_CD163(+)_CD3(+)CD8(-)-TO-MAGEC2
BIVARIATE FEATURE SCORE VALUE FOR THE NEW PATIENT

FIG. 41

PSA Recurrence Score = 0, if $(S_{LGALS3}+S_{MAGEC2}+S_{IHC}+S_{(LGALS3-MAGEC2)}+$
$S_{(IHC-MAGEC2)}) < 2.5$

$= 1$, if $(S_{LGALS3}+S_{MAGEC2}+S_{IHC}+S_{(LGALS3-MAGEC2)}+$
$S_{(IHC-MAGEC2)}) > 2.5$

FUNCTION THAT DETERMINES THE PSA RECURRENCE
SCORE FOR THE NEW PATIENT

# FIG. 42

A HIGH SCORE
INDICATES A HIGH
PROBABILITY OF
RECURRENCE

$(S_{LGALS3}+S_{MAGEC2}+S_{IHC}+S_{(LGALS3-MAGEC2)}+S_{(IHC-MAGEC2)}) = (1+0+1+0+0) = 2$

because 2 < 2.5 the "PSA Recurrence Score" is 0.

DIAGNOSTIC PHASE

DETERMINE THE PSA RECURRENCE SCORE
FOR THE NEW PATIENT

# FIG. 43

55

IHC_Dist_CD163(+)_CD3(+)CD8(-)<--MAGEC2 ◉

WINDOW
50

51

0.5

53

54

0.4

0.3

0.2

0.1

52

0.0

PSA RECURRENCE RISK

0 (11 CASES)    1 (12 CASES)

# FIG. 44

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20170270420 A1

**Non-patent literature cited in the description**

- **HARDER et al.** *Journal for Immunotherapy of Cancer*, 07 November 2017, vol. 5 (2), 2
- **JIA-MEI CHEN et al.** *Tumor Biology*, March 2017, 1-12
- **PRANTIK CHATTERJEE et al.** *Journal of Bioinformatics and Computational Biology*, 24 February 2016, vol. 14 (1), 1650003
- **VANDESOMPELE J et al.** Accurate normalization of real-time quantitative RT-PCR data by geometric averaging of multiple internal control genes. *Genome Biol.*, 2002, vol. 3 (7)